# EUROPEAN PATENT APPLICATION

(11) **EP 4 831 293 A1**
(43) Date of publication of application: **28.10.2026**
(21) Application number: 24906212.6
(22) Date of filing: 12.12.2024
(51) Int. Cl.: C12P 1/06, C07H 1/08, C12R 1/465

(54) **BIOSYNTHETIC GENE CLUSTER FOR POLYENE MACROLIDE NATURAL PRODUCT MANDIMYCIN, AND NATURAL PRODUCT AND USE THEREOF**

(30) Priority: 20.12.2023 CN 202311762214; 14.05.2024 CN 202410595877
(71) Applicant: China Pharmaceutical University, Nanjing, Jiangsu 211198 (CN)
(72) Inventor: WANG, Zongqiang, Nanjing, Jiangsu 211198 (CN); DENG, Qisen, Nanjing, Jiangsu 211198 (CN); LI, Yinchuan, Nanjing, Jiangsu 211198 (CN)
(74) Representative: Murgitroyd & Company
(86) International application number: PCT/CN2024/138651
(87) International publication number: WO 2025/130729

(57) **Abstract**

Disclosed in the present invention are a biosynthetic gene cluster for a polyene macrolide natural product mandimycin, and a natural product and use thereof. The nucleotide sequence of the mandimycin biosynthetic gene cluster is shown in SEQ ID NO. 1, and the compound structural formulas of the natural products mandimycin and mandimycin B are shown in formula I and formula II. The natural product mandimycin of the present invention can target phospholipid molecules in fungal cell membranes, especially phosphatidylinositol, to cause efflux of important ions in fungal cells, thereby leading to the death of fungal cells. The natural products mandimycin and mandimycin B of the present invention have strong in-vivo and in-vitro antifungal activity and a broad antifungal spectrum against multiple multi-drug resistant fungal key pathogens published by WHO, comprising *Candida, Aspergillus, Cryptococcus, Mucor* and *Fusarium.*

## Description

### TECHNICAL FIELD

The present invention belongs to the field of microbial natural products, and in particular to a biosynthetic gene cluster for a polyene macrolide natural product mandimycin, and a natural product and use thereof.

### BACKGROUND

With the widespread emergence of antifungal drug resistance, the increasing number of immunodeficient and immunocompromised patients, and the limited categories of available antifungal agents, fungal infections have become a major safety issue threatening human health. Therefore, there is an urgent need to discover novel antifungal drugs. At present, the antifungal agents used in clinical practice primarily fall into four categories: *Echinocandins* (such as *Caspofungin*), polyene macrolides (such as amphotericin B), Azoles (such as *Fluconazole*), and 5-fluorocytosine (Nature Reviews Microbiology 2022; 20:9 557-571). Specifically, polyene macrolide antibiotics, exemplified by amphotericin B, exhibit broad-spectrum, potent antifungal activity and a low propensity for resistance development. Since the 1950s, they have served as first-line agents for the clinical treatment of fungal infections, and amphotericin B has been included by the WHO in the *List of Essential Medicines for Treating Fungal Infections* (Antibiot Annu. 1955; 3:587-91; Curr Opin Microbiol. 2022; 70:102208). To date, numerous polyene macrolide antibiotics with diverse structural features have been isolated from nature. These compounds typically contain 26-, 28-, 36-, or 38-membered macrolactone rings, three to seven conjugated double bonds, and one to two deoxysugar substituents (Mol Phylogenet Evol. 2018; 127:239-247). Currently, known polyene antibiotics exert their potent antifungal activities primarily by binding to ergosterol, a sterol molecule present in the fungal cell membrane, thereby forming transmembrane channels. This process leads to the leakage of essential intracellular ions and small molecules, ultimately killing the fungal pathogens (Proc Natl Acad Sci USA. 2012; 109(7):2234-9; Proc Natl Acad Sci USA. 2011; 108(17):6733-8).

Although marketed polyene antifungal drugs (such as amphotericin B, nystatin, and natamycin), exhibit significant fungicidal activity, broad-spectrum activity, and a relatively low incidence of resistance of fungal pathogens, and are therefore potent antifungal agents, they fail to meet clinical needs due to issues such as increasing fungal resistance resulting from long-term use, their extremely low water solubility, and severe toxic and side effects. In addition to its broad-spectrum and potent activity, amphotericin B has the notable advantage of a low propensity for resistance development. However, its clinical application is limited by a lack of activity against clinically emerging multidrug-resistant strains, significant toxic and side effects, and low oral bioavailability.

### SUMMARY

Objectives of the present invention: In order to address the problems existing in the prior art, the present invention aims to provide a biosynthetic gene cluster for a polyene macrolide natural product mandimycin, where a nucleotide sequence of the mandimycin biosynthetic gene cluster is shown in SEQ ID NO. 1, and the gene cluster of the present invention is capable of producing a novel polyene macrolide antifungal natural product. Further, the present invention constructs an engineered strain CPU002 using genetic manipulation to knock out a key glycosyltransferase gene in the mandimycin biosynthetic gene cluster. Using the engineered strain CPU002, a novel antifungal antibiotic natural product mandimycin B lacking atratcynose A is biosynthesized. This method overcomes the technical limitations associated with current clinical antifungal drugs, such as a limited variety of available agents, high toxicity, low oral bioavailability, and ineffective against multidrug-resistant fungi, which restrict their clinical application. The present invention also provides polyene macrolide natural products mandimycin and mandimycin B, and use thereof.

Technical solutions: In order to achieve the above objectives, the present invention provides a biosynthetic gene cluster for a polyene macrolide natural product mandimycin, where a nucleotide sequence of the mandimycin biosynthetic gene cluster is shown in SEQ ID NO. 1. A preparation method for the polyene macrolide natural product mandimycin includes the following steps:
preparing a strain containing the mandimycin biosynthetic gene cluster into a seed culture, and performing cultivation, fermentation, extraction, isolation, and purification on the seed culture to obtain the natural product mandimycin.

Specifically, the strain containing the mandimycin biosynthetic gene cluster is *Streptomyces netropsis* DSM 40259.

Use of the biosynthetic gene cluster for a polyene macrolide natural product mandimycin provided by the present invention in the preparation of mandimycin and mandimycin B is further provided.

A preparation method for an antifungal polyene macrolide natural product mandimycin B includes the following steps:
knocking out a *MandQ* gene in the strain containing the mandimycin biosynthetic gene cluster to obtain an engineered strain CPU002; preparing a seed culture, followed by cultivation, fermentation, extraction, isolation, and purification to obtain the natural product mandimycin B; where a nucleotide sequence of the *MandQ* gene is shown in SEQ ID NO. 2.

Specifically, the engineered strain CPU002 is obtained by knocking out the MandQ gene in the mandimycin biosynthetic gene cluster of *Streptomyces netropsis* DSM 40259 using intergeneric conjugation between *Streptomyces* and *Escherichia coli,* and the resulting engineered strain CPU002 with a glycosyltransferase gene deleted is subjected to fermentation, extraction, and purification to obtain mandimycin B.

The present invention provides a polyene macrolide natural product mandimycin or mandimycin B and a pharmaceutically acceptable salt thereof. A chemical structure of the natural product mandimycin is shown in formula I, and a chemical structure of the natural product mandimycin B is shown in formula II:

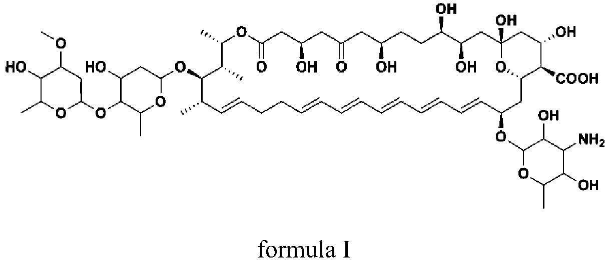

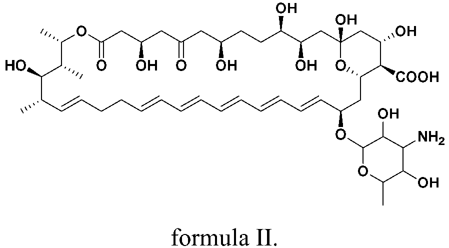

Further, a core macrolactone ring of the polyene macrolide natural product mandimycin or mandimycin B has a 38-membered macrolactone structure, where: C20-C29 form a conjugated pentaene structure; C32 forms a monoene structure; C19 is substituted with a mycosamine moiety; C3, C7, C10, and C13 are each substituted with a β-hydroxyl group; C15 is substituted with an α-hydroxyl group; C13 and C15 are connected via an oxygen bridge; C1 is an ester group; C5 is a ketone group; C34, C36, and C37 are each substituted with an α-methyl group; and C16 is substituted with a formyl group.

The present invention provides use of the polyene macrolide natural product mandimycin or mandimycin B and a pharmaceutically acceptable salt thereof in the preparation of an antifungal drug.

Further, the fungus is any one of various key fungal pathogens, such as *Candida*, *Aspergillus*, *Cryptococcus*, *Mucor*, and *Fusarium* species.

Further, the *Candida* is *Candida albicans*, *Candida auris*, *Candida glabrata*, *Candida tropicalis*, or *Candida parapsilosis*.

The present invention provides a pharmaceutical composition containing the antifungal drug, including the polyene macrolide natural product mandimycin or mandimycin B and the pharmaceutically acceptable salt thereof.

Further, the pharmaceutical composition is in the form of a capsule, a powder, a tablet, a granule, a pill, an injection, a syrup, an oral solution, an inhalant, an ointment, a suppository, or a patch.

The present invention further provides use of the antifungal pharmaceutical composition in the preparation of an antifungal drug.

Further, the fungus is any one of various key fungal pathogens, such as *Candida*, *Aspergillus*, *Cryptococcus*, *Mucor*, and *Fusarium* species.

The present invention identifies a biosynthetic functional gene cluster, designated as mandimycin-BGC, expressing mandimycin from a microbial secondary metabolite database (MiSM) using a phylogeny-guided targeted mining approach.

The mandimycin-BGC contains six core genes (*mandD*, *mandE*, *mandF*, *mandL*, *mandM*, and *mandN*), encoding 19 polyketide synthase modules.

Through combinatorial biosynthesis, a natural product named mandimycin has been discovered, and it exhibits potent, broad-spectrum activity against multidrug-resistant fungi. Mandimycin is a 38-membered macrolactone featuring a unique chemical architecture with a ketone at position 5, and contains three deoxysugar substituents (including a C19 mycosamine and a C35 dideoxysugar atratcynose A (α-L-oleandropyranosyl-(1→4)-β-D-digitoxopyranoside), as well as a C20 pentaene unit and a C32 monoene unit.

The mandimycin compound of the present invention is a natural product produced by fermentation of *Streptomyces netropsis* DSM 40259. The screening of the *Streptomyces* strain is performed using a targeted mining technique combining big data analysis, phylogenetic analysis, and clustering analysis. Using a conserved mycosamine glycosyltransferase as a sequence tag, a hidden Markov model (HMM) is employed to mine all biosynthetic gene clusters containing the corresponding mycosamine sequence tag from a microbial secondary metabolite database (MiSM). Further, phylogenetic analysis is combined to directionally identify a novel branch of biosynthetic gene clusters, designated as the mandimycin biosynthetic gene cluster (mandimycin-BGC), where a gene sequence thereof is shown in SEQ ID NO. 1. Microbial strains potentially containing the gene cluster are analyzed, and the specific actinomycete *Streptomyces netropsis* DSM 40259 is obtained, which includes the mandimycin-BGC. Mandimycin is unstable under acidic conditions and is prone to dehydration and loss of its side-chain glycosyl groups. Therefore, mandimycin may be dissolved in neutral or weakly alkaline organic solvents.

When exploring the mechanism of action of the compound, feeding experiments, scanning electron microscopy, ion concentration analysis, and isothermal titration calorimetry (ITC) are employed. During the preparation of mandimycin, fermentation conditions are optimized to remove fermentation by-products, and crude products are subjected to extraction, isolation, and purification processes.

The present invention discovers a novel antifungal drug target. Mandimycin targets phospholipid molecules, especially phosphatidylinositol, in the fungal cell membrane (including phosphatidylinositol, phosphatidylglycerol, phosphatidylserine, phosphatidylethanolamine, phosphatidylcholine, sphingomyelin, and cardiolipin), resulting in efflux of essential intracellular ions (including K⁺, and the like) and leading to the death of fungal pathogens. This mechanism has not yet been reported for antifungal agents.

Due to its unique multi-target mechanism, mandimycin does not develop resistance under laboratory conditions and exhibits potent activity against strains resistant to clinical antifungal drugs.

The present invention identifies a novel biosynthetic gene cluster in the strain *Streptomyces netropsis* DSM 40259 through pan-genome mining. Further fermentation, purification, and gene knockout experiments confirm that the product of this gene cluster is mandimycin.

Mandimycin is a polyene macrolide antifungal antibiotic with a novel mechanism of action. It exerts antifungal activity by targeting phospholipid molecules, especially phosphatidylinositol, in the fungal cell membrane, resulting in efflux of intracellular ions and small molecules and causing fungal cell death. Mandimycin exhibits potent and broad-spectrum antifungal activity against various key fungal pathogens, including *Candida*, *Aspergillus*, *Cryptococcus*, *Mucor*, and *Fusarium* species. Further, through intergeneric conjugation between *Streptomyces and Escherichia coli*, the present invention obtains an engineered strain CPU002 with a glycosyltransferase gene deleted by knocking out a glycosyltransferase gene *MandQ* responsible for biosynthesis of atratcynose A in the mandimycin biosynthetic gene cluster. The engineered strain is further fermented to biosynthesize a novel polyene macrolide natural product mandimycin B. Mandimycin B of the present invention is a natural product produced by fermentation of the engineered strain CPU002, which is obtained by knocking out the *MandQ* gene in the mandimycin biosynthetic gene cluster of *Streptomyces netropsis* DSM 40259. The engineered strain CPU002 is cultured and fermented in an FS/9 medium to produce mandimycin B.

During the preparation and synthesis of the natural product mandimycin B, a key enzyme *MandQ* responsible for biosynthesis of atratcynose A is identified. Through gene knockout, a 1072-bp fragment of *MandQ* is deleted, thereby disrupting the glycosyltransferase function of *MandQ* and obtaining the *MandQ*-deficient engineered strain CPU002. The engineered strain is used to synthesize mandimycin B, a novel mandimycin derivative lacking atratcynose A. Experiments have revealed that mandimycin B also exhibits potent broad-spectrum antifungal activity. Unlike mandimycin, mandimycin B exerts antifungal activity by binding to ergosterol in the fungal cell membrane. Mandimycin B, the novel mandimycin derivative provided by the present invention, differs from mandimycin, despite sharing the same core skeleton, mandimycin B does not have the disaccharide structural unit of atratcynose A. Mandimycin B has a mechanism of action distinct from that of mandimycin. It exerts antifungal activity by binding to ergosterol in the fungal cell membrane and shows no affinity for the phospholipid targets of mandimycin.

Beneficial effects: compared with the prior art, the present invention has the following advantages:
The present invention provides, for the first time, a biosynthetic gene cluster of the polyene macrolide natural product mandimycin. The strain containing the mandimycin biosynthetic gene cluster is used to prepare a seed culture, and the seed culture is subject to cultivation, fermentation, extraction, isolation, and purification to obtain the natural product mandimycin. The natural product mandimycin of the present invention has strong antifungal activity and a broad antifungal spectrum against multi-drug resistant fungal key pathogens published by WHO, including *Candida*, *Aspergillus*, *Cryptococcus*, *Mucor* and *Fusarium*. The minimum inhibitory concentrations (MICs) of the compound of the present invention against multidrug-resistant fungi, including *Candida albicans* and *Candida auris*, are 1 to 2 times the clinically used nystatin and are comparable to those of amphotericin B, falling within a range of 0.25-1 µg/mL. Its activity against multidrug-resistant *Cryptococcus* neoformans reaches 0.125 µg/mL, which is 32-fold more potent than caspofungin, 64-fold more potent than fluconazole, and 128-fold more potent than 5-fluorocytosine. Its activity against multidrug-resistant *Aspergillus* fumigatus reaches 2 µg/mL, which is 32-fold more potent than fluconazole and 16-fold more potent than 5-fluorocytosine. Mandimycin also exhibits favorable antifungal activity against amphotericin B-resistant strains. This demonstrates that mandimycin has broad-spectrum antifungal activity and strong efficacy against clinical strains resistant to existing antifungal drugs. The natural product mandimycin B of the present invention has strong antifungal activity and a broad antifungal spectrum against multi-drug resistant fungal key pathogens published by WHO, including *Candida*, *Aspergillus*, *Cryptococcus. Mucor* and *Fusarium*. Specifically, the minimum inhibitory concentrations (MICs) against *Candida* species, especially multidrug-resistant strains including *Candida albicans* and *Candida auris*, fall within a range of 1-2 µg/mL. Its activity against multidrug-resistant *Cryptococcus* neoformans reaches 0.5 µg/mL. Its activity against multidrug-resistant *Aspergillus* fumigatus reaches 2 µg/mL, which is 32-fold greater than fluconazole and 16-fold greater than 5-fluorocytosine.

The natural product mandimycin of the present invention acts on an antifungal target distinct from that of known antifungal drugs. It acts on phospholipid components of the fungal cell membrane. Specifically, phosphatidylinositol is the strongest binding target, with a dissociation constant (*K*_{d}) value of 21.9 µM; followed by phosphatidylglycerol, with a *K*_{d} value of 28.2 µM; phosphatidylserine, with a *K*_{d} value of 30.9 µM; phosphatidylethanolamine, with a *K*_{d} value of 36.5 µM; phosphatidylcholine, with a *K*_{d} value of 36.8 µM; sphingomyelin, with a *K*_{d} value of 50 µM; and cardiolipin, with a *K*_{d} value of 63.6 µM. Under laboratory conditions, mandimycin at low concentrations does not induce resistance development. Compared with amphotericin B, the compounds of the present invention exhibit significantly improved aqueous solubility, which is approximately 9,700 times greater than that of amphotericin B. *In vivo* antifungal activity studies in mice demonstrate that mandimycin exhibits significant antifungal activity against multidrug-resistant *Candida albicans* and *Candida auris*, showing a dose-dependent effect. Specifically, at a dose of 5 mg/kg, fungal burden of *Candida albicans* strain BNCC 186382 is reduced by 3 log₁₀ CFU, and *Candida auris* strain BNCC 357785 is reduced by 2 log₁₀ CFU. At a dose of 10 mg/kg, fungal burden of *Candida albicans* BNCC 186382 is reduced by 3.6 log₁₀ CFU, and *Candida auris* BNCC 357785 is reduced by 2.5 log₁₀ CFU. At a dose of 20 mg/kg, fungal burden of *Candida albicans* BNCC 186382 is reduced by 4.2 log₁₀ CFU, and *Candida auris* BNCC 357785 is reduced by 3 log₁₀ CFU. In a mouse model of infection with pan-drug-resistant *Candida auris* resistant to amphotericin B, mandimycin still exhibits good *in vivo* antifungal activity, reducing fungal burden by 2.3 log₁₀ CFU at a dose of 10 mg/kg. No acute toxicity was observed in all *in vivo* animal experiments at different doses. Therefore, the natural product mandimycin of the present invention can be used for the preparation of antifungal drugs.

### BRIEF DESCRIPTION OF THE DRAWINGS

FIG. 1 illustrates a process of mining a mandimycin biosynthetic gene cluster.
FIG. 2 illustrates a mandimycin biosynthetic gene cluster and a structure thereof.
FIG. 3 illustrates a predicted biosynthetic pathway of mandimycin.
FIG. 4 illustrates a UV spectrum of mandimycin.
FIG. 5 illustrates HPLC profiles and activity comparison between a mandimycin gene-knockout strain and a wild-type strain.
FIG. 6 illustrates high-resolution mass spectrometry (HRMS) spectra of mandimycin (A: positive ion mode; B: negative ion mode).
FIG. 7 illustrates a ¹H-NMR spectrum of mandimycin (DMSO-*d*₆).
FIG. 8 illustrates a ¹³C-NMR spectrum of mandimycin (DMSO-*d*₆).
FIG. 9 illustrates an HSQC spectrum of mandimycin (DMSO-*d*₆).
FIG. 10 illustrates an HMBC spectrum of mandimycin (DMSO-*d*₆).
FIG. 11 illustrates a COSY spectrum of mandimycin (DMSO-*d*₆).
FIG. 12 illustrates an ROESY spectrum of mandimycin (DMSO-*d*₆).
FIG. 13 illustrates a structure and 2D NMR spectrum of mandimycin.
FIG. 14 illustrates a chiral bioinformatics analysis of mandimycin polyketide reductase.
FIG. 15 illustrates the cytotoxicity of mandimycin against HepG2 cells.
FIG. 16 illustrates the hemolytic activity of mandimycin.
FIG. 17 illustrates the *in vivo* antifungal activity of mandimycin, wherein: (A) multidrug-resistant *Candida albicans* strain BNCC 186382 infection model; (B) multidrug-resistant *Candida albicans* strain BNCC 357785 infection model; (C) pan-drug-resistant *Candida auris* strain AMR05 infection model.
FIG. 18 illustrates a mouse model of disseminated candidiasis.
FIG. 19 illustrates a mouse model of invasive candidiasis.
FIG. 20 illustrates a mouse model of fungal skin infection.
FIG. 21 illustrates a mouse model of vaginal infection.
FIG. 22 illustrates *in vivo* pharmacokinetic study of mandimycin.
FIG. 23 illustrates *In vivo* nephrotoxicity evaluation of mandimycin.
FIG. 24 illustrates resistance results of mandimycin and changes in activity fold against other polyene antifungal agents, wherein: (A) amphotericin B-resistant strains; (B) natamycin-resistant strains; (C) nystatin-resistant strains.
FIG. 25 illustrates time-kill curves of mandimycin.
FIG. 26 illustrates morphology of *Candida albicans* treated with mandimycin as observed by scanning electron microscopy.
FIG. 27 illustrates changes in K⁺ concentration in *Candida albicans* treated with mandimycin.
FIG. 28 illustrates inhibition curves of mandimycin activity by different fungal cell components.
FIG. 29 illustrates exothermic curve of an interaction between small-molecule phospholipids and mandimycin.
FIG. 30 illustrates a 1072-bp fragment of *MandQ* from the mandimycin biosynthetic gene cluster knockout.
FIG. 31 illustrates identification of mandimycin B by HPLC.
FIG. 32 illustrates a ¹H-NMR spectrum of mandimycin B.
FIG. 33 illustrates a ¹³C-NMR spectrum of mandimycin B.
FIG. 34 illustrates an HSQC spectrum of mandimycin B.
FIG. 35 illustrates an HMBC spectrum of mandimycin B.
FIG. 36 illustrates a COSY spectrum of mandimycin B.
FIG. 37 illustrates a TOCSY spectrum of mandimycin B.
FIG. 38 illustrates correlations in key HMBC and COSY spectra of mandimycin B.
FIG. 39 illustrates the determination of a mandimycin B target using UV-vis.
FIG. 40 illustrates the determination of a mandimycin B target using ITC.

### DETAILED DESCRIPTION OF EMBODIMENTS

The technical solutions of the present invention are further described below with reference to the accompanying drawings. Unless otherwise specified, the materials and reagents used in the following examples are commercially available. Experimental methods for which specific conditions are not indicated in the examples are generally performed according to conventional conditions or according to the conditions recommended by the manufacturers. Specifically, the strain *Streptomyces netropsis DSM 40259* was purchased from DSMZ. Pancreatic digest of casein was purchased from Guangdong Huankai Microbial Science and Technology Co., Ltd. (Catalog No. 024048). Enzymatic digest of soybean was purchased from Beijing Hongrun Baoshun Technology Co., Ltd. (Catalog No. Y030A 220816). Beef extract was purchased from Beijing Hongrun Baoshun Technology Co., Ltd. (Catalog No. Y014C 230809).

### Example 1

### Discovery of the mandimycin biosynthetic gene cluster

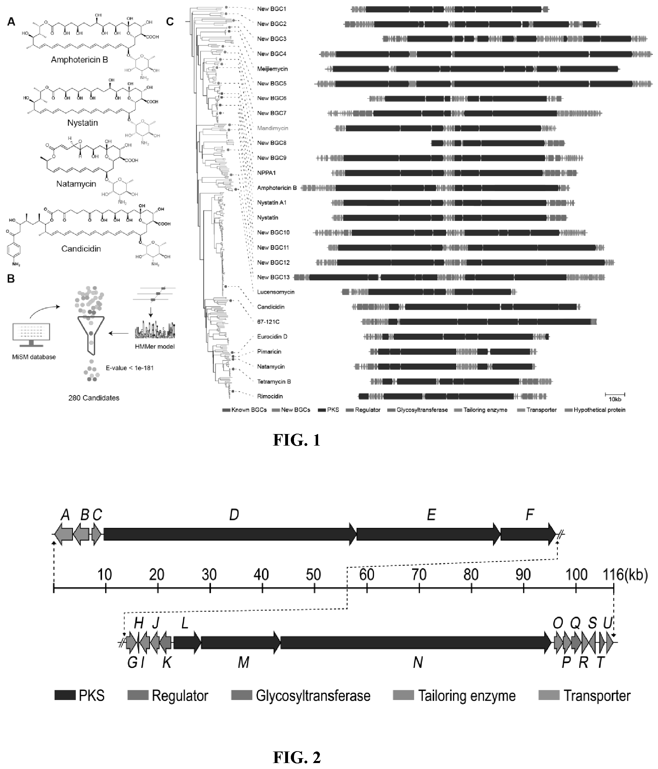

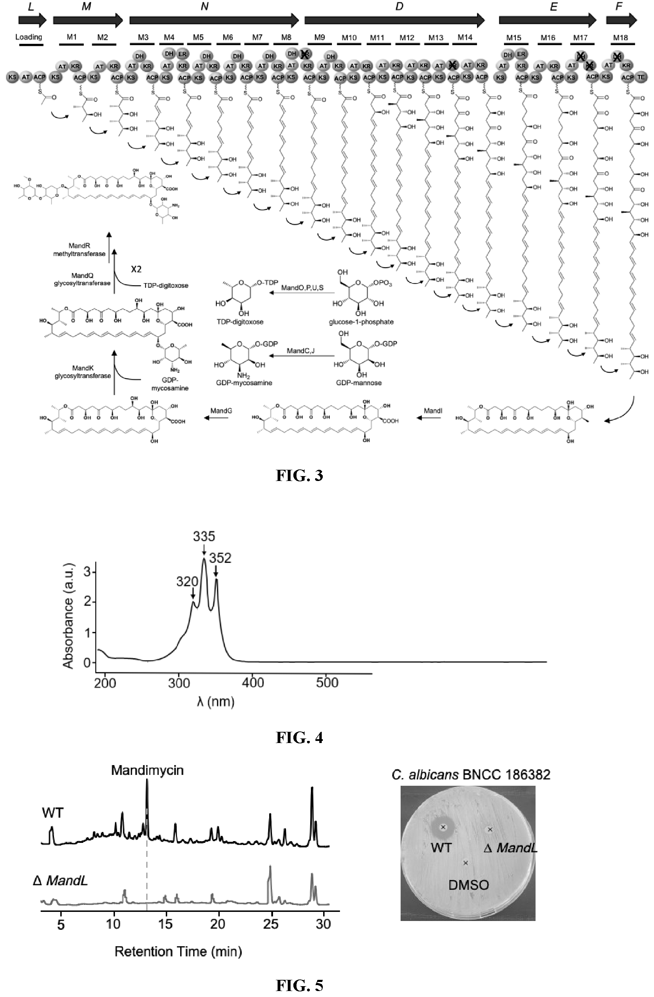

FIG. 1 illustrates a process of mining the mandimycin biosynthetic gene cluster. Conserved protein structural sequences were analyzed and identified based on sequence information of mycosamine glycosyltransferases from known polyene macrolide natural products (Table 1). A specific hidden Markov model for polyene mycosamine glycosyltransferases was constructed. Sequence similarity scanning against a microbial secondary metabolite database was performed using the model. Protein sequences having similarity values of less than 1e⁻¹⁸¹ were regarded as mycosamine sequences associated with polyene natural products, and the functional gene clusters in which such sequences were located were thus considered to be potential functional gene clusters encoding novel polyene natural products. A search using the method identified 280 non-redundant candidate sequences. A phylogenetic tree was constructed for these sequences to analyze the phylogenetic evolutionary relationships among different sequences. As shown in FIG. 2, the results reveal a novel evolutionary branch that encodes a completely novel polyene natural product. The corresponding biosynthetic gene cluster (BGC) is designated as mandimycin-BGC, a sequence thereof is shown in SEQ ID NO. 1. FIG. 3 illustrates a predicted biosynthetic pathway of mandimycin, with functional annotations of the biosynthetic genes detailed in Table 2. Further screening revealed that *Streptomyces netropsis* DSM 40259 harbors a biosynthetic gene cluster designated as mandimycin-BGC, and this strain could be for the fermentation production of the natural product mandimycin.

**Table 1 Information of mycosamine sequences from polyene macrolide natural product**

| Polyene Macrolide Antibiotic | Glycosyltransferase ID | Protein size(aa) |
|---|---|---|
| 67-121A | AGM21646.1 | 457 |
| Amphotericin B | AAK73511.1 | 483 |
| Candicidin | AAQ82555.1 | 458 |
| Eurocidin D | WP_102918851.1 | 459 |
| Lucensomycin | QSE03587.1 | 474 |
| Natamycin | ADD85130.2 | 458 |
| NPPA | OLM18863.1 | 471 |
| NystatinA1 | AVX51105.1 | 463 |
| Pimaricin | CAC20918.1 | 458 |
| Rimocidin | AAR16517.1 | 460 |
| Tetramycin | QIZ24108.1 | 462 |

**Table 2 Functional annotations of the mandimycin biosynthetic gene cluster**

| ORF | Gene name | Size (bp) | Putative Protein, [Source Organism], Accession number | Proposed Function |
|---|---|---|---|---|
| 1 | *mandA* | 2016 | ABC transporter ATP-binding protein/permease [*Streptomyces*]\|WP_130880883.1\| | Efflux |
| 2 | *mandB* | 1752 | ABC transporter transmembrane domain-containing protein [*Streptomyces* syringium]\|WP_245382703.1\| | Efflux |
| 3 | *mandC* | 1032 | GDP-mannose 4,6-dehydratase [*Streptomyces* sp. NRRL B-1568]\|KJY42921.1\| | Mycosamine biosynthesis |
| 4 | *mandD* | 28422 | type I polyketide synthase CP077658.1 | PKS modules 9-14 |
| 5 | *mandE* | 16182 | type I polyketide synthase [*Streptomyces* syringium]\|WP_209518050.1\| | PKS modules 15-17 |
| 6 | *mandF* | 6186 | type I polyketide synthase [*Streptomyces netropsis*]\|WP_130880878.1\| | PKS module 18 +thioesterase |
| 7 | *mandG* | 1188 | cytochrome P450 [*Streptomyces*]\|WP_130880877.1\| | Hydroxylation |
| 8 | *mandH* | 198 | ferredoxin [*Streptomyces*]\|WP_242626363.1\| | Ferredoxin |
| 9 | *mandI* | 1176 | cytochrome P450 [*Streptomyces*]\|WP_130880876.1\| | Hydroxylation |
| 10 | *mandJ* | 1059 | DegT/DnrJ/EryC1/StrS family aminotransferase [*Streptomyces*]\|WP_130880875.1\| | Mycosamine biosynthesis |
| 11 | *mandK* | 1389 | glycosyltransferase [*Streptomyces* syringium]\|WP_209518054.1\| type I polyketide synthase | Glycosyltransferase |
| 12 | *mandL* | 3243 | [*Streptomyces netropsis*]\|WP_229822576.1\| | PKS Loading module |
| 13 | *mandM* | 9492 | acyl transferase domain-containing protein/acyl carrier protein [*Streptomyces* syringium]\|MBP2406714.1\| | PKS modules 1-2 |
| 14 | *mandN* | 32331 | type I polyketide synthase [*Streptomyces netropsis*]\| | PKS modules 3-8 |
| 15 | *mandO* | 1068 | glucose-1-phosphate thymidylyltransferase [*Streptomyces*]\|WP_130880348.1\| | Digitoxose biosynthesis |
| 16 | *mandP* | 981 | dTDP-glucose 4,6-dehydratase [*Streptomyces netropsis*]\|WP_130880349.1\| | Digitoxose biosynthesis |
| 17 | *mandQ* | 1167 | glycosyltransferase [*Streptomyces* syringium]\|MBP2406718.1\| | Glycosyltransferase |
| 18 | *mandR* | 795 | FkbM family methyltransferase [*Streptomyces*]\|WP_130880350.1\| | 4-O-methyl-digitoxose biosynthesis |
| 19 | *mandS* | 765 | NAD-dependent epimerase/dehydratase family protein [*Streptomyces netropsis*]\|WP_130880351.1\| | Digitoxose biosynthesis |
| 20 | *mandT* | 576 | LuxR family transcriptional regulator [*Streptomyces* sp. NRRL B-1568]\|KJY41371.1\| | Regulator |
| 21 | *mandU* | 771 | alpha/beta fold hydrolase [*Streptomyces netropsis*]\|WP_130880353.1\| | Digitoxose biosynthesis |

### Example 2

### Biological fermentation of mandimycin

### (1) Preparation of spore suspension:

A *Streptomyces netropsis* DSM 40259 strain harboring a mandimycin biosynthetic gene cluster was inoculated onto ISP4 solid medium (containing 10.0 g soluble starch, 1.0 g dipotassium hydrogen phosphate, 1.0 g magnesium sulfate, 1.0 g sodium chloride, 2.0 g ammonium sulfate, 2.0 g calcium sulfate, 0.001 g ferrous sulfate, 0.001 g manganese chloride, 0.001 g zinc sulfate, and 15.0 g agar per liter of medium, pH = 7.2), and cultured at 30°C for 5 d to prepare the spore suspension.

### (2) Preparation of seed culture:

1 mL of the spore suspension was taken and inoculated into 50 mL of TSB medium (containing 17.0 g pancreatic digest of casein, 3.0 g enzymatic digest of soybean, 5.0 g sodium chloride, 2.5 g dipotassium hydrogen phosphate, and 2.5 g D-glucose monohydrate per liter of medium, pH = 7.3), followed by incubation in a shaking incubator (200 rpm at a temperature of 30°C) for 2 days to prepare the seed culture.

### (3) Preparation of fermentation broth:

0.5 mL of the seed culture prepared in the step (2) of this Example was transferred into 50 mL of F2 fermentation medium (containing 69.0 g glucose, 25.0 g beef extract, 9.0 g CaCO₃, and 0.1 g KH₂PO₄ per liter of medium) and cultured for 10 days (30°C at a speed of 200 rpm of the shaking incubator) to prepare the fermentation broth.

### Example 3

### Extraction, isolation, and purification of mandimycin

After completion of fermentation, *n*-butanol was added to a fermentation flask (at a volume ratio of fermentation broth to n-butanol = 1:1), followed by extraction with stirring overnight (100 rpm); and the *n*-butanol extract was evaporated to dryness using a rotary evaporator and then dissolved in methanol to obtain a crude extract. The crude extract was purified using a two-step method. First, isocratic elution column chromatography was performed using a column packed with YMC-GEL C18 (12 nm × 50 µm). The column was equilibrated with 2 column volumes (CV) of 10% methanol (H₂O:CH₃OH = 9:1). A methanolic solution was then loaded onto the column and eluted using stepwise gradient elution with methanol solutions (10%, 30%, 50%, 70%, 90%, and 100%), with each fraction collected in 100 mL portions. The fractions were analyzed using UPLC-MS, and fractions containing mandimycin (an eluate obtained with 90% aqueous methanol) were collected and evaporated to dryness. The UPLC-MS conditions were as follows: a C18 column (Waters, T3, 1.8 µm, 2.1 × 100 mm); mobile phase A-pure water (0.1% formic acid); mobile phase B-acetonitrile (0.1% formic acid); with a flow rate of 0.6 mL/min; a gradient of mobile phase B from 30% to 90% at a rate of 10% per minute. An MS detection range was 200-2,000, with simultaneous operation in both positive and negative ion modes. A semi-purified product was then further purified using semi-preparative reversed-phase high-performance liquid chromatography (RP-HPLC). The RP-HPLC conditions were as follows: a C18 column (Shimadzu, ShimNet HE C18-AQ, 5 µm OBD, 19 × 250 mm); solvent A, deionized water; solvent B, acetonitrile. A flow rate was 3 mL/min. A gradient of solvent B increased from 30% to 90% at a rate of 1.5% per minute, and full-wavelength detection (190-800 nm) was performed. As shown in FIG. 4, an ultraviolet spectrum of mandimycin exhibits characteristic ultraviolet absorption peaks of a conjugated pentaene (320, 335, and 352 nm). The natural product mandimycin with a purity greater than 95% (yellow powder) was collected as the purified natural product mandimycin, with a yield of 4-6 mg/L fermentation broth.

### Example 4

### Genetic knockout validation of mandimycin biosynthesis

To investigate the relationship between mandimycin-BGC and the conjugated pentaene polyene natural product, genetic manipulation at a genomic level was performed on the *Streptomyces* strain producing the compound. 1 kb upstream and downstream homologous arms of *mandL* were amplified by PCR (primer sequences shown below) and cloned into a pKC1139 vector to construct a knockout vector pKC1139-*mandL*_KO. The latter was introduced using a *Streptomyces* conjugation transfer experiment to delete the critical 692-bp sequence of *mandL* from position -334 to position +358 (as shown in Table 2). A comprehensive comparative analysis of secondary metabolites produced by the wild-type strain and the knockout strain was performed. As shown in FIG. 5, the conjugated pentaene natural product was confirmed to be the biosynthetic product of mandimycin-BGC, and the compound was designated as mandimycin.

| Primer name | Primer sequence |
|---|---|
| *mandL_KOUF* | aataacccttgacgctggaggtgtcctggg |
| *mandL_KOUR* | cgatatcgcgcgcggccgcgCGGATATCACCCACGGCACCAC |
| *mandL_KODF* | gctgcaggtcgactctagagtccgcagattgtccggcagcagctcc |
| *mandL_KODR* | ctccagcgtcaagggttattgtcatgggctcgactcgg |

### Example 5

### Structural elucidation of mandimycin

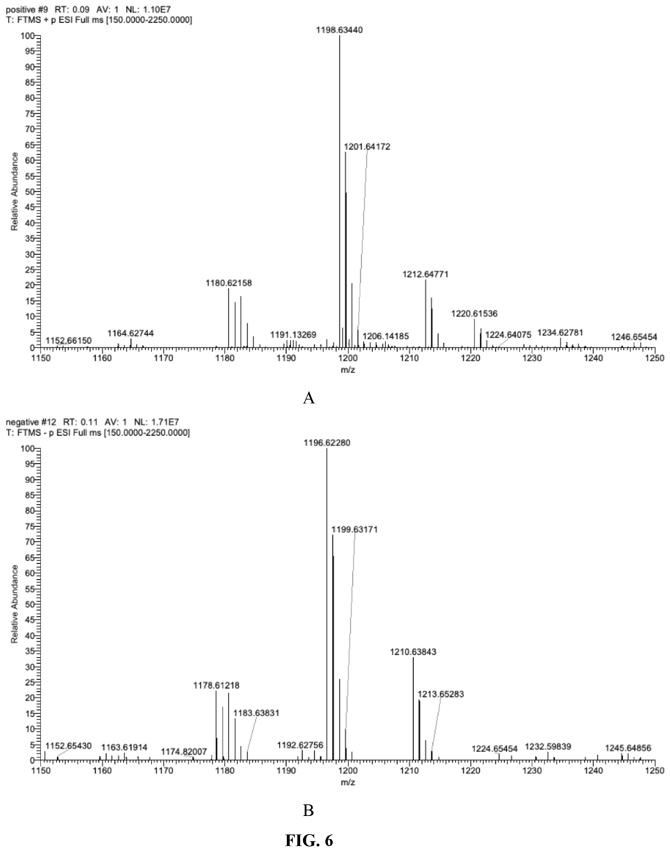

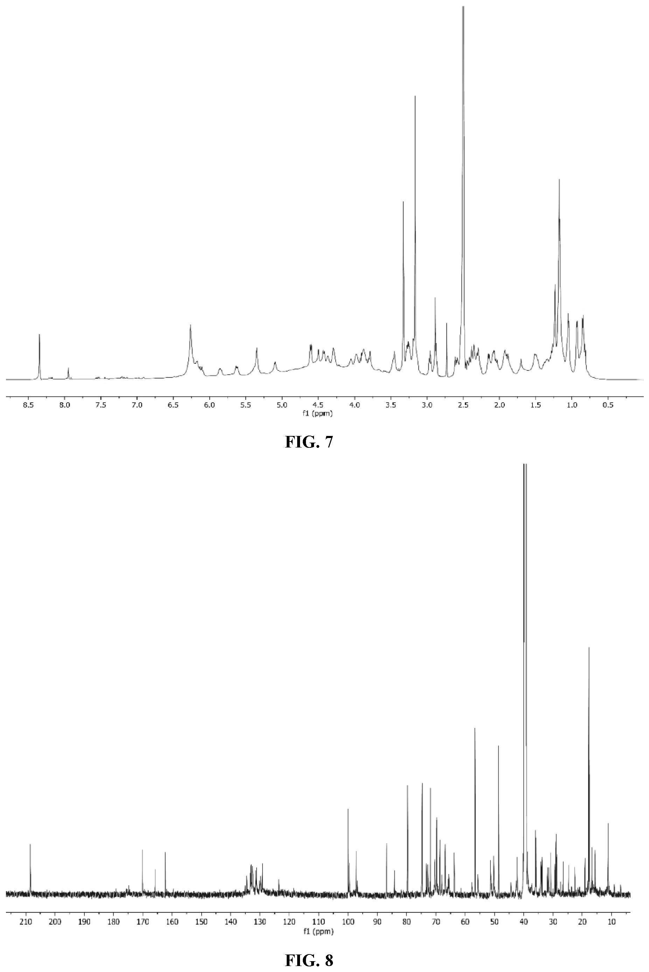

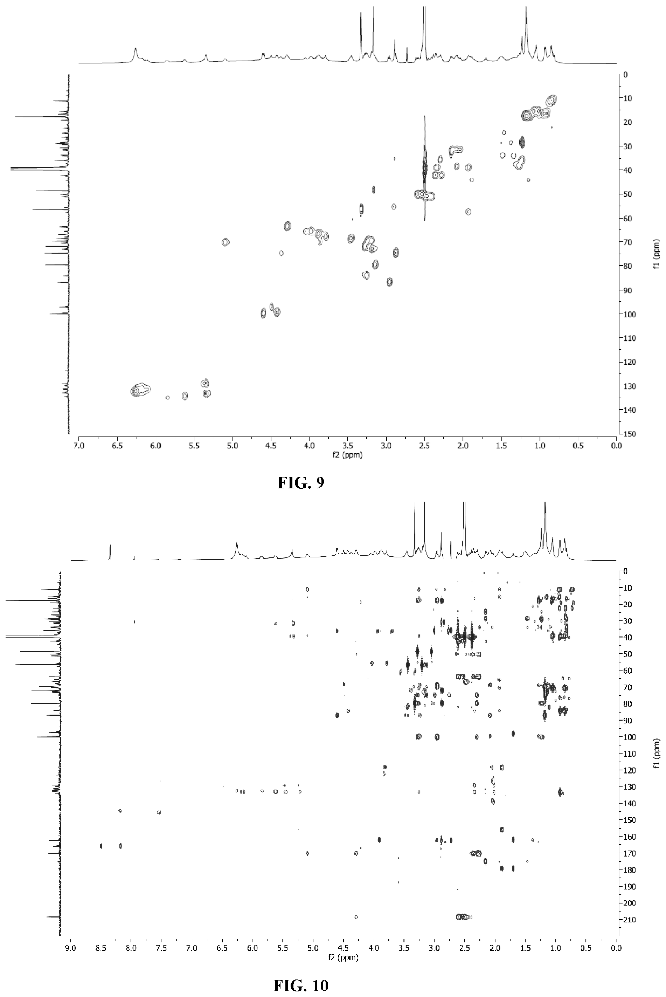

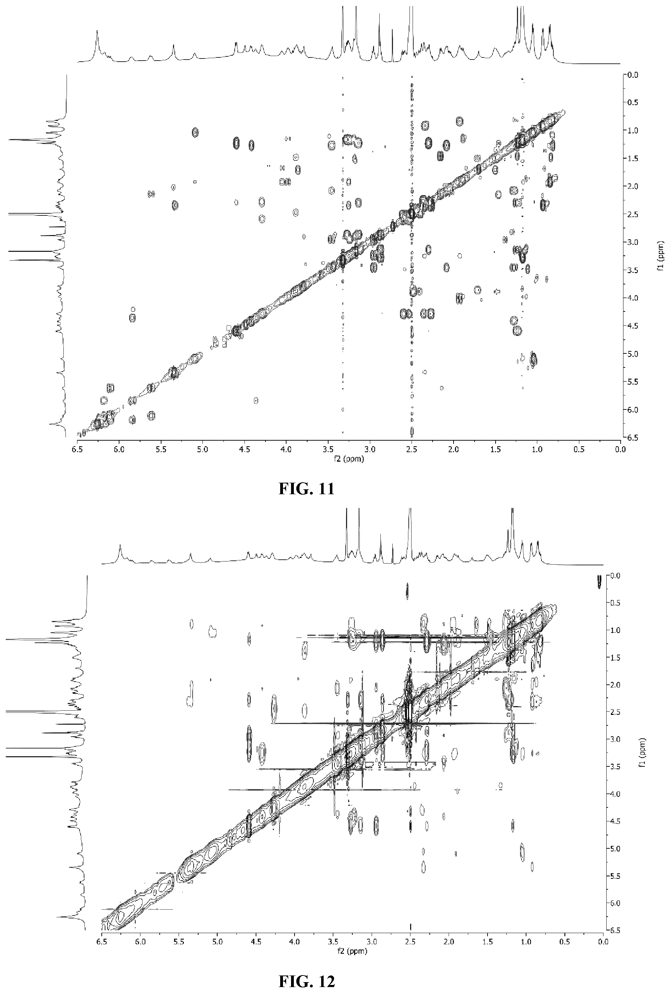

The mandimycin purified in Example 3 was a yellow powder. As shown in FIG. 6, Orbitrap high-resolution mass spectrometric analysis revealed a protonated molecular ion at m/z 1198.6344 [M+H]⁺ and a deprotonated molecular ion at m/z 1196.6228 [M-H]⁻, indicating a molecular formula of C₆₀H₉₅NO₂₃ (Δppm -1.97), containing 14 degrees of unsaturation (DBE). As shown in FIG. 4, the ultraviolet-visible spectrum of mandimycin in methanol exhibits maximum absorption wavelengths at 320, 335, and 352 nm, indicating the presence of a conjugated pentaene structure. The mandimycin isolated and purified in Example 3 was dissolved in deuterated DMSO and analyzed using a 700 MHz nuclear magnetic resonance spectrometer. As shown in FIGS. 7-12, ¹H-NMR, ¹³C-NMR spectra, and 2D NMR spectra including HSQC, HMBC, COSY, and the like, were obtained to determine a structure of mandimycin. The ¹H NMR and ¹³C NMR spectroscopic data of mandimycin exhibited NMR signals corresponding to 12 olefinic protons (δ_{H} 5.60-6.30, δ_{C} 129.0-136.0), a plurality of oxygenated methine signals (δ_{C} 63.0-87.0), and three carbonyl signals (δ_{C} 208.4, 174.8, and 170.1), indicating that mandimycin possesses a highly oxygenated polyene macrolactone skeleton. Analysis was performed using a series of two-dimensional (2D) NMR techniques (HSQC, HMBC, COSY, and ROESY) to establish a 38-membered macrolactone skeleton. Furthermore, HMBC correlations from H3 (δ_{H} 4.29), H4 (δ_{H} 2.53 and 2.59), and H6 (δ_{H} 2.42 and 2.47) to C5 (δ_{C} 208.4) further confirmed that C5 was substituted with a ketone group, which differs from other 38-membered macrolactone polyene antibiotics in which C5 is substituted by a hydroxyl group or a methylene group.

The presence of three sugar moieties in mandimycin was established based on the NMR signals corresponding to three anomeric carbons (δ_{H-1 '}4.49, δ_{C-1 '}96.8; δ_{H-1 "}4.42, δ_{C-1 "}99.5; δ_{H-1 ‴}4.61, δ_{C-1 ‴}100.0). COSY and HMBC spectral analyses revealed the presence of one mycosamine, one digitoxose, and one 3-*O*-methyldigitoxose in mandimycin. The linkage of mycosamine to C-19 of the macrolactone was established based on the observation of an HMBC correlation from H-1 '(δ_{H} 4.49) to C-19 (δ_{C} 74.7). The linkage of digitoxose to C-35 was established based on the HMBC correlation observed from H-1 "(δ_{H} 4.42) to C-35 (δ_{C} 84.1). Further, it was determined that a glycosidic linkage between C-1 ‴of 3-*O-*methyldigitoxose and C-4 "of digitoxose was formed based on the observation of HMBC correlations from H-1 ‴(δ_{H} 4.61) to C-4 "(δ_{C} 86.8). This is the first report of trisaccharide substitution in a polyene macrolide antibiotic. ¹H NMR and ¹³C NMR data (DMSO-*d*₆) are shown in Table 3.

**Table 3 ¹H NMR and ¹³C NMR data (DMSO-d₆) of mandimycin**

| Pos . | δ_{C}, type* | δ_{H}, mult. (J in Hz)# | Pos. | δ_{C}, type* | δ_{H}, mult. (J in Hz)# |
|---|---|---|---|---|---|
| Polyene macrolide | | | 32 | 129.2, CH | 5.37 |
| 1 | 170.1, C | | 33 | 133.3, CH | 5.34 |
| 2 | 42.3, CH₂ | 2.27; 2.36 | 34 | 38.7, CH | 2.34 |
| 3 | 63.7, CH | 4.29 | 35 | 84.1, CH | 3.26 |
| 4 | 50.3, CH₂ | 2.53; 2.59 | 36 | 38.7, CH | 1.92 |
| 5 | 208.4, C | | 37 | 70.4, CH | 5.1 |
| 6 | 51.3, CH₂ | 2.42; 2.47 | 16-COOH | 174.8, C | |
| 7 | 66.9, CH | 3.89 | 34-CH₃ | 16.7, CH₃ | 0.93 |
| 8 | 34.2, CH₂ | 1.33; 1.47 | 36-CH₃ | 11.1, CH₃ | 0.85 |
| 9 | 28.8, CH₂ | 1.37; 1.49 | 37-CH₃ | 15.6, CH₃ | 1.05 |
| 10 | 66.8, CH | 3.91 | Mycosamine | | |
| 11 | 65.8, CH | 4.00 | 1' | 96.8, CH | 4.49 |
| 12 | 44.4, CH₂ | 1.15; 1.90 | 2' | 67.9, CH | 3.79 |
| 13 | 97.2, C | | 3' | 55.7, CH | 2.9 |
| 14 | 42.6, CH₂ | 1.50; 1.71 | 4' | 73.3, CH | 3.14 |
| 15 | 65.6, CH | 3.98 | 5' | 71.9, CH | 3.27 |
| 16 | 57.7, CH | 1.94 | 6' | 17.8, CH₃ | 1.17 |
| 17 | 65.5, CH | 4.05 | Digitoxose | | |
| 18 | 37.2, CH₂ | 1.68; 1.86 | 1" | 99.5, CH | 4.42 |
| 19 | 74.7, CH | 4.36 | 2" | 38.5, CH₂ | 1.28; 2.08 |
| 20 | 135.1, CH | 5.85 | 3" | 68.7, CH | 3.45 |
| 21 | 131.2, CH | 6.18 | 4" | 86.8, CH | 2.96 |
| 22 | 129.1-134.4, CH | 6.05-6.32 | 5" | 69.7, CH | 3.24 |
| 23 | 129.1-134.4, CH | 6.05-6.32 | 6" | 17.8, CH₃ | 1.17 |
| 24 | 129.1-134.4, CH | 6.05-6.32 | 3-O-methyldigitoxose | | |
| 25 | 129.1-134.4, CH | 6.05-6.32 | 1‴ | 100.0, CH | 4.61 |
| 26 | 129.1-134.4, CH | 6.05-6.32 | 2‴ | 35.7, CH₂ | 1.23; 2.30 |
| 27 | 129.1-134.4, CH | 6.05-6.32 | 3‴ | 79.7, CH | 3.14 |
| 28 | 131.4, CH | 6.11 | 4‴ | 74.7, CH | 2.88 |
| 29 | 134.4, CH | 5.62 | 5‴ | 71.9, CH | 3.28 |
| 30 | 31.9, CH | 2.16 | 6‴ | 17.6, CH₃ | 1.17 |
| 31 | 31.5, CH₂ | 2.03; 2.15 | 3‴-OCH₃ | 56.7, CH₃ | 3.32 |

| | | | | | |
|---|---|---|---|---|---|
| * The assignment of some carbon signals was supported by HSQC and HMBC correlations. # The coupling constants for proton signals were not provided as most signals are highly overlapped or broad. | | | | | |

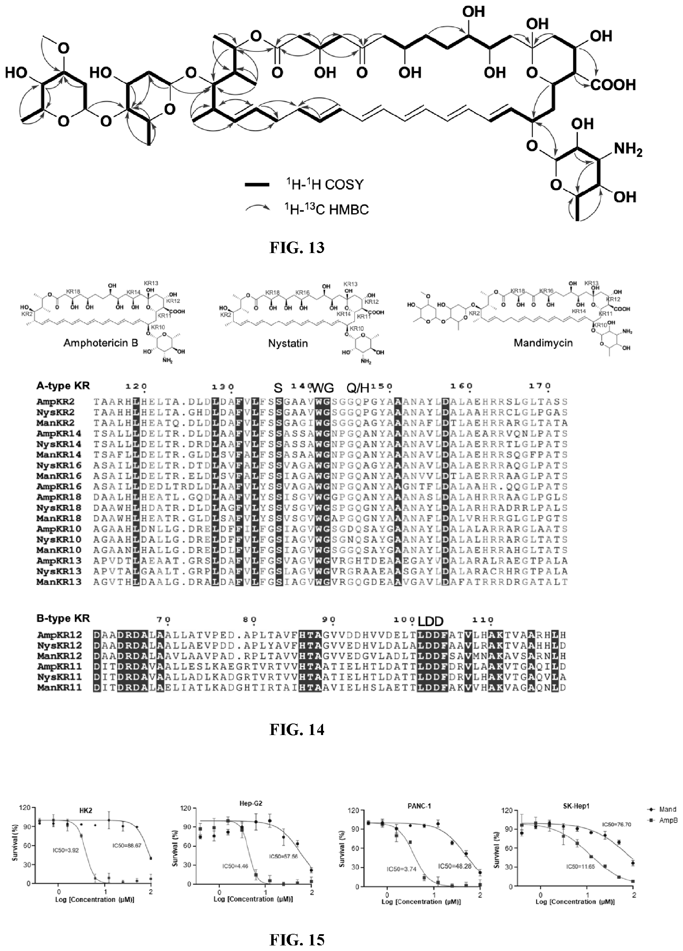

Further, based on analyses of the ¹H NMR and ¹³C NMR spectra, and 2D NMR spectra including HSQC, HMBC, COSY, and the like (FIGS. 7-13), together with bioinformatic analysis of the mandimycin polyketide synthase (FIG. 14), the specific configuration of the natural product mandimycin of the present invention was determined to be 2*R*, 10*R*, 11*S*, 12*S*, 13*R*, 14*R*, 16*R*, and 18*R*. The natural product mandimycin is represented by formula I:

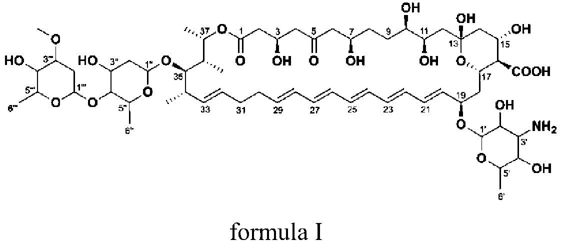

### Example 6

### Water solubility of mandimycin

Amphotericin B (1.2 mg/mL) and mandimycin purified in Example 3 (12 mg/mL) were separately dissolved in methanol to prepare stock solutions of amphotericin B (1.2 mg/mL) and mandimycin (12 mg/mL), respectively. A 100 µL aliquot of each of the resulting stock solutions was pre-frozen using liquid nitrogen, and then lyophilized in a freeze dryer for 24 h. Subsequently, 50 µL (for amphotericin B) or 2 µL (for mandimycin) of 10 mM Tris-HCl buffer (pH = 7.4) was added to the freeze-dried samples, and the mixtures were vortexed for 15 min to obtain saturated solutions. The mixtures were centrifuged at 12,000 rpm for 3 min, and the resulting supernatants were diluted with Tris-HCl buffer (10-fold dilution for amphotericin B and 1,000-fold dilution for mandimycin). A 10 µL aliquot was injected, and concentrations of the compounds in solution were determined by measuring the UV absorption spectra using a Shimadzu PDA detector spectrophotometer. Specifically, amphotericin B and mandimycin were accurately weighed and dissolved in DMSO to prepare 2 mg/mL stock solutions. The characteristic heptaene UV absorption peak of amphotericin B at its maximum absorption wavelength of 384 nm was used for quantification based on peak area. The characteristic pentaene UV absorption peak of mandimycin at its maximum absorption wavelength of 334 nm was used for quantification based on peak area. Standard curves correlating UV absorption peak area with mass were generated for mandimycin and amphotericin B, respectively, to calculate the aqueous solubilities of mandimycin and amphotericin B. As shown in Table 4, the solubility of mandimycin was 8.07 mg/mL, which was 9,700 times that of amphotericin B.

**Table 4 Solubility test results of mandimycin**

| | mandimycin | Amphotericin B | Fold (Man/Amp) |
|---|---|---|---|
| Solubility (10 mM Tris-HCl, pH7.4) | 8.07 (mg/mL) | 0.83 (µg/mL) | 9700 |

### Example 7

### Biological activity evaluation of mandimycin

### (1) In vitro activity assay against multidrug-resistant fungi

The antifungal activity of mandimycin against priority fungal pathogens listed by the WHO was evaluated using CLSI standards. The results are shown in Table 5. Mandimycin exhibited potent, broad-spectrum antifungal activity against multidrug-resistant fungi.

**Table 5 Antifungal activity of mandimycin**

| **WHO Priority group** | **MDR Pathogens** | **Resistant Phenotype** | **MIC (µg/mL)** |
|---|---|---|---|
| Critical priority | *Candida albicans* BNCC 186382 | FCAR | 0.25 |
| | *Candida albicans* BNCC 337321 | FCAR, 5-FUR, TBFR | 0.5 |
| | *Candida albicans* BNCC 336485 | FCAR, 5-FUR, TBFR | 0.5 |
| | *Candida auris* BNCC 357785 | FCAR, 5-FUR, TBFR | 1 |
| | *Candida auris* BNCC 357784 | FCAR, 5-FUR, TBFR | 1 |
| | *Candida auris* AMR05 | AmpR, FCAR, 5-FUR, TBFR | 2 |
| | *Cryptococcus neoformans* BNCC 225501 | CPFR, FCAR, 5-FUR | 0.125 |
| | *Cryptococcus neoformans* BNCC 356067 | CPFR, FCAR, 5-FUR | 0.125 |
| | *Aspergillus fumigatus* BNCC 122691 | FCAR, 5-FUR, TBFR | 2 |
| | *Aspergillus fumigatus* BNCC 340016 | FCAR, 5-FUR, TBFR | 2 |
| High priority | *Candida glabrata* BNCC 337348 | CPFR, FCAR, 5-FUR, TBFR | 0.25 |
| | *Candida tropicalis* BNCC 340288 | 5-FUR | 0.5 |
| | *Candida parapsilosis* BNCC 337317 | TBFR, 5-FUR | 1 |
| | *Mucor* sp. BNCC 195604 | FCAR, 5-FUR | 0.25 |
| | *Fusarium* sp. BNCC 340967 | CPFR, FCAR, 5-FUR | 1 |
| Medium priority | *Candida krusei* BNCC 185429 | CPFR, FCAR, 5-FUR, TBFR | 2 |
| | *Cryptococcus gattii* BNCC 354891 | FCAR, 5-FUR, TBFR | 0.25 |

| | | | |
|---|---|---|---|
| Note: CPFR, Caspofungin resistance; FCAR, Fluconazole resistance; TBFR, Terbinafine resistance; 5-FUR, 5-Fluorocytosine resistance; AmpR, Amphotericin B resistance. All MIC values were measured in duplicate and repeated three independent times with consistent results. | | | |

In addition, under the same experimental conditions described above, minimum inhibitory concentrations (MICs) of the natural product mandimycin against multidrug-resistant fungi, including *Candida albicans* and *Candida auris,* were 1 to 2 times the clinically used nystatin A1 and were comparable to those of amphotericin B, falling within a range of 0.25-1 µg/mL. Its activity against multidrug-resistant *Cryptococcus* neoformans reached 0.125 µg/mL, which was 32-fold more potent than caspofungin, 64-fold more potent than fluconazole, and 128-fold more potent than 5-fluorocytosine. Its activity against multidrug-resistant *Aspergillus* fumigatus reached 2 µg/mL, which was 32-fold more potent than fluconazole and 16-fold more potent than 5-fluorocytosine. Mandimycin also exhibited favorable antifungal activity against amphotericin B-resistant strains.

### (2) Cytotoxicity of mandimycin

The cytotoxicity of mandimycin was determined using the MTT (3-(4,5-dimethylthiazol-2-yl)-2,5-diphenyltetrazolium bromide) assay. HepG2 cells cultured in DMEM (containing 10% fetal bovine serum) were seeded into 96-well flat-bottom microplates (2,500 cells per well) and incubated at 37°C in an atmosphere containing 5% CO₂. After 24 h, the culture medium was removed, and 100 µL of fresh medium containing serial concentrations of mandimycin (with a final DMSO concentration of less than 0.25%) was added. Following incubation at 37°C for 48 h, the culture medium was removed, and 110 µL of MTT solution (10 µL of 5 mg/mL MTT premixed with 100 µL of DMEM in mandimycin) was added to each well. After incubation at 37°C in 5% CO₂ for 3 h, 100 µL of solubilization solution (40% DMF, 16% SDS, and 2% aqueous acetic acid) was added to dissolve the precipitate. Subsequently, an absorbance of each well at OD₅₇₀ nm was then measured using a microplate reader (Epoch microplate spectrophotometer, BioTek). Amphotericin B was used as a positive control. The IC₅₀ value (calculated using GraphPad Prism 7.0) was defined as a concentration of each compound required to inhibit cell growth by 50% relative to the compound-free control. Mandimycin exhibited IC₅₀ values of 57.56 µM against HepG2 cells, 88.67 µM against HK-2 cells, 48.28 µM against PANC-1 cells, and 76.70 µM against SK-Hep-1 cells. As shown in FIG. 15, mandimycin exhibited low cytotoxicity.

### (3) Hemolytic activity of mandimycin

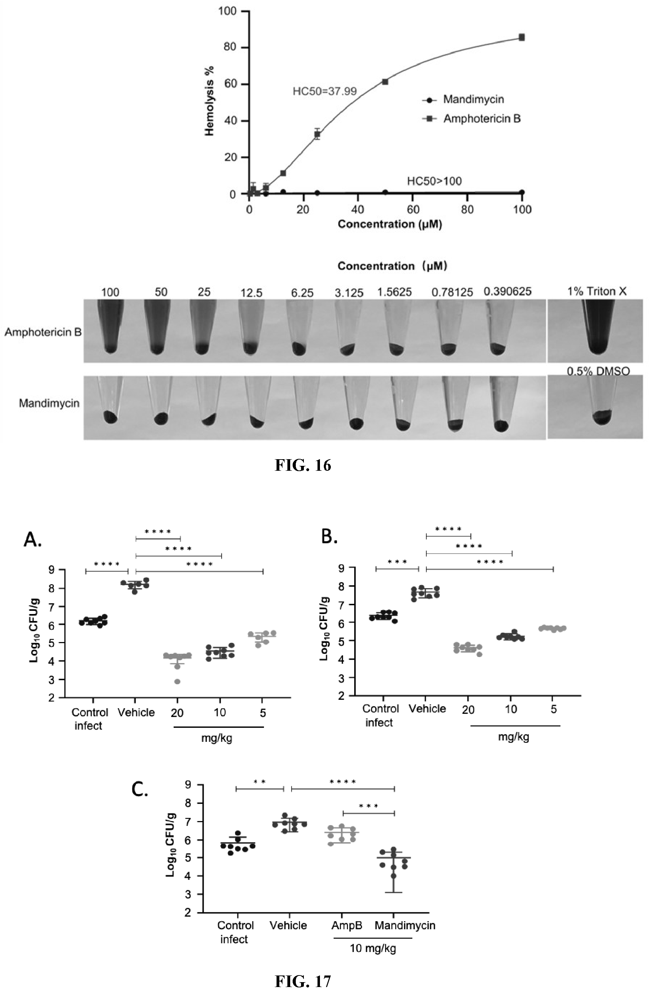

The hemolytic activity of mandimycin was evaluated according to a previously reported method (Kelvin JY Wu et al., 2024, Science, 383(6684):721-726). Fresh sterile defibrinated sheep blood was centrifuged at 3,000 rpm for 10 min at 4°C to separate the blood cells. The blood cells were then resuspended in PBS (pH 7.4) to prepare a suspension at a concentration of 1 × 10⁹ cells/mL. Test compounds at concentrations ranging from 0.39 µM to 100 µM were prepared and mixed with the suspension to a final volume of 500 µL. DMSO (0.5%) and Triton X-100 (1%) were used as negative (0% hemolysis) and positive (100% hemolysis) controls, respectively. After incubation at 37°C for 3 h, the samples were centrifuged at 3,000 rpm for 20 min. Supernatants were collected and transferred to 96-well polypropylene plates. The absorbance of the supernatants at OD540 nm was measured using an enzyme-linked immunosorbent assay (ELISA) reader to determine the extent of hemolysis. As shown in FIG. 16, the results indicated that mandimycin exhibited no hemolytic activity even at a high concentration of 100 µM, whereas amphotericin B caused severe hemolysis at 12.5 µM, proving that mandimycin possesses improved safety.

### Example 8

### Evaluation of in vivo antifungal activity of mandimycin

### (1) Mouse model of neutropenic thigh muscle infection

Female ICR mice, aged 6 weeks, weighing 23-27 g and free of specific pathogens, were used. Cyclophosphamide (Sigma-Aldrich) was administered intraperitoneally at 150 mg/kg and 100 mg/kg at 4 days and 1 day, respectively, prior to pathogen infection. On the day of infection, each mouse was inoculated intramuscularly in the thigh with 0.05 mL of inoculum, where the inoculum contained 2 × 10⁷ CFU/mL multidrug-resistant *Candida albicans* BNCC 186382, multidrug-resistant *Candida auris* BNCC 357785, or amphotericin B-resistant pan-drug-resistant *Candida auris* AMR05. Two hours after infection, mice were treated by subcutaneous injection of mandimycin prepared according to Example 3 at different doses (20 mg/kg, 10 mg/kg, and 5 mg/kg). The injections were administered every 8 h for a total of three injections over a treatment period of 24 h. The mice were then euthanized by cervical dislocation, and the numbers of fungal colonies in the thigh muscle tissues were determined. As shown in FIG. 17, the mandimycin-treated groups demonstrated significant therapeutic efficacy. Specifically, at a dose of 20 mg/kg, fungal burdens of *Candida albicans* BNCC 186382 and *Candida auris* BNCC 357785 were reduced by 4.2 log₁₀ CFU and 3 log₁₀ CFU, respectively. Even at a lowest dose of 5 mg/kg, fungal burdens of *Candida albicans* BNCC 186382 and *Candida auris* BNCC 357785 were reduced by 3 log₁₀ CFU and 2 log₁₀ CFU, respectively. These effects were superior to those observed with amphotericin B in the corresponding treatment groups. Against amphotericin B-resistant pan-drug-resistant *Candida auris*, mandimycin exhibited favorable *in vivo* antifungal activity. At a dose of 10 mg/kg, fungal burdens of *Candida auris* AMR05 were reduced by 2.4 log₁₀ CFU. Furthermore, in both mouse infection models established using the two strains, no acute toxicity was observed in the mice treated with mandimycin at the dose of 20 mg/kg.

### (2) Mouse model of disseminated candidiasis

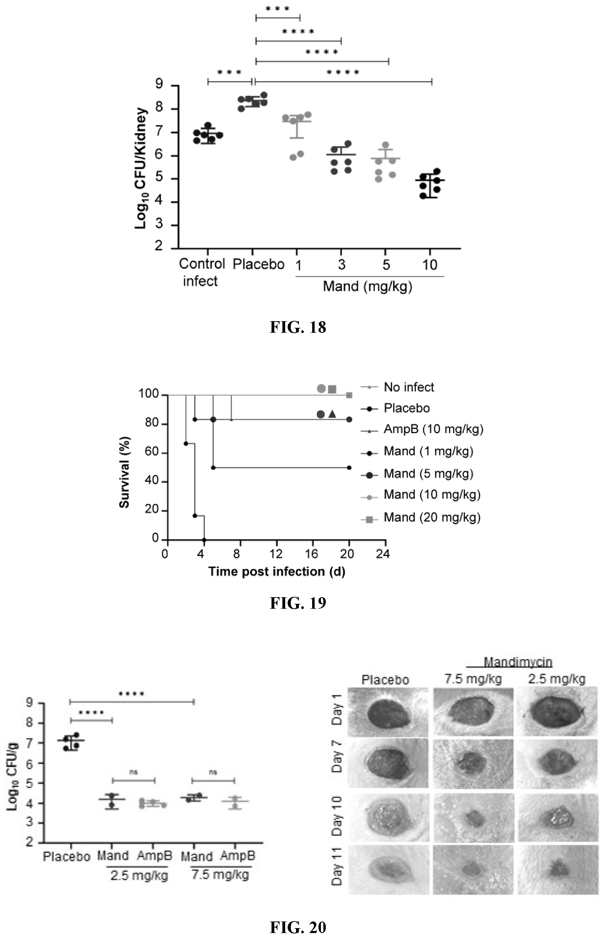

Specific pathogen-free female ICR mice (Hangzhou Medical College, China), aged 6 weeks and weighing 23-27 g, were used. The mice were randomly housed, with four mice per cage, and were acclimated for three days prior to the experiment. A single colony of *Candida albicans* BNCC 186382 was inoculated into 5 mL of YPD liquid medium and cultured overnight at 30°C with shaking at 220 rpm. The overnight fungal culture was washed three times with sterile 0.9% saline and then diluted to a final concentration of 2 × 10⁷ CFU/mL. Subsequently, the mice were injected with 50 µL of a diluted fungal suspension via tail vein to establish a systemic infection model at an inoculum of approximately 1 × 10⁷ CFU. Six hours after infection, mice received a single subcutaneous injection of mandimycin (formulated in 10% DMSO and 10% Tween 80) at doses of 1 mg/kg, 3 mg/kg, 5 mg/kg, or 10 mg/kg, respectively. Mice in the oral administration group received mandimycin orally at a dose of 10 mg/kg, formulated in a solution containing 5% DMSO and 10% Tween 80. 24 hours after infection, the mice were euthanized. Kidneys and lungs of the mice were aseptically harvested, weighed, homogenized, and plated on YPD agar for incubation at 30°C. Fungal burdens were determined by colony-forming unit (CFU) counting. All graphical data were plotted as individual data points for each group. As shown in FIG. 18, treatment with mandimycin at doses of 1, 3, 5, and 10 mg/kg reduced the fungal burdens of multidrug-resistant (MDR) *Candida albicans* in kidney tissues by 0.93, 2.34, 2.52, and 3.43 log₁₀ CFU, respectively, within 24 h. Statistical analyses were performed using GraphPad Prism 9.

### (3) Mouse model of invasive candidiasis

Specific pathogen-free female ICR mice (Hangzhou Medical College, China), aged 6 weeks and weighing 23-27 g, were used. The mice were randomly housed, with six mice per cage, and were acclimated for three days prior to the experiment. To induce immunosuppression, the mice received intraperitoneal injections of cyclophosphamide (200 mg/kg) and subcutaneous injections of cortisone acetate (500 mg/kg) on Day -2 and Day +3, respectively. To prevent cross-infection, the mice received enrofloxacin in drinking water at a concentration of 50 µg/mL from Day 1 to Day 3, followed by subcutaneous injection of ceftazidime (200 µg/kg) from Day 0 to Day 9. Invasive candidiasis was induced by intravenous injection of 1 × 10⁶ CFU of *Candida albicans* BNCC 186382. Treatment was initiated 16 h after infection, including subcutaneous injection of mandimycin (formulated in 10% DMSO and 10% Tween 80) at doses of 1 mg/kg, 5 mg/kg, 10 mg/kg, and 20 mg/kg once daily for four consecutive days, together with amphotericin B at a dose of 10 mg/kg. The mice were monitored for a total of 20 days. Survival curves were plotted using GraphPad Prism 9. The results are shown in FIG. 19. When mandimycin was administered at a dose of 10 mg/kg, a survival rate of mice reached 100%. In contrast, the survival rate of the mice in the group treated with amphotericin B was only 80%, indicating that mandimycin exhibited superior efficacy in improving the survival rate.

### (4) Mouse model of fungal skin infection

A mouse skin infection model was established to evaluate the efficacy of mandimycin in the treatment of fungal skin infections. BALB/c mice weighing 20-22 g (Hangzhou Medical College, China) were used. The mice were randomly housed, with four mice per cage, and were acclimated for three days prior to the experiment. Neutropenia was induced by intraperitoneal administration of cyclophosphamide at a dose of 50 mg/kg on Day -3 and Day -1 prior to infection. Subsequently, the mice were anesthetized via intraperitoneal injection of sodium pentobarbital at a dose of 50 mg/kg, and full-thickness skin wounds were created on the dorsal skin using a biopsy punch with a diameter of 0.8 cm. A suspension of *Candida albicans* BNCC 186382 (1 × 10⁸ CFU/mL, 50 µL per mouse) was inoculated into the circular wound, followed by gentle blowing until the skin appeared moist without excess liquid remaining. One day after infection, the wounds were treated topically with mandimycin (2.5 mg/kg or 7.5 mg/kg), amphotericin B (2.5 mg/kg or 7.5 mg/kg), or vehicle (PBS containing 10% DMSO and 10% Tween 80). The mice with created wounds that were not infected with fungi served as a negative control group and received drug treatment only. All compounds were administered once daily for five consecutive days. On Days 1, 5, 9, and 11 after infection, photographs of the wounds were taken and wound sizes were measured. On Day 11, fungal counts in wound specimens were determined, and wound specimens were collected. As shown in FIG. 20, mandimycin demonstrated significant activity in the skin infection model. At a dose of 2.5 mg/kg, the fungal burden was reduced by more than 2 log₁₀ CFU, and wound size and inflammation were also markedly alleviated.

### (5) Mouse model of vaginal infection

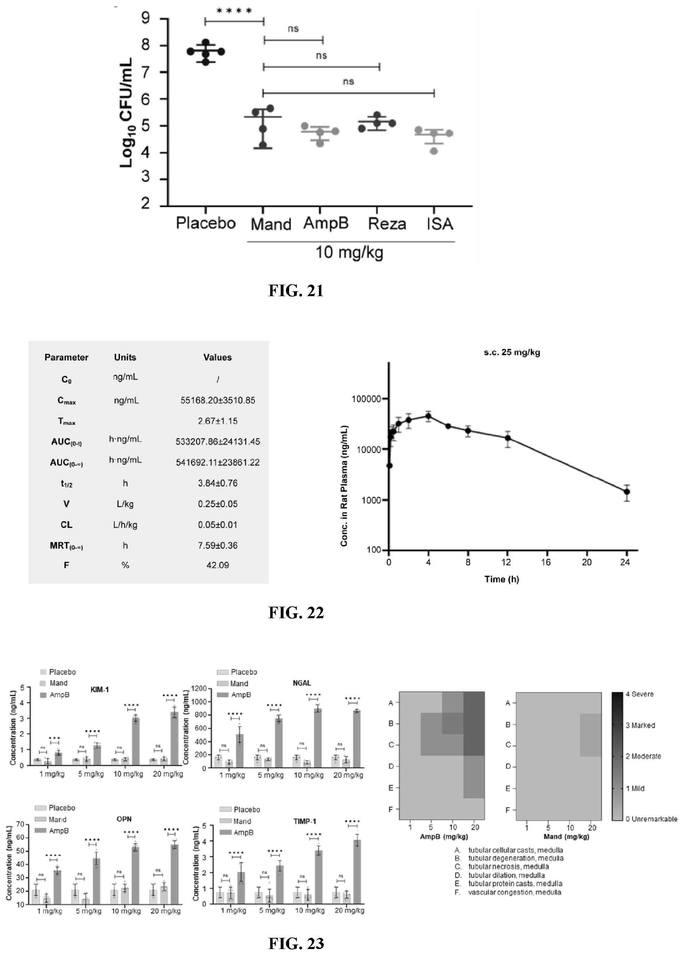

Female BALB/c mice, weighing 19-21 g, were acclimated for three days prior to the experiment. The mice were randomly housed, with four mice per cage, and were acclimated for three days prior to the experiment. On Day 1, the mice received subcutaneous injections of estradiol benzoate at a dose of 10 mg/kg once daily for five consecutive days to induce estrus. On Day 6, 50 µL of a suspension of *Candida albicans* BNCC 186382 (1 × 10¹⁰ CFU/mL) was inoculated intravaginally into each mouse using a pipette. Following vaginal inoculation, the mice were maintained in an inverted position for 5 min. After three consecutive days of infection, the mice were maintained under normal feeding conditions for one day and subsequently received subcutaneous injections of mandimycin (10 mg/kg), amphotericin B (10 mg/kg), rezafungin (10 mg/kg), or isavuconazole (10 mg/kg) once daily for five consecutive days. The mice infected with *Candida albicans* but not treated with the compounds served as a control group and were injected with PBS containing 10% DMSO and 10% Tween 80. On the day following the final administration, the vaginae of the mice were repeatedly lavaged with sterile PBS (20 µL), and the vaginal lavage fluids were aspirated using pipettes. The samples were cultured on MRS agar plates for enumeration of *Candida albicans* colonies. Finally, all animals were anesthetized and euthanized using diethyl ether, and vaginal tissues were collected. As shown in FIG. 21, mandimycin also exhibited significant efficacy in the treatment of vaginal candidiasis. After 5 days of treatment (10 mg/kg, subcutaneous injection, once daily), the vaginal fungal burden was significantly reduced by 2.51 log₁₀ CFU. This efficacy was comparable to that of other well-known antifungal antibiotics (including amphotericin B, rezafungin, and isavuconazole). In addition, inflammation was markedly alleviated in mice treated with mandimycin, and the vaginal mucosa was almost completely restored.

As demonstrated by Examples 7-8, the natural product mandimycin of the present invention not only exhibits excellent antifungal activities both *in vitro* and *in vivo*, but also shows no hemolytic activity or acute toxicity, making it suitable for the preparation of antifungal drugs.

### Example 9

### In vivo pharmacokinetic study of mandimycin

The study was conducted using specific pathogen-free male Sprague-Dawley rats (aged 7-8 weeks and weighing 180-220 g; n = 3 per group). The rats were randomly housed, with three rats per cage, and were acclimated for three days prior to the experiment. The rats received a subcutaneous injection of mandimycin at a dose of 25 mg/kg. Blood samples (approximately 0.15 mL) were collected via a jugular vein catheter into tubes containing sodium heparin at 5, 10, 20, and 30 min before administration, and at 1, 2, 4, 6, 8, 12, and 24 h after administration. Following the collection of blood samples, the samples were placed on ice and centrifuged (8,000 × g, 5 min) to separate plasma. The plasma was then transferred and immediately frozen (-70°C or below) until analysis. Mandimycin concentrations in rat plasma were analyzed by liquid chromatography-electrospray ionization-tandem mass spectrometry (LC-ESI-MS/MS), which consists of an AB SCIEX Triple Quad 6500 system coupled to an HPLC system equipped with a Quaternary Solvent Manager-R solvent delivery module and a Sample Manager FTN-R autosampler. Diazepam was used as the internal standard (IS). Quantitative analysis was performed using multiple reaction monitoring (MRM) in positive ion mode by monitoring the transition of mandimycin from m/z 1198.30 to m/z 725.20 and the transition of diazepam from m/z 285.00 to m/z 193.00. Mandimycin and the IS were separated by high-performance liquid chromatography using a Waters ACQUITY C18 column (1.9 µm, 100 × 2.1 mm). An isocratic mobile phase consisting of 80% acetonitrile and 20% (v/v) 5 mM ammonium acetate was delivered through the electrospray ionization source of the mass spectrometer at a flow rate of 0.4 mL/min for 3 min. The plasma concentration-time profile was fitted using GraphPad Prism 9. Values of maximum plasma concentration (Cₘₐₓ), time to reach Cₘₐₓ (tₘₐₓ), apparent elimination half-life (t_{1/2}), mean residence time (MRT), area under the plasma concentration-time curve (AUC), clearance (CL), and volume of distribution (V) were estimated by noncompartmental analysis using Phoenix WinNonlin 8.3. Bioavailability was calculated according to the following equation: (AUCs.c./AUCi.v.) × (Dosei.v./Doses.c.)×100% The results are shown in FIG. 22. Mandimycin exhibited a favorable pharmacokinetic profile, with a half-life of 3.84 h, a maximum concentration (Cₘₐₓ) of 55,168.20 ng/mL, and an area under the curve (AUC0-∞) of 541,692.11 h•ng/mL. These *in vitro* and *in vivo* studies jointly demonstrate that mandimycin possesses a broad therapeutic window.

### Example 10

### In vivo nephrotoxicity evaluation of mandimycin

Specific pathogen-free female ICR mice (Hangzhou Medical College, China), aged 6 weeks and weighing 23-27 g, were used. The mice were randomly housed and divided into 9 groups, with 4 mice in each group. The mice were acclimated for three days prior to the experiment. Amphotericin B and the vehicle without any antibiotics were used as a positive control and placebo, respectively. The compound mandimycin was formulated in a solution containing 10% DMSO and 10% Tween 80. Subsequently, the mice in each group received subcutaneous injections of the compound or placebo at doses of 1 mg/kg, 5 mg/kg, 10 mg/kg, or 20 mg/kg (amphotericin B and mandimycin) once daily. Concentrations of toxicity-related biomarkers were then determined using commercially available assay kits (Cloud-Clone Corp., China) according to the protocols provided by the manufacturer. The biomarkers included kidney injury molecule-1 (KIM-1), tissue inhibitor of metalloproteinase-1 (TIMP-1), neutrophil gelatinase-associated lipocalin (NGAL), and osteopontin (OPN). Finally, all mice were euthanized, and kidney tissues were collected, fixed, dissected, and subjected to H&E staining. Histopathological changes, including renal tubular degeneration, necrosis, cellular casts, dilation, congestion, and protein casts, were evaluated and scored in a blinded manner by clinical pathologists. As shown in FIG. 23, following administration of mandimycin, the nephrotoxicity-related indicators were comparable to those of the placebo group, whereas the nephrotoxicity markers in the group treated with amphotericin B were markedly increased. Histopathological scoring also demonstrated that mandimycin caused virtually no renal injury. All animal experimental procedures were approved by the Animal Ethics Committee of China Pharmaceutical University.

### Example 11

### Study on the mechanism of action of mandimycin

### (1) Drug resistance test of mandimycin

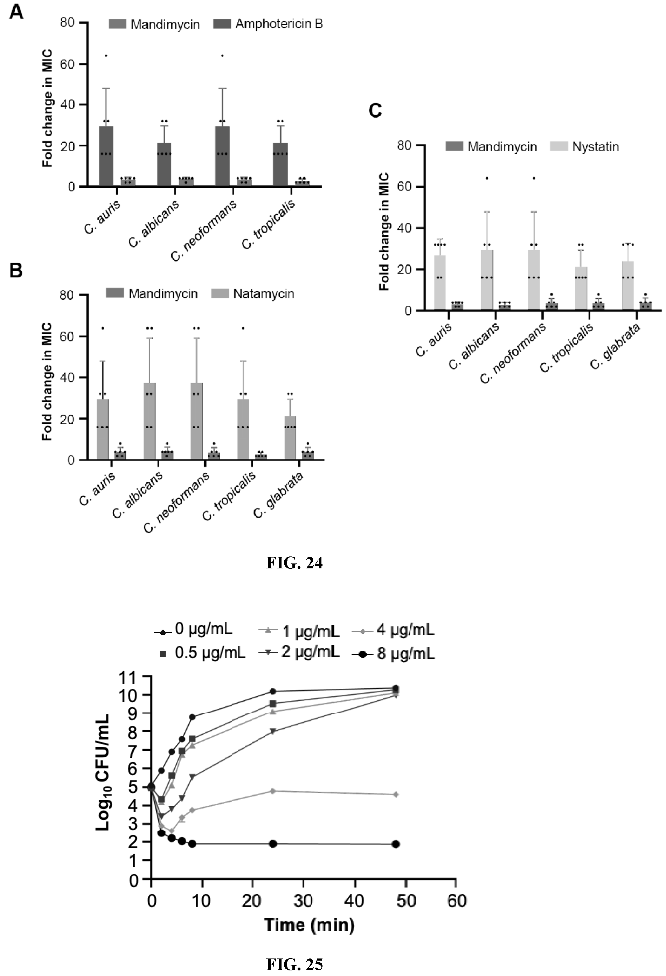

Different *Candida* species and other fungi were cultured overnight in YPD broth with shaking (200 rpm, 30°C) for 16 h. A fungal suspension having a concentration of approximately 10¹⁰ CFU/mL was prepared, and 0.1 mL aliquot of the suspension was spread onto YPD agar plates containing 8× MIC concentrations of mandimycin, amphotericin B, nystatin, or natamycin. The inoculated plates were incubated at 30°C for 2 days to identify resistant colonies. A number of colonies growing in the presence of the antifungal agents was calculated. As shown in FIG. 24, mandimycin did not generate resistant strains. In contrast, amphotericin B produced an average of 6 resistant *Candida auris* colonies, 5 resistant *Candida albicans* colonies, 12 resistant *Cryptococcus* neoformans colonies, 560 resistant *Candida tropicalis* colonies, and 5 resistant *Candida parapsilosis* colonies per agar plate. Nystatin produced an average of 2 resistant *Candida auris* colonies, 24 resistant *Candida albicans* colonies, 3 resistant *Cryptococcus* neoformans colonies, 55 resistant *Candida tropicalis* colonies, 2 resistant *Candida parapsilosis* colonies, and 23 resistant *Candida glabrata* colonies per agar plate. Natamycin produced an average of 13 resistant *Candida auris* colonies, 4 resistant *Candida albicans* colonies, 34 resistant *Cryptococcus* neoformans colonies, 40 resistant *Candida tropicalis* colonies, 8 resistant *Candida parapsilosis* colonies, and 5 resistant *Candida glabrata* colonies per agar plate. The results indicate that, unlike clinically used polyene antifungal agents, mandimycin did not induce resistant strains even under induction conditions using high-density fungal inocula, suggesting that resistance is less likely to emerge during further potential clinical applications.

### (2) Time-kill curve of mandimycin

Fresh colonies of *Candida albicans* BNCC 186382 grown at 30°C and 220 rpm for 16 h were suspended in physiological saline and adjusted to an OD600 value = 1, corresponding to a fungal concentration of 6.6 × 10⁷ CFU/mL. The suspension was then diluted 66-fold to obtain a standardized suspension (1 × 10⁶ CFU/mL). The standardized suspension was diluted with YPD liquid medium and stock solutions of the test compounds to obtain an initial inoculum of approximately 10⁵ CFU/mL. Mandimycin was added to test tubes containing 4 mL of culture medium (initial inoculum: 10⁵CFU/mL) to achieve final concentrations of 0.5 µg/mL, 1 µg/mL, 2 µg/mL, 4 µg/mL, 8 µg/mL, and 16 µg/mL, respectively. The cultures were continuously incubated with shaking at 30°C and 220 rpm. At 0, 2, 4, 6, 8, 24, and 48 h, 0.1 mL aliquots of appropriately diluted samples were taken and spread onto three YPD agar plates. After incubation for 24 h, viable counts were determined by enumerating colony-forming units. Growth controls for each organism were prepared and assayed simultaneously in the absence of antifungal agents. As shown in FIG. 25, mandimycin is a potent fungicidal agent. At a high concentration (8 µg/mL), the cell count began to decline after 2 h, and *Candida* cells were completely eradicated within 8 h.

### (3) Scanning electron microscopy (SEM) observation of the morphology of Candida albicans treated with mandimycin

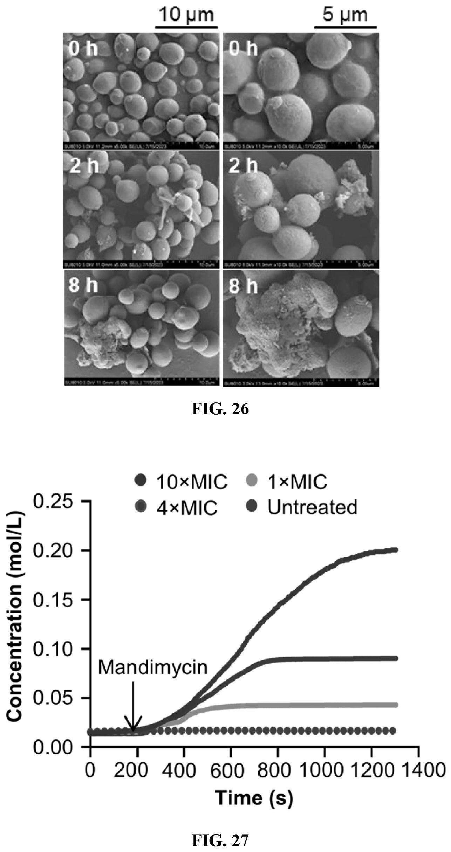

Samples of *Candida albicans* BNCC 186382 treated with mandimycin at 8 × MIC for 0 h, 4 h, and 8 h were selected for microscopic observation via SEM. As shown in FIG. 26, under the action of mandimycin, lysis of the fungal cell membrane was observed at 2 h, and complete collapse of the membrane occurred at 8 h.

### (4) Determination of changes in intracellular and extracellular ion concentration following mandimycin treatment

*Candida albicans* BNCC 186382 was cultured overnight in 50 mL of YPD liquid medium using a shaking incubator (200 rpm, 30°C). The cultures were washed three times with 10 mM Tris-acetate buffer (pH 7.4) containing 100 mM NaCl. The washed *Candida albicans* cells (6.6 × 10⁸ CFU/mL, OD₆₀₀ = 1.0) were resuspended in the buffer to prepare 20 mL of fungal suspension. Mandimycin was added to the suspensions at concentrations corresponding to 1 × MIC (0.5 µg/mL), 4 × MIC (2 µg/mL), and 10 × MIC (5 µg/mL), respectively. Changes in K⁺ concentrations were measured using an Orion SensorLink PCM-700 pH/ISE meter (with the electrode calibrated using standard solutions containing 0.01, 0.1, or 1.0 mM KCl in 100 mM NaCl). A control group was established by adding an equal volume of solvent without mandimycin to the fungal suspension, and changes in K⁺ concentrations were monitored. As shown in FIG. 27, *Candida albicans* treated with mandimycin began to release large amounts of K⁺, whereas no changes were observed in the control group. Moreover, the extent of ion efflux exhibited a dose-dependent relationship with the concentration of mandimycin.

### (5) Determination of the fungal cell membrane binding targets of mandimycin

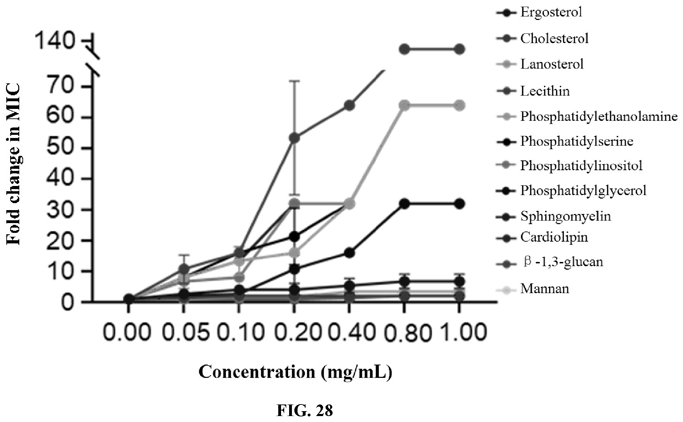

The effects of the addition of eukaryotic membrane phospholipid components (including phosphatidylinositol, phosphatidylserine, phosphatidylethanolamine, phosphatidylglycerol, lecithin, sphingomyelin, and cardiolipin), ergosterol and cell wall components β-1,3-glucan and mannan, on the antifungal activity of mandimycin were evaluated using *Candida albicans* BNCC 186382 and the broth dilution method. Various membrane components used in the experiments were dissolved in 10% DMSO to prepare the concentrations required for the experiment, followed by addition to the culture medium used for MIC determination of mandimycin. The effects of fungal cell membrane components on the MIC of mandimycin were then observed. As shown in FIG. 28, an ergosterol target of polyene macrolide antifungal antibiotics did not inhibit the antifungal activity of mandimycin. In addition, a β-1,3-glucan target of echinocandin, another class of antifungal agents, likewise did not inhibit the activity of mandimycin, suggesting that mandimycin may possess a different mechanism of action. Further studies revealed that small phospholipid molecules present in fungal cell membranes (including lecithin, cardiolipin, phosphatidylinositol, phosphatidylserine, phosphatidylethanolamine, sphingomyelin, and phosphatidylglycerol) strongly inhibited the activity of mandimycin in a dose-dependent manner. Specifically, as the concentration of lecithin increased, the fold-change in the MIC of mandimycin against *Candida albicans* increased from 1-fold to 128-fold. As the concentrations of cardiolipin, phosphatidylinositol, phosphatidylserine, phosphatidylethanolamine, and sphingomyelin increased, the fold-change in the MIC of mandimycin increased from 1-fold to 64-fold. As the concentration of phosphatidylglycerol increased, the fold-change in the MIC of mandimycin increased from 1-fold to 32-fold. This experiment demonstrated that, unlike known polyene natural products that act through the ergosterol target, mandimycin acts on small phospholipid molecules present in fungal cell membranes.

### (6) Determination of the targets of mandimycin using isothermal titration calorimetry (ITC)

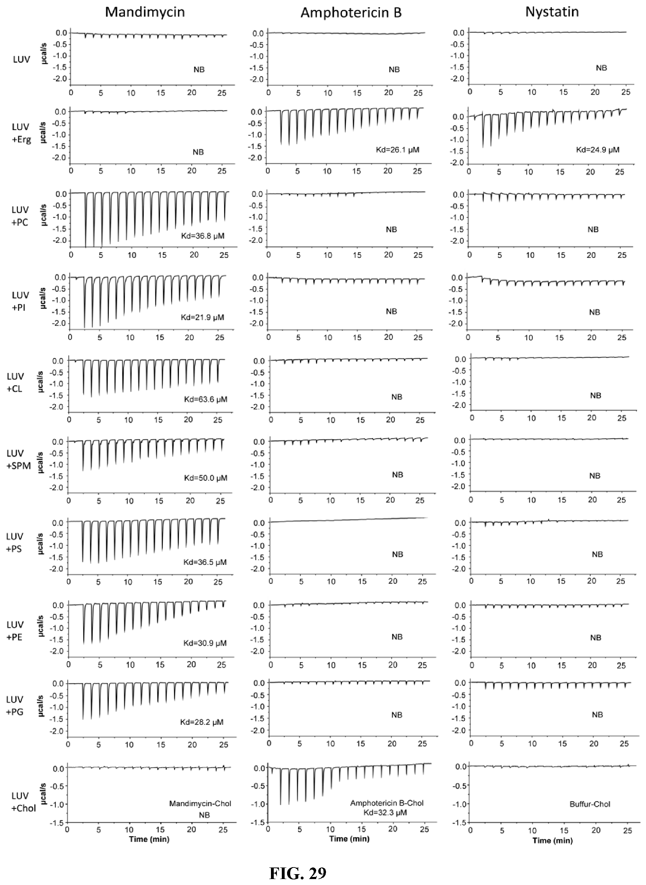

20 mM mandimycin and amphotericin B were diluted with 5.0 mM HEPES (pH = 7.4) containing 5% DMSO to a final concentration of 1 mM. A 600 µM mixture was prepared by dissolving various fungal membrane phospholipid components (lecithin, cardiolipin, phosphatidylinositol, phosphatidylserine, phosphatidylethanolamine, sphingomyelin, and phosphatidylglycerol) and ergosterol in 5.0 mM HEPES (pH = 7.4) containing 5% DMSO. Subsequently, DOPC phospholipid (Avanti Polar Lipids, 610014-1Ea) was hydrated with 5.0 mM HEPES (pH = 7.4), and 100 nm liposomes were prepared using an Avanti Mini Extruder. The heat release associated with binding between mandimycin and phospholipids was measured using a PEAQ-ITC isothermal titration calorimeter. At 25°C, 1 mM solution of mandimycin (40 µL) was loaded into an automatic injection syringe, and a liposome suspension (250 µL) containing 600 µM sample was placed in the sample cell. A volume of the initial injection was 0.23 µL. Subsequently, 18 injections of 2 µL each were performed at an interval of 80 s between injections, with a stirring speed of 500 rpm. As shown in FIG. 29 and Table 6, small phospholipid molecules exhibited favorable exothermic binding curves with mandimycin. Specifically, phosphatidylinositol exhibited the strongest binding affinity, with a *K*_{d} value of 21.9 µM; followed by phosphatidylglycerol, with a *K*_{d} value of 28.2 µM, phosphatidylserine, with a *K*_{d} value of 30.9 µM, phosphatidylethanolamine, with a *K*_{d} value of 36.5 µM, phosphatidylcholine, with a *K*_{d} value of 36.8 µM, sphingomyelin, with a *K*_{d} value of 50 µM, and cardiolipin, with a *K*_{d} value of 63.6 µM. In contrast, amphotericin B and nystatin did not bind to phospholipid components. The ITC experiments further confirmed that mandimycin does not bind to the conventional ergosterol target, instead, it strongly interacts with seven phospholipid molecules, especially phosphatidylinositol, through a novel targeting mechanism.

**Table 6 Affinity values between mandimycin and phospholipid components**

| Membrane components | *K*_{d} (µM) | | |
|---|---|---|---|
| | Mandimycin | Amphotericin B | Nystatin |
| LUV | NB | NB | NB |
| LUV+Ergosterol | NB | 26.1 | 24.9 |
| LUV+Phosphatidylcholine | 36.8 | NB | NB |
| LUV+Phosphatidylethanolamine | 30.9 | NB | NB |
| LUV+Phosphatidylserine | 36.5 | NB | NB |
| LUV+Phosphatidylinositols | 21.9 | NB | NB |
| LUV+Cardiolipin | 63.6 | NB | NB |
| LUV+Sphingolipid | 50 | NB | NB |
| LUV+phosphatidylglycerol | 28.2 | NB | NB |

As demonstrated in Example 11, the natural product mandimycin of the present invention has a mechanism of action distinct from those of known polyene antifungal agents. Mandimycin acts by specifically binding to seven phospholipid molecules, especially phosphatidylinositol, present in fungal cell membranes, thereby leading to the efflux of essential intracellular ions and ultimately resulting in the death of fungal pathogens. This specific binding mode not only confers potent, broad-spectrum antifungal activity against multidrug-resistant fungal pathogens, but also contributes to its low propensity for the development of resistance. Owing to its novel trisaccharide-containing polyene scaffold, potent and broad-spectrum activity against drug-resistant fungi, novel mechanism of action, and favorable resistance profile, mandimycin shows promise for development into a next-generation antifungal agent.

### Example 12

### Construction of the MandQ-knockout engineered strain CPU002

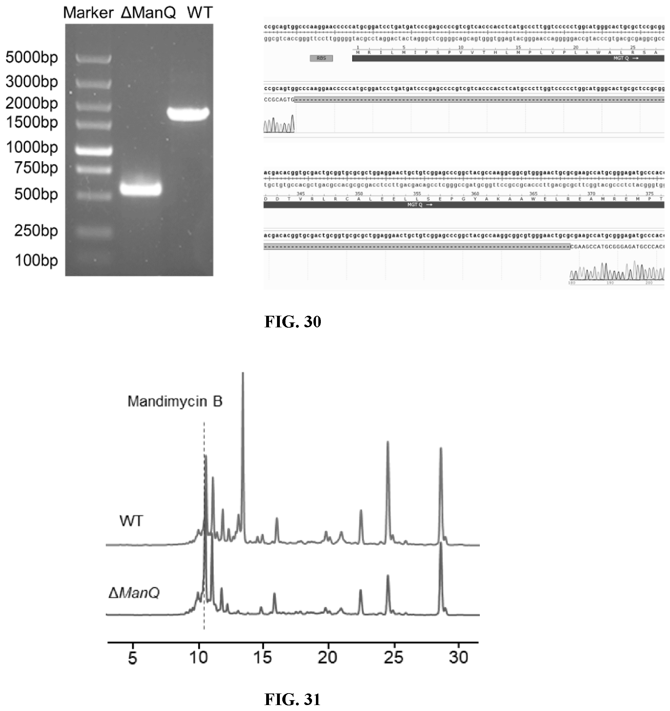

In order to obtain a mandimycin derivative lacking atratcynose A, a bioinformatic analysis was performed on the mandimycin biosynthetic gene cluster in *Streptomyces netropsis* DSM 40259, identifying *MandQ* (SEQ ID NO. 2) as playing a critical role in the formation of atratcynose A. To knock out *MandQ*, genomic DNA from *Streptomyces netropsis* DSM 40259 was used as a template. Upstream and downstream fragments of *MandQ* were amplified by PCR using primer pairs *MandQ*_KOUF/R and *MandQ*_KODF/R, respectively, and these fragments were cloned into the XbaI-digested pKC1139 vector to obtain plasmid pKC1139-*MandQ*_KO. The resulting vector was introduced into *Streptomyces netropsis* DSM 40259 by intergeneric conjugation between *Streptomyces* and *Escherichia coli* to knock out *MandQ*. The knockout strain was verified using *MandQ*_TestF/R primers. As shown in FIG. 30, a 1072-bp fragment of *MandQ* was successfully deleted, thereby yielding the *MandQ*-knockout engineered strain CPU002. A comparative analysis of secondary metabolites from the wild-type and knockout strains was performed. As shown in FIG. 31, it is confirmed that *MandQ* was successfully knocked out, and a novel conjugated pentaene glycosylated natural product was generated, which was designated mandimycin B.

### Primer sequences:

*MandQ*_KOUF: gggctgcaggtcgactcacacccgaatcgaccact;
*MandQ*_KOUR: atggcttcgacggggctcgggatcatcag;
*MandQ*_KODF: gccccgtcgaagccatgcgggagatg;
*MandQ*_KODR: cgcggccgcggatcctcgggcagtcatcacaccatc;
*MandQ*_TestF: cgacgagtccatggtccg;
*MandQ*_TestR: cgccgatgtccaggatcac.

### Example 13

### Biological fermentation using the engineered strain

### (1) Preparation of seed culture:

50 mL of TSB medium (containing 17.0 g pancreatic digest of casein, 3.0 g enzymatic digest of soybean, 5.0 g sodium chloride, 2.5 g dipotassium hydrogen phosphate, and 2.5 g glucose monohydrate; pH = 7.3 per liter of ddH₂O) was added in to a 250-mL baffled Erlenmeyer flask. The engineering strain CPU002 cultured on ISP4 agar plates (containing 10.0 g soluble starch, 1.0 g dipotassium hydrogen phosphate, 1.0 g magnesium sulfate, 1.0 g sodium chloride, 2.0 g ammonium sulfate, 2.0 g calcium sulfate, 0.001 g ferrous sulfate, 0.001 g manganese chloride, 0.001 g zinc sulfate, and 15.0 g agar per liter of ddH₂O; pH = 7.2) was inoculated into the TSB medium and incubated in a shaking incubator (200 rpm, 30°C) for 2 days to prepare the seed culture.

### (2) Preparation of fermentation broth:

A 0.5 mL aliquot of the seed culture was transferred into 50 mL of FS/9 fermentation medium (containing 40.0 g glucose, 30.0 g soybean meal, and 10.0 g CaCO₃ per ddH₂O) contained in a 250-mL baffled Erlenmeyer flask, and fermented for 5 days (200 rpm, 30°C). After completion of fermentation, an equal volume of *n*-butanol was added to the fermentation broth, followed by extraction overnight with stirring at 100 rpm. An *n*-butanol extract was concentrated under reduced pressure using a rotary evaporator and dissolved in methanol to obtain a crude extract.

### Example 14

### Isolation and purification of mandimycin B

Purification was performed using the two-step procedure. First, the crude extract obtained in Example 13 was subjected to isocratic elution column chromatography using a YMC-GEL C18 powder column (12 nm × 50 µm). The column was equilibrated with 2 column volumes (CV) of 10% methanol (H₂O:CH₃OH = 9:1). A methanolic solution was then loaded onto the column to elute using gradient elution (10%, 30%, 50%, 70%, 90%, and 100%), with each fraction collected in 100 mL portions. The fractions were analyzed using UPLC-MS, and fractions containing mandimycin (an eluate obtained with 90% aqueous methanol) were collected and evaporated to dryness. The UPLC-MS conditions were as follows: a C18 column (Waters, T3, 1.8 µm, 2.1 × 100 mm); mobile phase A-pure water (0.1% formic acid); mobile phase B-acetonitrile (0.1% formic acid); with a flow rate of 0.6 mL/min; a gradient of mobile phase B from 30% to 90% at a rate of 10% per minute. An MS detection range was 200-2,000, with simultaneous operation in both positive and negative ion modes. A semi-purified product was then further purified using semi-preparative reversed-phase high-performance liquid chromatography (RP-HPLC). The RP-HPLC conditions were as follows: solvent A, deionized water; and solvent B, acetonitrile. A flow rate was 3 mL/min. A gradient of solvent B increased from 30% to 90% at a rate of 1.5% per minute, and full-wavelength detection (190-800 nm) was performed. Fractions containing mandimycin B with a purity greater than 95% were collected based on UV analysis. A yield of mandimycin B was 3-4 mg/L of fermentation broth.

### Example 15

### Structural elucidation of mandimycin B

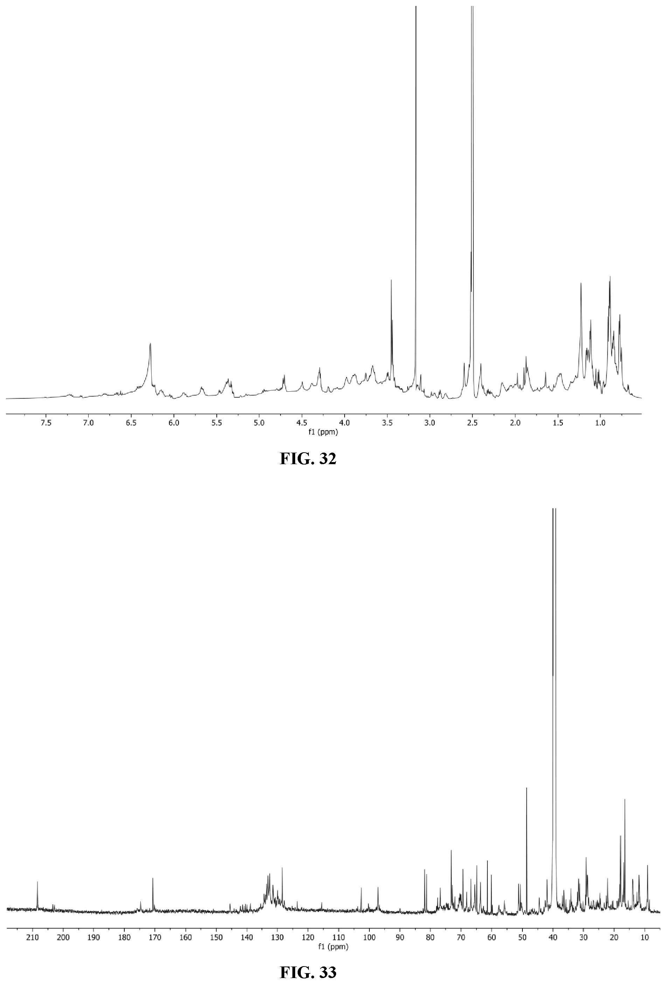

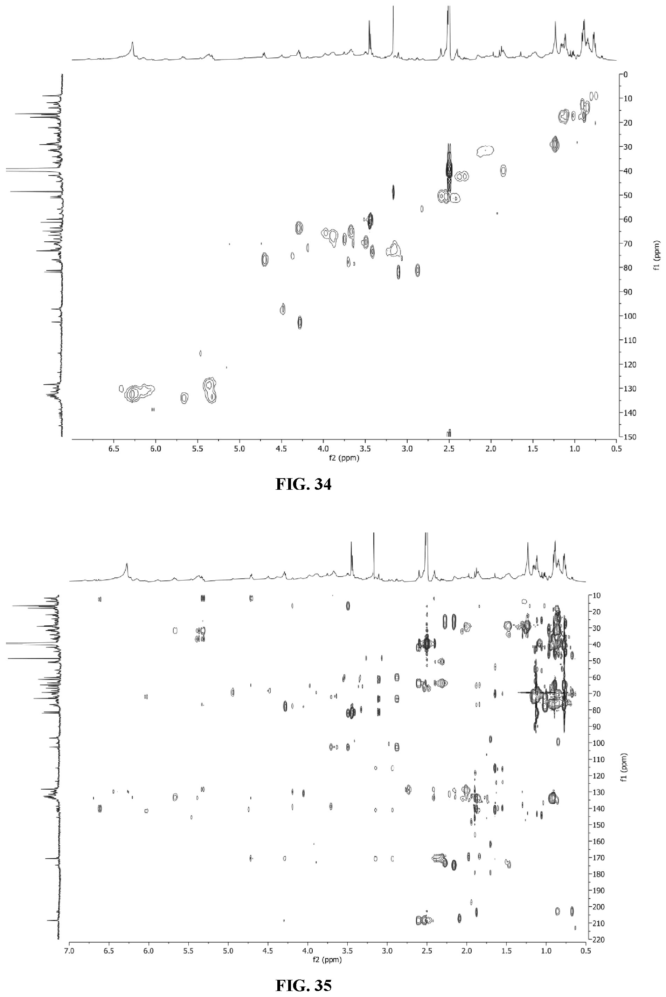

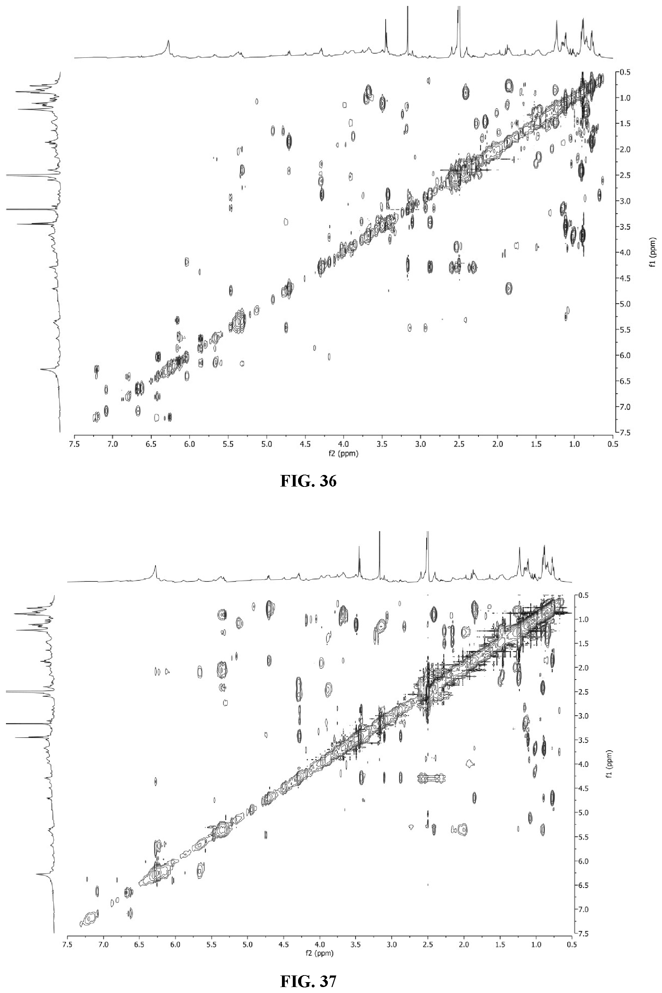

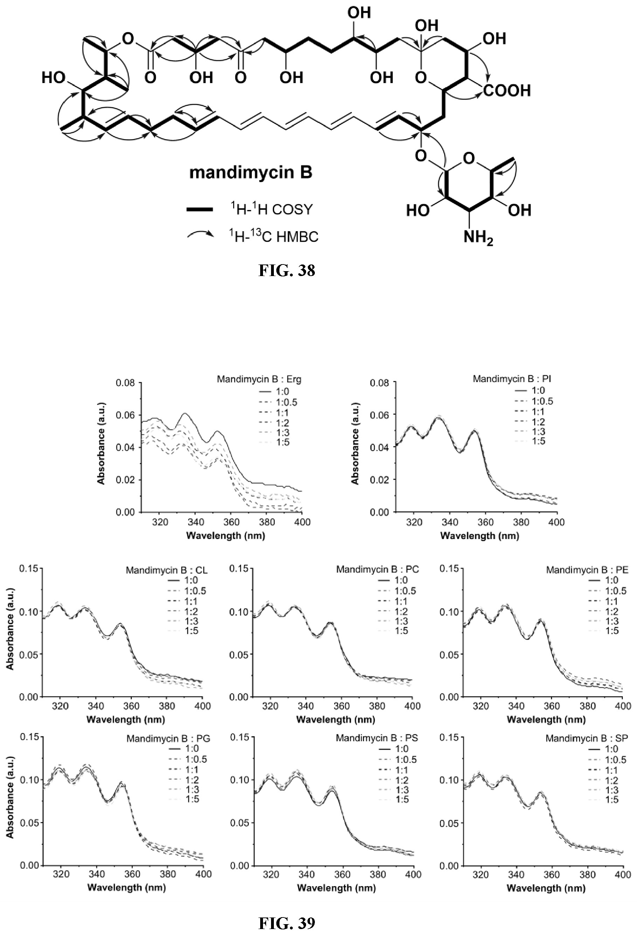

The ultraviolet-visible spectrum of mandimycin B in methanol exhibits maximum absorption wavelengths at 320, 335, and 352 nm, indicating the presence of a conjugated pentaene structure. HR-ESI-MS analysis revealed a protonated molecular ion at m/z 924.4957 [M+H]⁺ and a deprotonated molecular ion at m/z 922.4792 [M-H]⁻, indicating a molecular formula of C₄₇H₇₃NO₁₇. The ultraviolet-visible spectrum of mandimycin in methanol exhibits maximum absorption wavelengths at 320, 335, and 352 nm, indicating the presence of a conjugated pentaene structure. The mandimycin B was dissolved in deuterated DMSO and analyzed using the 700 MHz nuclear magnetic resonance spectrometer to obtain ¹H NMR, ¹³C NMR spectra, and 2D NMR spectra including HSQC, HMBC, COSY, TOCSY, and the like, as shown in FIGS. 32-37. A comparison of the 1D and 2D NMR data of mandimycin B with those of mandimycin revealed that the principal difference lies in the absence of resonances corresponding to dimethoxysugar and 3-*O*-methyldimethoxysugar moieties, which was consistent with the fact that mandimycin B was produced by the glycosyltransferase gene with the strain CPU002 knocked out. The significant upfield shifts of C-35 (Δδ_{C} -7.3) and C-37 (Δδ_{C} -5.6) further supported this conclusion. In addition, comparison of the ¹H-NMR and ¹³C-NMR data of the mycosamine moiety in mandimycin and mandimycin B revealed nearly identical chemical shifts, indicating that the mycosamine moiety was retained in mandimycin B. Key HMBC and COSY correlations of mandimycin B are shown in FIG. 38, thereby establishing a structure of mandimycin B. ¹H-NMR (700 MHz, DMSO-*d*₆), ¹³C-NMR (700 MHz, DMSO-*d*₆), HMBC, HSQC, and COSY data of mandimycin B are summarized in Table 7.

**Table 7 ¹H NMR and ¹³C NMR data (DMSO-d₆) of mandimycin B**

| Pos. | δ_{C}, type* | δ_{H}, mult. (*J* in Hz)^{#} | Pos. | δ_{C}, type* | δ_{H}, mult. (*J* in Hz)^{#} |
|---|---|---|---|---|---|
| Polyene macrolide | | | | | |
| 1 | 170.7, C | | 25 | 129.9-134.4, CH | 6.08-6.28 |
| 2 | 41.8, CH₂ | 2.30; 2.38 | 26 | 129.9-134.4, CH | 6.08-6.28 |
| 3 | 63.8, CH | 4.30 | 27 | 129.9-134.4, CH | 6.08-6.28 |
| 4 | 50.7, CH₂ | 2.53; 2.60 | 28 | 130.8, CH | 6.13 |
| 5 | 208.4, C | | 29 | 134.3, CH | 5.66 |
| 6 | 51.3, CH₂ | 2.43; 2.47 | 30 | 32.2, CH₂ | 2.13 |
| 7 | 66.7, CH | 3.92 | 31 | 31.3, CH₂ | 2.02; 2.11 |
| 8 | 33.6, CH₂ | 1.32; 1.49 | 32 | 128.4, CH | 5.38 |
| 9 | 28.5, CH₂ | 1.36; 1.43 | 33 | 133.6, CH | 5.33 |
| 10 | 66.7, CH | 3.89 | 34 | 36.8, CH | 2.41 |
| 11 | 65.7, CH | 3.98 | 35 | 76.8, CH | 4.71 |
| 12 | 44.5, CH₂ | 1.15; 1.90 | 36 | 40.2, CH | 1.85 |
| 13 | 97.2, C | | 37 | 64.8, CH | 3.67 |
| 14 | 42.7, CH₂ | 1.49; 1.70 | 16-COOH | 174.7, C | |
| 15 | 66.0, CH | 3.98 | 34-CH₃ | 16.9, CH₃ | 0.91 |
| 16 | 57.6, CH | 1.94 | 36-CH₃ | 8.9, CH₃ | 0.77 |
| 17 | 65.8, CH | 4.03 | 37-CH₃ | 16.9, CH₃ | 0.89 |
| 18 | 37.4, CH₂ | 1.65; 1.91 | Mycosamine | | |
| 19 | 74.8, CH | 4.36 | 1' | 97.3, CH | 4.49 |
| 20 | 135.0, CH | 5.87 | 2' | 68.2, CH | 3.76 |
| 21 | 131.0, CH | 6.16 | 3' | 56.0, CH | 2.91 |
| 22 | 129.9-134.4, CH | 6.08-6.28 | 4' | 73.3, CH | 3.14 |
| 23 | 129.9-134.4, CH | 6.08-6.28 | 5' | 71.6, CH | 3.20 |
| 24 | 129.9-134.4, CH | 6.08-6.28 | 6' | 17.9, CH₃ | 1.16 |

Based on analyses of the ¹H-NMR and ¹³C-NMR spectra, and 2D NMR spectra including HSQC, HMBC, COSY, and the like, together with bioinformatic analysis of the mandimycin B polyketide synthase, the specific configuration of the natural product mandimycin B of the present invention was determined to be 2*R*, 10*R*, 11*S*, 12*S*, 13*R*, 14*R*, 16*R*, and 18*R*. The natural product mandimycin is represented by formula II.

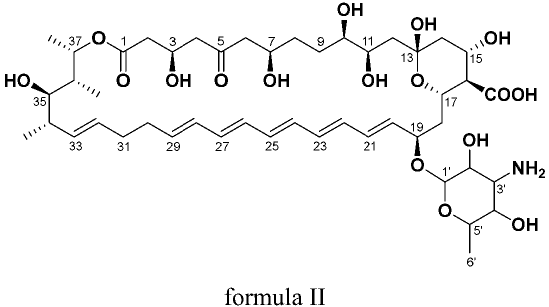

### Example 16

### In vitro activity assay against multidrug-resistant fungi of mandimycin B

The antifungal activity of mandimycin B against priority fungal pathogens listed by the WHO was evaluated using CLSI standard according to the method described in Example 7. The results are shown in Table 8.

**Table 8 Antifungal activity of mandimycin B**

| **Type** | **MDR Pathogens** | **MIC** (**µg/mL**) |
|---|---|---|
| Fungi | *Candida auris BNCC 357785* | 2 |
| | *Candida albicans BNCC 186382* | 1 |
| | *Cryptococcus neoformans BNCC 225501* | 0.5 |
| | *Aspergillus fumigatus BNCC 122691* | 2 |
| | *Candida krusei BNCC 185429* | 4 |
| | *Mucor sp. BNCC 195604* | 0.5 |
| | *Fusarium sp. BNCC 340967* | 1 |

As can be seen from Table 8, mandimycin B exhibits potent and broad-spectrum antifungal activity against multidrug-resistant fungi. Specifically, the minimum inhibitory concentrations (MICs) against *Candida* species, especially multidrug-resistant strains including *Candida albicans* and *Candida auris,* fall within a range of 1-2 µg/mL. Its activity against multidrug-resistant *Cryptococcus* neoformans reached 0.5 µg/mL. Its activity against multidrug-resistant *Aspergillus* fumigatus reached 2 µg/mL, which was 32-fold greater than fluconazole and 16-fold greater than 5-fluorocytosine.

### Example 17

### Mechanism of action of mandimycin B

### (1) Binding curves of mandimycin B with sterol molecules or phospholipid molecules

In this example, the binding of mandimycin B to sterol molecules or phospholipid molecules was investigated using a UV-vis method (Maji, A. Nature 2023, 623, 1079-1085). Mandimycin B was dissolved in DMSO. 1 mM mandimycin B was mixed with sterol solutions (cholesterol and ergosterol) or phospholipid solutions (lecithin, cardiolipin, phosphatidylinositol, phosphatidylserine, phosphatidylethanolamine, sphingomyelin, and phosphatidylglycerol) at various ratios to obtain a final volume of 1 mL. The mixed solutions were allowed to stand at room temperature for 30 min, followed by UV-Vis scanning (310-400 nm) using a FlexA-200 microplate reader. The obtained values were plotted using Origin software. The results are shown in FIG. 39. Phospholipids of various concentrations were mixed with mandimycin B, which did not affect the UV absorbance values of mandimycin B, indicating that different phospholipid molecules do not bind to mandimycin B. However, when sterols at different concentrations (including cholesterol and ergosterol) were mixed with mandimycin B, the UV absorbance values of mandimycin B changed significantly, indicating that mandimycin B binds to sterol molecules, including cholesterol and ergosterol.

### (2) Determination of the binding affinity of mandimycin B to sterol molecules or phospholipid molecules using ITC

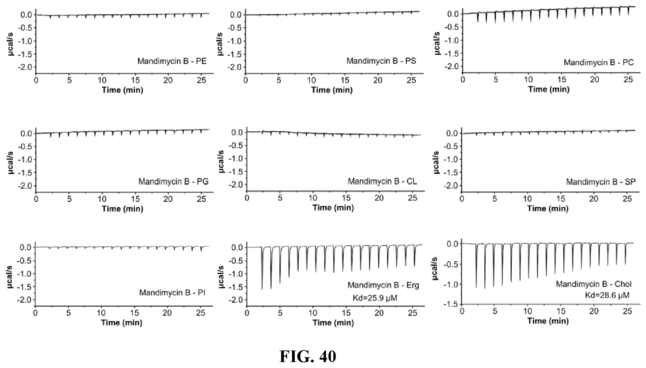

In this example, 20 mM mandimycin B (using DMSO as solvent) was diluted with 5.0 mM HEPES (pH = 7.4) containing 5% DMSO using the ITC to a final concentration of 1 mM. A 600 µM mixture was prepared by dissolving various phospholipid components (lecithin, cardiolipin, phosphatidylinositol, phosphatidylserine, phosphatidylethanolamine, sphingomyelin, and phosphatidylglycerol), ergosterol and cholesterol in 5.0 mM HEPES (pH 7.4) containing 5% DMSO. Subsequently, the purchased liposome membranes were hydrated with 5.0 mM HEPES (pH = 7.4), and suspensions containing various 600 µM phospholipid components and ergosterol suspensions were extruded six times through a 100 nm polycarbonate filter using an Avanti Mini extruder to prepare large unilamellar vesicles (LUVs). The heat release associated with binding between mandimycin B and phospholipids or sterols was measured using a PEAQ-ITC isothermal titration calorimeter. At 25°C, 1 mM solution of mandimycin B (40 µL) was loaded into an automatic injection syringe, and 600 µM LUV (250 µL) suspension was placed in the sample cell. A volume of the initial injection was 0.23 µL, followed by 18 injections of 2 µL each of the above compound at an interval of 80 s between injections, thereby ensuring that the instrument returned to a stable baseline before the next injection. The stirring speed for each experiment was 500 rpm. The results are shown in FIG. 40, which demonstrates that phospholipid molecules exhibited no binding curves with mandimycin B, whereas sterols exhibited obvious binding curves with mandimycin B. Specifically, the binding affinity of mandimycin B for ergosterol had a *K*_{d} value of 25.9 µM, while the binding affinity for cholesterol had a *K*_{d} value of 28.6 µM. This demonstrates that the mechanism of action of mandimycin B differs from that of mandimycin, and that mandimycin B exhibits strong binding affinity with sterol molecules.

A nucleotide sequence of the mandimycin biosynthetic gene cluster, designated as SEQ ID NO. 1, as cited the claims and specification of the present invention, is divided into nine segments in the nucleotide and amino acid sequence listing, corresponding collectively to SEQ ID NOs. 1-9 in the sequence listing. In addition, a nucleotide sequence of the key gene *MandQ*, designated as SEQ ID NO. 2 in the claims and specification, corresponds to SEQ ID NO. 10 in the sequence listing.

A nucleotide sequence of the mandimycin biosynthetic gene cluster (SEQ ID NO. 1) is shown below (115,505 bp):

cgtgcacggcctttcgcgtggcccggaccaaatcgcccctcacgctaggcggccgggccgcgaagaaagatggggtaagtcatct
ttcccctcaataaggtgaatgcggcgatcgcctcgcttaacgatcttgtgagtggggtgtgcgtggtcccgtggccggaaccccaccc
cgtggctaccgttcgaggcatccgcccggcacgaccgaggagcagccccgatgaccatccctccccagaacctcagcgacccccct
ggtgagatccctggccctcgcgatcaacgccggtgaccgggagggcttcctgtcgctgctgaccgacgacgcgaccctgtccgac
gacggctccgaccgtgacctccagcagtggatcgacaaggagatcttcacctccgggggccacatcgacgtgcagtccgagtcgg
acggcggccgctcgctgatcgccgacttccgcaacgacacctggggtcagatgcgcaccgcgtggaggttcgacatggcggacg
gcaggatcagccgcatcgagaccggtcaggcctgactggccggctcacccgcctcacccgactcctgcccgggcgccagggtgc
gcgaggcgtacatccgcgcgtaggtgcccgcgttcccgatcagctcctcgtgcgtgccccgctcgatgatccgcccggcctccatg
acgacgatgacgtcggcgtcccggatggtcgagagccggtgcgcgatgacgaagctggtgcggcccgcgcgcagcgagttcag
cgcccgctggatgaggacctccgtgcgggtgtcgaccgaactcgtcgcctcgtccagcaccaggatcgccggcccggtcaggaac
gcccgcgccaccgtgatcagttgcttctcgccggcgctgacgctcgacgtctcgtcgtcgagcacggtgtcgtacgcgtcgggcag
ggtccgcacgaagcggtccacacaggtggcgcgcgcagcggccacgatctcctcgcgggtggcgtcggggcgcccgtacgcga
tgttctcggcgatcgtgccgccgaacagccaggcgtcctggaggacgagaccgatgttcgcgcgcagctcctcgcgggtcatcgc
ggcgatgtccgtgccgtccagcaggatgtggccgccgtcgacctcgtagaagcgcatgaggagattgcccagcgtggtcttgcccg
ccccggtcggaccgacgatcgccacggtctggccgggccgcacggtgagcgacaggtcctcgatcagcggggtgtccggggtgt
aggcgaacgacacccgctcgaactccacgtgcccctcgacccgttcgggccgcgcgggccgcttcgggtcggggccctgctcgtc
cgcgtcgagcagcgcgaagacccgctcggcggacgctgcgcccgactgcagccggcccgcgacgctcgccgcctcgacgatcg
gccggctgaacccgccggagtacaggatgaacgcctgcacctcgccgatcgacagcgatccgctcacgacccgcagcgcaccga
cgacggccacgacgatgtagctgaggttggcgacgaacatcatcgacggttcgatggtgcccgacacggactgcgccttcgcgcc

cgcccggtacatggcctcgttgtgctcgtcgaaggcccgctccgccaactcccgcctgccgaagcccttcacgagcgagtgcccgc
tgtacatctcctcgacatgggcgttgagcgtcccggtggccgcccactgcgcggcgaactgcggctgtgcccgcttgacgatcaac
gtggccagcagcgccgacacgggcacgccgatcagcatgatcagggccagcagcggcgagatcacgaacatcaggaccagcat
gctcagcgtggagaacaccgcggtgatcagctggctcagtgactgctggagggtctgctggaggttgtcgacgtcgttggtgaccc
ggctcagcacctcgcccggcggattgcggtcgaagtagctgagcggcaggcgcgcgagcttctcctccaccctctcgcgcagatc
gaacacgacctgctggacgacggccgtggccaaccaccccctgcaggagcgtgaacagtgacgccgcggcgtacagcgcgagga
cgaccaacagcagccgtccgaccccgtcgaagtcgatgccctgaccgggcaccacgtccaccgtgctgaagacattggcgagcg
agtcgtggccctcggcgcgcagccgctcgacgacggcggccttcgactccccggtggacacctgcttgccgacgacaccggcga
agaccaggtccgtggcatcaccgaggagcttcgggccgagggcgttcagggtgacgccgacgatcccgaggccgagcagcacg
gcgatccgcccgcgctgcgggcggagcagtcccagcacccggcggaccgtaccgcgcgggtcgcgtgccttctccgacgcctcg
gtgccctccggcggggtgtcctgcccccgtccgggacggcttcgggctcccgcggtggtgtgcccggtgccgcgccgaccttga
ccgggggagtcatgccgccccgcctttcctctgggttccgggggggttggggtgacccctggcctacgagcgcgacggcgctcccg
ccgctgtcacggctgtgtcgccgcaccggcggcctcctcgctggtgagctgggagagcgcgatctcgcggtacgtggtgctgtggc
gcatcagctcctcgtgggtgccggtgcccacgacgcgcccggcgtcgaggaccacgatgcggtcggcgtgccggacggtgctga
cccgctgggcgacgacgaccaccgtcgccccggagatctcgggggtgagagccgcccgcagcgcggcgtccgtggcgtagtcg
agtgccgagaaacagtcgtcgaagagatagacgtcgggccggcgcaccagcgtgcgcgcgatggacagccgctgtcgctgccc
gcccgagagattggtgccgccctgggcgatccgcgcgtccaggccgccgggcatgcgctcgacgaattcccgggcctggacgat
ctccagtgcgcgccacagttcctcgtcggtcgcccccgggctgccgaaccgcagattgctcgccacggtgccggagaagagatag
ggcttctgcggtacgaagccgatggtgcgggagagggcgtcggcgtcgtacgcgcgcacgtcgacgccgcccaccaggaccga
accgcccgtgcggtcgaacagccgcagcagcacgttcagcagggtcgtcttgccgctgcccgtactgccgaggatcgccaccgtct
ccccgggcccggccgtgaggtcgatgtcccgcagcaccggctcctccgcccccggatagcggaactccacggcacgcaactcca
ggcggccgacgtccgagccggagtggaccggtgtcgcgggagcgatgacgctcggttcggtgtcgagcacttcctggatgcgttc
ggcggacacccgcgcgcgcggtgcggccaggaagacgaacgtcaccatcacgaccgacatgaggatcagaccgagatagctca
ggaaggcgctgagcgagccgagctgcatcgacccggcctcgatccggcgggcgccgaaccagatcagccccaccatgaaggtg
ttcatcaccatcatgacgagggggagcatcatcgctatcagccgggcgacgcgcagcgacagcgcggtcatctcggcgttcgtccg
ggtgaaccgttcgcgttcgtgggtgtcccgcacgaaggcccgcaccacccggaccccggtgatctgctcgcgcagcatccggttga
cctggtccacgcgctgttgcatcagggcgtaccacgggctcatccgggtcaggacgagcgtgaagacaccggcgatcaccacgac
gatcgccaccaggaacatcgacagcggcacgtcctgacgcaacgccagcagcacactgccgacgcacatgatgggtgccgacac
cgcgatgccgaaggccgactgggccagcgcctggacctgctggacgtcgttgaccgttctggtgatcagcgagggggtgccgaac
cggcccacctcgcgggcggagaagtccagcacgcggcggaagaccgcggagcgcagatcccgcccgagggccatcgaggtg
cgggagccgaggttcaccgcgccgatcgcgcaggcgatctgcgcgaccgccacgacgagcatgacgccgccgatccgccagat
gtaatcggtatcggccttgaccacgccgttgtcgatgaccgcggcaccgaacatgggcagcagcaacgtggccaggacctgcacc
agttgaagcagcacgaggagggtgacggagcggccgtaggggcgcaggcgggcacgcaggagtctgatcagcaccgaactctc

cggacggcagggccggcggaccgccgacccgcccagtccatcgcacaacacacaggagaatccctagtgttcaccccacgggc
cgctcccgggcgtcggccgatggtgcccgcggggtccgagtctcgcaaattccctagtgtgtcggcattttcttgacactgccgtc
gggcgactgcgagcgtgcggtcgcatatatcggccggacagaagcgcattcggtgccagttcatcgaccggtcattcttccggtgcg
ttcaccgtcggactttcgcatgttccgctcccgtggtgggacagctctgcgtgggggcggtctgtccgggggagagaaggggatga
catgtccaggcgcgcgttgatcactgggatcaccggacaggacggttcttacctggcggaacacctgctctcgcagggttaccaggt
atggggattgatcaggggacaggccaatccgcggaaatcgcgggtgagccgtctggtctccgatttgtcgttcgtggacggcgatct
catggatcaggcaagtctcgtatcggccgtggacacggtgcagccggacgaggtgtacaacctcggcgcgatttcctttgtgccgat
gtcgtggcagcaggccgaactggtcaccgagatcaacggcatgggtgtcctgcggatgctcgaagccatccgcatggtgagcggg
ctgaacggctcccgccacgcgggcggtggccagatccgtttctaccaggcgtcctcctcggagatgttcggcaaggccgccgaga
ctccgcagagcgagaccaccaggttccacccccgcagcccgtacggcgtcgcgaagacgtacgggcacttcatcacgaagaacta
ccgtgagtccttcgggatgtacgcggtgtccgggatgctcttcaaccacgagtccccgcgccgtggcgcggagttcgtcacccgga
agatctcgctggcggccgcccgcatcaagctgggccaccagaagaagctggacctcgggaacctcgacgccgtgcgtgactggg
gcttcgccggcgactacgtccgcgcgatgcacctcatgctccagcaggaggaaccgaccgactacgtggtggaaccggccagat
gcacacggtccgggacgcggtgcgctatgccttcgagcacgtggacctcgactggaaggactacgtctgcgtcgaccccgccctg
gtgcgtccggccgaggtcgaggtgctgtgcgcggacacccagcgggtccgcaccgagttgggctgggagccgaccgtcgacttc
ccgcagctgatgcacatgatggtcgagtccgacctccagcaggtgtcccgtgagcacgagtacggggaccttctgctggccggcag
ctggtagcggccgcacagcggcacaccacgccggtgcggcaccggccatcaggaccagcaacagacaaacaaacagacaagc
aacaggcaagcagcagacaaagaggcgtcaagaagaccgacgcggcgtgacgcgtacccgcgtcccgccgcgtcgtcgcgac
gaagcgccgcagaactagggatttcttgccgacgtcccgacgagactcgcctcatggcattccgcggacgcggatgccgcgagtct
cgtcgcccccgggccaaggacgtgcctgccgggtgacgttcgggcggcgcgattcaggttcggccactggctgtcgagattggttga
gttgagatggacaacgaacagaagctccgggactacctcaagcgagcgacagccgatctccgacggacgcggcagcgggtgca
ggagctcgagtcggcctcccaggagcccatcgccatcatcggcatgacctgccgctacccgggtggtgtggccggcccggagga
cctgtggcggatggtcgaggccggcgggaacggcatcaccgacttccccacggaccgtggctgggaccccgaggccctcgccg
cctcccagacgaactccggtggcttcctgcacgacgcggccgagttcgacaccggcttcttcgggatctccccgcgcgaggcggtg
gccatggacccgcagcagcgggtgctgctggagtcgtcctgggaggccttcgaacgcgccggcatcgaccccacctccatccgg
ggcacccagaccggtgtgttcatgggcgcgatggcccaggactaccgcgtcggccccgccgacggcgtcgagggcttccagctc
accggcaacaccggcagtgtgctgtccggccgcatctcctacgtcttcggtgccgtcggtcccgccgtgaccgtcgacaccgcctg
ctcctcctcgctcgtggccctccacctcgccacccaggcgctgcgcgcgggcgagtgctcgctcgctctcgccggtggtgtcacggt
catggcgagccccacgacgttcgtcgagttcagccgccagggcgggctgtccgccgacggccgctgccgttccttcgccgactcc
gccgacggcaccggctgggcggagggcgtcggtgtgctggtgctggagcggctctccgacgcccagcgcaacgggcacgagat
cctcgccgtggtgcgcggcagcgccgtcaaccaggacggcgcgtccaacggcctgaccgccccgaacggcccctcccagcagg
cggtgatccagcaggcactggtcaacgcccggctgtcggccgacgaggtcgacgtcgtcgaggcacacggcacgggcaccacg
ctgggcgaccccgtcgaggcccaggcactgctcgccacgtacggccgcaaccgcgacgagagccggccgctgctcctcggctc

ggtgaagtcgaacatcagccacacccaggccgccgccggcgtcgccggtgtcatcaagatggtcatggccatgcgccacggcgtc
ctgcccaagaccttgatggtcgacgccccctcgtcccacgtggactggtcacagggcgccgtccgcctcctcaccgagaacaccgc
ctggcccgagaccgggcggccgatgcgtgcggccgtctcctccttcggcatcagcggcaccaacgcccacaccatcgtcgagca
ggcccccgagcccgagaacgaccccgagggctcggcggacgtcaccgtcgccccgggcgtcgtgcccgtgccggtctccggcc
ggacccgcgaggccctgcgggcccaggccggacgactgctgtccgccctgaccgccgaccctcggcccggcgtcctcgacacc
gcgtactccctggccacgtcgcgcgccggcttcgagcaccgcgccgtgctcgtcgccgccggacacgacgacgtcgtcgccggtc
tgaccgccctggccgaggaccgcccggcgcccggctgcgtccagggcaccacccagggcgagggcaaactcgcgttcctcttct
ccggccagggcagccaacgcctcggcatgggccgggagctgtacgagcggttccccgtcttcgccgaggccctcgacgaggtcc
tcgccgcattggacagccacctggacgggtcgctgcgcgacgtcatgtggggcgagcaggaggaggagctggagcgcaccggc
tacgcccagcccgccctgttcgccgtcgaggtcgctctcttccggctgacgcagtcgtggggcgtcaagcccgacttcctggcggg
ccactccatcggcgaggtcgtcgccgcccacgtcgccggggtgctgtcgctgagcgacgcggccgccctggtcgccgcccgcgg
ccgtctcatgcaggcactgccggccggtggcgccatggtggccgtccaggccaccgaggacgaggtcctgccgctgctggccga
ccagccggccgaccaggtgtccgtcgccgcggtcaacggccccggcgccgtcgtcgtcgccggcgccgaggacgcgctcgccg
gcatcgtcgcccggctggaggccgacggccgcaagaccaagcggctgcgcgtctcccacgccttccactcgccgctcatggagc
cgatgctcgacgagttccgcgcggtggtgtccggcctgaccctccaggccccgctgatgccgttcgtgtcgaacctgaccggcgag
cccgtgaccgtcgcacaggtcacctccaccgactactgggtcgaccacgtccgccgggccgtccgcttcgccgacggcgtcgact
ggctgcgcggccacggcgtgaccaccttcctggagctcggccccgacggtgtcctcgccgccatggcccagagctgcctcgacga
agccggcgccgacggccacgccgccctgccgctgctgcgcgccggccgcccccgaggtgcaggccgtcaccaccggcctcgcc
ggtctccacacccgaggcgtcccggtgacgtgggacgcctacttcgagggcaccggagcgcgccgcgtcgacctgccgacctac
gccttccagcggcggcggtactggcccaaggacaaccccggccagtccggcgacctgcgcgccgcgggactcggcgtggcaca
ccacccgttgctgtccgcggcggtctccctcgccgactccgacggcgccctgctcaccggccgcctgtcactgcactcccacccctg
gctcgccgaccacgccgtacggggcacggtgctgctgcccggcaccgccttcctggagctggcgatccgcgccggtgacgaggt
cggctgcgaccgggtcgaggagctgacgctggccgcaccgctgacgctgcccgaacagggcggcatccaggtgcagttgtggat
cggcagccccgacggaccggtcgccggaccctcaacgtctactcccgcccggacggcgccgacgagctgccgtggacgcagc
acgccaccggtgtgctcgccgccggtgagcaccgcatgccgttcgacgcggccgcctggccgccgccgggtgccgagccggtgt
cgctcgacggcctctacgaccgcctcgcggaggacgggttcggctacggcgcgacgttccagggactgcgggccgcctggcggc
tcggcgacgaggtgttcgccgaggtgagcctgcccgagggcggcgaggccgacgcgggcgcgttcggcctccaccccgcgctg
ctggactcggcgctgcacgcggccgggttcaccgacctcggcgcgcagggccaaggcgggctgcccttctcctgggagggcgtg
tcgctgcacgccaccggggccacggcgctgcgggtgcggctcgccccggccggtgagggcacggtctcgatcgccgtcgccga
cacctccggcgagccggtcgcctccgtcgactccctggtgctgcgtccggtgtcggagcagcgcctcggcgacgccgccggggc
ggcccgggacgcgctcttccgtctggactgggcgccggtacgggccgagtcggccgagcccgggtcggtggccctggtgggcc
gggacgccttcgggctggccgaggcgctgccgggtgcggtgccccaccaggacctggcgtccttgccggcctcggacgcggcg
atgccggccgtggtgctggtccccgtcgccggtgagttcggtgacgggatggtcgggtccgcgcacgcgctggtggcccgggccc

tgggccacgtacagaactggctggccgacgagaggttcgtcggctcgcgcctggtgttcgtgacccggggtgccctgggcggcgg
cgaccctgccgcggcatcggtgtggggtctggtgcgctcggcgcagacggagaaccccggctgcttcgggctgctggacctgga
gacggaggccggggacgacgcctcggccgccgcgctcacacgggcgctggcttccgacgagccgcagctggccgtccgcgcg
ggcgagctcaaggccgcgcgcctggcccgggtaccggtgtcggacgccgtgtccgggacggccgtctgggacgccgagggcc
gggtgctcgtcaccggcggcaccagcggcctgggtgcggtcgtggcgcggcacctggtggccgaacacggtgtgcggcggctg
ctgttggtgagccgccggggtctcgccgccgacggtgtggacgacctggtcgcggagctggcggagctgggcgccgaggccgtg
gtggccgcgtgcgacatcgccgaccgtgacggtctcgccgagctgctcgctcagcacccggtcaccgccgtggtgcacgcggcg
gggtcctcgacgacggtgtcatcgcttccctgacgcccgaacgcgtcgacgcggtgctgcgcccgaaggtcgacgcggtgtgga
acctgcacgagctgacccgcgacgcggacctgtccgccttcgtggtgttctcctccgtcgccggtaccttcggcggtgccggccag
ggcaactacgcggccggcaacgccttcctcgacgccctgatgcagcggcgccgcgaggagggcctgcccgggatctccctggtg
tggggcccctgggaccagtccggcggcatgaccggcacgctcggcgacgccgatctccagcggctggcccgcgcgggcatgcc
ggcgctgtcggccgagcagggcgtcgcgctgttcgacgcggccctggccaccgacgagcccgtcgtcctgccggtccgcctcga
cctgcccgcgttccgtgcgagcggcgacgtcccgcccctgctgcgcggcctcatccgtacgcgcgcccggcgcgccgccgtcgc
cggatcgagcacggccaccggcctcgtgcagcgtctgggacggctggcggaggccgaccgtgacgaggccctgctggacctgg
tgcgcggtcaggtcgccctggtgctcgggcacgcggacgccggggagatcgacgtcgcccgcccgttccgcgacctcggcttcg
actcgctgacggcggtcgagctgcggaaccggctgaacaccgtgaccgggctccgactgcccgccacccttgtcttcgactacccg
agcgtgcgcaccctggccgcgtacgtcctcgacgagctgctcggctcggaggccgccgtcaccgcgcccgccgcgcgggccgt
ggacaccgacgacccgatcgtgatcgtggggatggcctgccgttatccgggcggggtgacgtcgccggaggacctgtggcggct
cgtcaccgagggcaccgacgcggtctccggcttccccgccaaccgcggctgggacgtcgacggcctgtaccaccccgaccccga
ccaccccggcacggcctacacccgatcgggcggcttcctgcacgacgcgggcgagttcgaccccgccttcttcggcatgagcccg
cgcgaggcgctcgccaccgactcgcagcagcgtctgctgctggaggcgtcctgggaggccgtcgagcgcgccgggatcgatccg
gtgtccctgcgcggcagccagaccggtgtcttcgccggtgtgatgtacagcgactacagcgccgtactcgccggtggtgacttcgag
ggcttccagggcagcggcacctccccgagcctggtctcgggccgcgtgtcctacaccttcgggctcgagggcccggcggtcaccg
tcgacacggcctgctcctcctcgctggtgaccctgcacctggccgctcaggccctgcgggcgggcgagtgctcgctggcgctggc
cggcggtgtgacggtcatgtcgacgccgtccgtgttcgtcgagttctccaagcagcgggggctgtccgcggacggccgctgcaag
gcgttctccgacgcggccgacggcgtcggctggtccgagggcgtcggtgtgctcgtcgtcgagcggctctccgacgcacggcgca
acggccacccggtcctcgcggtcgtccgcggcagcgccatcaaccaggacggcgcctccaacggtctgacggcccccaacggc
ccctcccagcagcgcgtcatccgccaggccctcgccagcggtggactctcgcccgccgacgtcgacgcggtcgaggcccacggc
accggcaccaccctcggcgacccgatcgaggcccaggccctcctggccacgtacgggcgggaccgggacgaggaccggccgc
tgctgctcggctcggtcaagtccaacatcggccacacccaggccgccgccggtgtcgccggcgtcatcaagatgatcatggcgatg
cgccacggcatcctgccgcgcaccctgcacgtcgacgcccccctccagcaaggtcgactggaccgagggcgccgtcgagctgctc
accgagcagacggcgtggcccgagaccgaccgcccccgccgcgcgggcatctcctccttcggcatcagcggcaccaacgtccac
accatcatcgagcagcccccggcccccgcccccgagcggaccgccccgaacgccacagccgtcgtcccgtggctgatctccggc

cgtacccgcgacgccctccgggcgcaggcggcacggctgcggtcctacgtcgaggcgcgccccgagctggaccccgccgacgt
cgcgtactccctgctgaccacccgctcccgcttcgaccagcgcgccgccgcggtggcgggcgaccgcgacgcgctgctgcgcgc
gctcgccgcgctggccgccgaccggcccgacgcccacctggtcgagggcgaggccgcccaccccggccggacggcgttcctct
tcgccggccagggcgcgcagcgcctcggcatgggccgcgaactgtacgagcggttccccgtcttcgccgaggccctcgacgcgg
tgctcaccgaactgaccccgcgtctggacacccatctggaccgcccgctgcgcgaggtgctcttcgccgccgcggactcggccga
cgccgccctcctggaccgtacgggctggacccagcccgccctcttcgcggtcgaggtggcgctgttccgcctcgtgtcgtcctggg
gcgtcacccccgactacgtcaccgggcactccatcggcgaactcgccgccgcccacgtcgcgggcgtcctctcgctcaccgacgc
ctgcacgctggtcgccgcgcgggccggactcatggaggcgctgccggccggtggcgccatggtggccgtccaggccaccgagg
aagagatcctgccgctgctcaccgacggcgtgtcgatcgccgcggtcaacgggccgacgtcggtcgtcgtcgccggtgacgagga
cgcggtccgggagatcgccgaccgtctcgcggcggaggggcgcaagaccaagcggctacgggtgagccatgccttccactcgc
cccgcatggacgcgatgctggacgacttccggcgcgtcgccgaggggctgacctacgcggcaccggccatcccgctggtgtcga
acctgaccgggcggctcgccaccgaggaagagctgcgctcccccgactactggacccggcacgtgcgcggcgcggtccgcttcg
ccgacggcgtccgcaccctgcacgacgcgggtgtgaccaccttcgtggaactcggccccgacggcgtgctgtccgccatggccca
ggacaccctcggcgacgaacacggcgccctgctcgtccccgtgctgcgcgccgaccgccccgaggagcagaccgtcaccgccg
cgctggcgcgcctgcaggtgcacggcgtgagcgtggagtggtccgccttcctcgcgggctccggcgcccaccgggtggacctgc
cgacgtacgccttccagcacgagttctactggccgcaggccgcctcgccggtgaccgccgacgcgtccgccgaccccgcgcacc
agcggctgtgggccgccgtggagcggggcgacgccgatgaactggccacgatcctgggcctcgcggacgaccagatctcctcgc
tcggctcgctgctgcccgccctgtcgtcctggcgccagggcaaccaggagaaggccctcctcgacacctggcgctaccgggtcga
gtggacccggctgcgaggcgccgccgccccggcggcggacggcagctggctgctcgtcaccaccgacgggatcgacggcgag
ctccgcgacgacctgctcgccgcgctgcacacccacggcacccgggcccgtctcctggtgctcgacgacccgtgtgccgaccgga
gcgtcctcgccgagcggctcaccgccgtcgaaggcgccgaaggcctcaccggtgtcctgtccctcctcccgctggacgagcgccc
cagcccggcctgtccgccgctcaccaccggactcgccctgacggtcgccctcacccaggcactcggcgacaccgggtggggcgc
ccggctgtggaccgccacccgcggcgccgtgtcgaccggccccgccgaccccgtcacccggcccgcccaggccgccgcgtgg
ggcctcggccgcgtcgccgccctggagtacccgcgaagctggggcggactcgtcgacctgcccgaggtgctcgacaagcacgcc
gtacagcgcctgctgggcgtcctgaccggcctcgacggcgaggaccaggtggcggtccgcgcctccggcctctccggccgccgc
ctcgcccgcgccttcgcccaggacctgcccgccgagaccgcgttcaccgcccgcggcaccgtgctgatcaccggcggcaccggg
gcgctcggcgccgaggtcgcccgctggctcgcccgcagcggcgccgagaacctggtgctcaccagccgccgcggtcccaccgc
ccccggcgccgccgaactccgcgccgaactggaggcgctgggcccccgcgtcgagatcgtcgcttgcgacgtcgctgaccgcga
cgcgctggccgccgtgctcgcggccgtccccgacgagctgccgctgaccggtgtcgtgcacaccgcgggcgtcggccaggccg
cacccctggagcagacccccgtcaccgccttcgccgagctgacgtcggccaaggcggcgggcgcggccaacctccacgccctg
ctcggggaccgcgaactcgacctcttcgtcctcttcggctcgatcgccggcgtgtggggcagcggcggccagagcgcctacggcg
cggccaacgcctacctcgacgccctggccgaacaccgccgcgcccacggcctgaccgcgacctccgtggcctggggcccctgg
gccgaggccggcatggccgtcgacgacgccatctccgacagcctggtccgccagggcctgggcttcctcaccccgcagtccgcc

atgaccgaactgcggcgcgcggtcgtgcaggggacgtcaccgtcaccgtcgccgacgtcgaatgggagcgctacttcccgctctt
cgcctcgatgcgccccagcgcgctgctcgccgacctgcccgagatccgcgccctccagctgagcaccgagcacgagaccggtgc
cgcctccgagtacgccgcacgcgtccgcgccctggccgaggaggagcgggagcggctcctcgtggacctcgtccgcgacgagg
ccgccgcggtcctcggacacggctcggcggactccgtccccgaacaacgggccttccgcgaggccggcttcgactcgctgaccg
ccgtggagctgcgcaagcggctcggcacggtcaccggcctcaccctgccggccaccatggtcttcgactaccccacgcccctcgc
cctggcgcggtacctggccgccgagatcttcggcgccggcatcgacgtggccggcccggtcgccacctcggcggcggccgacg
acgaaccgatcgcgatcatcggcatgagctgccgctacccgggcggggtcacctcgcccgagcagctctggcggctcgtcgcgg
acggggtggacgccatctcggagttccccgccaaccgcggctgggacaccaccggcctctacgacccggaccccgaccgggcc
ggtacgacctacacgacgcagggcggattcctgcacgaggcgggcgagttcgaccccgggttcttcggcatctccccgcgtgagg
cactgggcatggacccgcagcagcgcctgctgctggagaccacctgggaggccgtcgagcgggccggcatcgaccccgcctcg
ctgcggtccagcctgaccggcacgttcatcggctccagctaccaggagtacggccgcggcggcgccgagcccggctccgagggc
cacatggtcaccggcagccagccgagcgtcctctccggccgggtgtcctacgtcttcggcctggaaggccccgcggtcaccgtcg
acacggcctgctcctcctcgctggtcgccctgcacctggcctgccagtcgctgcgcaacggcgagaccaccctcgcggtcgcggg
cggcgtcacggtcatgacgaccaccgacccgttcgtcgccttcagccgccagcgggcgctggccaccgacggccggtccaaggc
gttcgccgaggaggcggacggcttcgcgctcgccgagggcgtcggcatcctgctcgtcgagcggctctccgacgcacggcgcaa
cggccacccggtcctcgcggtcgtccgcggcagcgccatcaaccaggacggcgcctccaacggcctcaccgcccccaacggcc
cctcccagcagcgcgtcatccgccaggcgctggccaacgcgcgactgtcccccctcggacgtcgacgcggtggaggcccacggca
ccggcacaccgctgggcgaccccatcgaggcccaggccctccaggtgacctacggccgcgaccgcgacgcggaccggccgct
gctgctcggctcggtgaagtccaacatcggccacacccagtcggcctccggcgtcgccggtgtcatcaagatggtcatggcgatgc
gccacggcctgctgccgaagtcgctgcacagcgacaacgcctcgtcccacgtcgactggtcggccggtgcggtcgagctgctcac
cgagccggtcgagtggccggcgggggagcggccgcgccgctgcgcggtgtcctccttcggcatcagcgggacgaacgcgcac
gcggtgctggaggaagcgccgcgcgccgaggacctcacggccgcggagcccgcctccccggccccggcggacgacgccgcg
gtgctgctcccatgggtggtgtccgcgcgcagcgcgcccgccgtccgggaccaggccgcgagcctggtgtcggccgtggaggcc
gagccgggctggcgtccggccgacatcgggttctcgctgacgtcggcacgctcgctgttcgaacaccgcgcggtcgtcgtcggca
cggaccgcgaggagctgctgaccggcctgcgggccgtcgccgccgacgcgtcgtcgcccgccgtcgtgcgcggtgccgcggac
gtcgacggcaagacggtgttcgtgttcccggggcagggttcgcagtgggtgggcatgggtgcccggctgctggaggagtcgcccg
tcttcgccgagcgcgtcgcccaatgcgccgacgccctcggcccgttcgtcgactggtcgctgaccgacgtcctgcgccaggcaccg
gacgcccccctccctggagcgggtcgacgtcgtacagccggcgtcgttcgcggtgatggtgtcgttggcggcgttgtggcaggcgca
tggtgtgcgtcctgatgctgtggtgggtcactcgcagggtgagatcgccgcggctgtggtgtcgggtgcgttgtcgttggaggacgg
cgcgcgggtggtggcgttgcggagccaggccatcggccgtcggctggcggggcgtggcgggatgatgtcggtcgcgctgccggt
cgcggaggtcgaggagcggctggacggtttcgcgggccgggtgtcggtcgcggcggtcaacgggccgcgttcggtggtggtttc
cggtgatccgcagggtctggacgacctggccgcgcagctcacggacgagggtgttcgggtccggcggatcgcggtggactacgc
ctcgcactccgctcaggtcgaggacctccacgacgaactgctgtccgagctgggaccgatccggccccgcgagtccgaggtgccg

ttcctgtcgaccgtcaccggcgcgtggctggacaccagcggcatggacgccgagtactggcatcgcaacctgcgcaacaccgtcg
agttcgaagcggccgtgcggtcgctcctcggtacgaaccaccgggtgttcgtggaggtcagctcccacccggtgctgaccgtcggc
gtccaggacatgctcgacgacgccaccggccaggggctggtgaccggcacgctgcggcgtgacgagggcggtctgcggcggtt
cctgacctcgctggccgaggtgttcgtccggggcacggccgtccgctggccggtggacctcaccgccgtcggcgcccgccgggt
cgacctgcccacgtacgccttccagcacgaacacctgtggatcgtgccctccgaaaccgaccggacgggtaccacggaccccgcc
gacgacgccttctgggcggccgtcgagcagcaggacgtctccgctctcacctcgagcctgcatatcgacgagaagtccctcgccgc
cgtgctgcccgccctgtcgtcgtggcgccgtgagcggcgtgagcagtccgcgctggacgcgtggcgctaccgcgtcgcctggtcg
ccgctcagtggtgtcccgcaggcttcggtgtcgggcacctggctggtggtcggtgccgaaggcatcgccgacgacgacgtggtgg
ccgcgctgtccggccagggcgccgaggtgcgccgcctggtgctggacgagtcctgcgtcgatcgcgccgtcctcgcggcccggc
tgcgggacgtcgcagacgtggccggcatcgtctcggtgctggcggcggaacagccgtgcgccgcgtacccggcactggcct
ccggtctcgccctgacggtcgcgctcgtccaggcgctgggcgatgcgggcatcgacgccccgctgtggtgcgtgacccgtggcgc
ggtctcgaccggccgttcggacctggtgacgaacccggtgcaggcgcaggtgctgggcgtcggctggacggccgcgctggagca
cccgcagcgctggggcggcatgatcgacctgcccgacacgctcgacgaacgcgccggacagcggctcgccgccgtcctcacgg
gtaccaccggcgaggaccagctcgcgatccgcccctccggcgtgttcacccgccgggtgctccccgccccggccggcgaaccga
acccggaccgcgcgtggtcgccccggggcaccacactgatcaccggcggcacgggcaccctgggcccccacatcgcgcgatgg
ctcgcgggccagggcgccgagcgcctggtcctcgtcagccgcagcggcatgaacgcccccggcgccgccgagctcgtggccga
actcgccgaacagggcgccgaggcctccgtcgccgcctgcgacatcaccgaccgcgacgcgctcgccgaactgatcgccacgct
gaaggccgacggccacaccatccgcacggccatccacaccgccgccgtcatcgagctgcactcgctggcggagaccaccatgga
ggccttcgccaaggtcgtccacgccaaggtcgccggagcacagaacctcgacgaactgctggacgacgccgaactggacgccttc
gtcctcttctcgtccgccgccggcatgtggggcagcggacagcacgccgcctacgtggcgggcaacgcgttcgtcagcgcgctcg
cggagaaccgccgcgcccgcggggtgaccgcgacgtccctgcactggggcatctgggccgacgacctcggcctcggccgggtc
gaccccgaccagatccggggcagcggcctggagttcatggacgcccggctggcgctggccggcatgcggcgcgccctggacga
cgacgagacggtgctcgccatcgcgggcgtcgcctgggacacctaccacccggtgttcacctcgggccgtcccaccacgctgttcg
acgaggtgcccgaggtccgccgactggccgaggccgccgagcagagcgtgcgcaagggcggcgaaggggagttcgcggccc
ggctgcgggccctcccggccgtcgagcaggaacgccaactgctggaactcgtccgctccgaggccgcgggcgtcctcggctaca
cctcgtccgaggcactgtccgaccagcgtgccttccgcgacgtcggcttcgactcgctgaccgccgtgggcctgcgcaaccgcctc
cgggagatcaccgggctgcggctgccgacgaccatggtcttcgactaccccacccccctcgcgctcgtggagttcctgcgcgccga
gatcgcgggcgtcgacatcgccgcggccgccccggcggcctccgccgcccgcaccgacgagccgatcgccatcgtcgggatga
gctgccgctaccccggcggggtcggctccgcgcaggcgctgtgggacctcgtcatgaacggcaccgacgccgtctccggcttccc
cgccgaccgcggctgggacgccgaggggctctacgacccggaccccgacagccacggcaagacctactccgtccagggcggat
tcctgcgggacgtcgcggacttcgacgccgggttcttcggcatctccccgcgtgaggccctggccatggacccccagcagcggttg
ctgctggaaaccgcctgggaagccctcgaaggcgccggcatcgaccccgactccctgcggggcagctcgaccggcaccttcatc
ggcgcgagctaccaggactacggaaccggcgccgccaacgccaccgagggttccgagggccacatggtcaccggcaccctctc

cagcatcctctccggccgggtctcctacctcttcggcttcgagggccccgcggtcacgctcgacacggcctgctcctcctcgctggtc
gccatgcacctcgcctgccagtcgctgcgcaacggcgagagctccctcgccctcgccggcggcgtcagcatcatggccaccccga
acgccttcgtcggcttcagccgtcagcgcgccctcgccgtcgacggccgctgcaaggcctactccgacgacgcggacggcatgac
cctcgccgagggcgtcggcctcgtcctgctggagcggctctcggacgcccgccgcaacggacaccaggtcctcgcggtcgtccg
cggcagcgccatcaaccaggacggcgcctccaacggcctcaccgcccccaacggcccctcccagcagcgcgtcatccgccagg
ccctcgccaacggcggcctcacgcccgccgacgtcgacgcggtcgacgggcacggcaccggcaccgccctgggcgacccgat
cgaggcccaggccctgctggccacctacggccaggaccgcgagcggccgctgctgctcgggtcggtcaagaccaacatcggcc
acacccagatggcttccggtgtcgcgagcgtcatcaagatggtcatggccctgcgcgagggcgtcctgccgaggacgctgcacgc
cgagacccccctcgtcccacgtggactggtcggcgggcgccatcgagctgctgaccgagccggccgagtggcccgagaccgggc
gtgcccgccgggccggtgtgtcctccttcggactcagcggcaccaacgtgcacaccatcctcgaacaggcaccgcccgccgaggc
acccccctgctgaggcccccccctgctgaggaactggcggcgcccgcgagcgggcccgcgcccggcatcgtgcccctgacgctgtc
cgccaagagcgacaccgccctgcgcgcccaggccgaccgtctgttgtccctgctcgccgagcggcccggactccacctcaccga
cctcgccttctccctggccacgtcccggaccggcttcgaacgccgggcgggcgtcgtggcgggggagcgcgaggaactgctgcg
cggcctcacggccctgcgcgacggcacccccgccccgggcgtcctccagggcaccgcgggccgcggccggctggcgttcctgtt
caccggacagggcagccagcggcccggcatgggccgggaactctacgagcggtacccggtgttcgccgacgcgctggactccg
tgatcgcccggatcgacaccgccctcgaccgcccccctgcgcgaggtgatcttcgcggaggacggcacgcccgaggccgagctgc
tcgacgccaccggctacgcccagcccgccctgttcgccgtcgaggtggcactcttccgcctcgtcgagtcctggggcatcaggccc
gactacctggccgggcactccatcggtgaactcgccgccgcgcacgtcgccggggtgctgtccctggaggacgcctgcgccctcg
tggccgcgcgcggccggctgatgcaggccctccccgccgggggcgccatggcggccctccaggccaccgaggacgaggtcctc
ccgttgctggccgaccacgccgaccgggtgtccgtcgcggccgtcaacggccccgcctcgctggtcgtctccggcgacgaggac
gtcgtggtggacatcgccgcccgcttcgaggccgacggacgcaagacccggctgctgcgggtgagccatgccttccactcgccgc
tgatggacgcgatgctcgacgacttccgccgcgtcgccgagagcgccacctaccacgcgcccaccatccccttcgtgtccaacacc
accggccgtctcgccaccgccgagcaggtgtgctccccggagtactgggtgcgccacgtccgcgacgcggtccgcttcgccgac
ggcgtgaactggctggccgagcacggcgtacgcaccttcctggaactcggccccgacggtgtgctctgcggaatggcccgcgaga
gcctcgccgacgacccgcacgccgtgctggtcccccctgctgcgccgcggccgccccgaggagtcggcgatcaccgccgcgctc
gtcgacgcccacgtgcacggggtgtccgtcgactggtccggattcttcgcgggttcgggtgcgcggcgggtggagctgccgacgt
acgcgttccagcgggagcggttctggccggagtcggtcgcggcgccggtggccggtgtggtggatccggtggatgccgagttctg
gtcggcggtggagcgggaggacctggggtcgctcgctgacacgctggacctggacggggagacggtgacggcgatggtgccgg
cgctgtcgtcctggcgccgccgccgtcgtgagcagtccgtcgcggacgcctggcgttaccgcgtcacctggaagcccctcaccgc
acccgccaccgccgcaccgcgcggcccctggctcgtcctcgccccggccggctcgggcacggacccctggacgtgcgccctga
ccgacggcctcggcgtggagaccgtccgcgtcgaggtgaccgcccccgaccgcgccgccctcgccgggcgcctgcgcgaactg
ctcgacgggggtgcccggttcgagggcgtcctgtcgctgctcgcactcgccgacgacgagcgggccgggcacccgggggtgcc
cgccgcactggccctgacggccgcggccgtccaggccgcgggcgacgccggactcgacgcgcccctgtggtgcgtcacccgcg

gcgccgtctccgtcggccgctccgagcacgtccaggaccccgcccaagcggcggtctggggactcggccgcgtcgccgccctgg
agaacccgcagcgctggggcggtctcctcgacctgcccgagcagctcgacgcccacaccgcccgccggatcgcgggcgtcctcg
cccggaccgacggcgaggaccaggtggccgtccgcgcctccggcgtcttcgtccgccgactcgcccacagccccgcggcggac
cggcccgtacgccgtgtgttcgccccgaccggcaccgtgctgatcaccggcggcaccggcggactcggcggccacgtcgcccgc
tggctggcccgcagcggcgcacagcacctgctgctcaccagccgccgcggcgcggacgcccccggcgccgccgcactgcgcg
acgaactcgagcagctgggcgccgacgtcaccgtcaccgcctgcgacgccgccgaccgcgacgcgctcgccgccgtactcgcc
gccgtccccgccgaccggccgctcaccgcggtcgtccacaccgcgggcgtcgtcgaggacggcgtgctggccgacctgacgcc
ggagaacttctccgccgtcatgaacgccaaggcggtctcggcccgcaacctgcacgagctgacgcgggacaccgccctgtcggc
gttcgtcctgttctcctccacggccggcaccctcggcgccgcgggacagggcaactacgccgccgccaacgcctacctggacgcc
ctcgccgaacaccgccgggccgccggcctgaccgcgacctccctcgcctggggcccctgggcccagtccggcatggtcgccgtc
gacgccgacgtcgagagccgcgtccgccgcggcggcttcgcaccgctcgcacccgagcgggccgtcgccgcgctgaagcagg
ccatcgagcacgacgacaccgtcctcgccctcgccgacatcgactggagccgctacgccgccaccgtcgcggccgtccggcccg
ccccgttcgtcgccgacctgcccgagatgcgacggaccgccacgaccggcaccggggaggacaccgccaccggcggcgaacc
cgcgctgcgccgccgcctcgcggagctgcccgccgccgaccgcggccggttcctggtggacctgctgcgcacccaggtggccg
cggtgctcggccactccgacgcctcccgggtggagtccgaccgggccttcaaggacctgggcttcgactcgctgaccgcgctcga
actgcgcaacgggctcgccgcggccaccggcctgagcctgcccgcctccctggtctacgaccacccgaccctgctggagctgtcg
gagttcctgctggccgagatcgcggggacgctgcccgacgccgtggcgacggcacccgccgcccgcccggccgacgacgacc
cgatcgcgatcgtcggcatcggctgccgcttccccggcggcgtgtcctccccggaggacctctggcgactgctcgccgacggccg
cgacggcatctccgccttcccggcagaccggggctgggacctcggtgccctcgccgccggggcctccgccaccaccgagggcg
gattcctcaccggcgtcgccgacttcgacgccgccttcttcgggatctccccgcgcgaggccctggccatggacccccagcagcgg
ctgctgctggagaccacctgggaggccctggaacgggccggcatcgaccccgcgtcgctgcgcggctcccgcaccggcgtcttc
gtcggcaccaacggccaggactaccccaccctgctgcgccggtcgtcctccgacgtccagggctacgtggcgaccggcaacacc
gccagcgtcatgtccggccggctctcctacacgctcggcctcgaaggcccggcggtcaccgtcgacaccgcctgctcggcgtcgct
cgtcgccacgcactgggcggccggggcactgcgctccggcgagtgcgaactggcgctcgtcggcggcgcgtcggtgatgtccgg
cccggactccttcatcgagttcagcacccagggcggcctggcccccgacggccgctgcaaggccttcgccgacggcgccgacgg
caccgcctggtccgagggcgtcggcatcctcgtcctggagcgcctctccgacgccgtgcgcaacggtcacgaggtccgcgccatc
gtgcgcggcaccgccgtcaaccaggacggcgcgtccaacggcctcaccgcgcccaacggcccggcacagcagcaggtcatccg
gcaggccctggccgacgcgggcctcaccgccgccgacgtcgacgccgtcgaggcgcacggcaccggcaccacgctcggcgac
ccgatcgaggcccaggcgctgctcgccacgtacgggcgggaccgggagcggccgctgctgctcggcgcggtcaagtccaacat
cggtcacacccaggccgccgccggcatggcgggcgtcatcaagatggtcctggccatgcgccacggcacgctgccgaagaccct
gcacatcgacgcgccctcgtcgcacgtcgactggtcggccggcgcgatcgagctgctgaccgagccggccgagtggcccgagac
cgggcacccgcgccgggcgggcgtctccgccttcggcgtcagcggcacgaacgcccacgtcatcgtcgagcaggccccgccgg
ccgaggccgcgcccgcagccgagcccgtgcgcacgaccgcaccggccgtcgtgccatgggtcgtctccggccggacccgcga

ggcgctcgacgagcagatcgagcggctcacctccttcgccgcggaccaccccgaactgtccccggtggacgtggggtactcgctc
gcgagcggccgctccgccttcgggcaccgcgcggtactgctggccggcgccgacggagtgacggaggccgcgcgcggcctgc
gcggcaccgcgggcacgtccgcgttcctgttctccgggcagggcagtcagcggatcggcatgggccgcgagctgtacgagcgctt
ccccgtcttcaccgaggccctggacgccgtcctggcccatctggacctggaactggaccgcccgctgcgggaggtgctgttcgccg
cggcgggcaccgacgaggcggagctgctggaccgcaccggctggacccagcccgcgctgttcgcgatcgaggtcgcgctgtac
cgcctggtcgagtcgtggggcatcacggcggacttcgtggcggccactccatcggcgaggtcaccgcggcccatgtggccggc
gtgttctccctggaggacgcctgccggctggtcgccgcccgcgccacgctgatgcaggcgctgccggcgggcggggcgatgctc
gcggtggaggccaccgaggacgaggtcaccccgctcctgtcggacgaggtcacgatcgcggcggtcaacggcccgaacgccgt
cgtcgtctccggagccgaggaagccctccttcggaccgccgagcggttcgccgccgacggccggcgcacgacacggctgcggg
tcagccacgcgttccactcgccgctgatggacccgatgctggacgcgttccgcgcggtcgcggacggtctgacgttcacggctccg
cgcatccccgtcgtctccaacctcaccggcgggctcgccgacgccgagcagctgtgcacggccgactactgggtgcggcacgtcc
gcgaggccgtgcgcttcgccgacggcgtccacaccctgcgcgccgccggtgtggggaccttcctcgaactcggccccgacggag
tgctctccgccctcgtccggcgcgtcctcgacggccaggagcccgagccggccgtcgtgccggtgctgcgcggcgaccggggcg
aggagcaggccgtggtcaccgcggccgcccgactgcacgtgcacgggctgcccgtgaactggtcggccctgttcgaaggttcgg
gtgcgcggcgggtggagttgccgacgtacgcgttccagcgggagcggttctggccggaggtggttgccgcgccggtggccgggg
cggtggatccggtggatgccgagttctggtcggcggtggagcgggaggacctggggtcgctcgctgacacgctggacctggacg
gggagacggtgacggcgatggtgccggcgctgtcgtcctggcgccgccgccgtcgtgagcagtccgtggccgacggctggcgtt
accgcgtcacctggaagcccctcaccacacccgccaccgccgcaccgcagggtcgcctactggtgatcgtgccggccgggtacg
ccgacgacgcgtggctggccgccgccgtcgccgcgctgggcgaggacaccgtccgcgtcgaggccgacgccaccgaccgggc
cgctctggccgaccggctgacgcgggcacagggcaccgacgccacgtacacggccgtcgtgtccctgctcgccgccacctcgga
ggacacctcgctccccgcgtccacggcatggccgaccgagctgctgaacgcgctcgacaccgccgggatcgaggcgcccctgtg
gtgcgtcacccgcggcgcggtctccgtgggccgttccgaaccccctggcgaacaccggccaggccgccgtgtggggccagggcc
gggtggtggcgctggagaacccggaccgctggggcggtctggtcgacctgcccgagaccctcgacccgcgcgccgccgaccgc
ttcgccaccgcactgggcggccacgacgacgaggaccagctcgcgatccgcccctccggggtgttcgcacggcgcctggtgcgc
gccggccgttcctcctccgccgcctgggccccgtccggcacggtgctcgtcaccggcggcacgggcgtgacgggcggccacgtc
gcccgctggctggcccggcacggcgccgagcacctggtgctcaccggccggaccgggaccgttgccgccgggacccccggcg
ccgccgacctcgaggccgagctgaccgagctgggcgccacggtcacgatcgccgcctgcgacctcaccggcgcccccggccgt
gccgccctgagcgccgcgctggacgccctccccgaggggaagccgctgaccgcggtcgtccacgccgacgaggagaccggca
ccgacgcggcggccgcgctcgccggggtgacccacctcgacgccgcactgggcgaccgggccctggacgcgttcgtcctcttcg
gctccatcgccggagtctggggcgtccgcgggcagggcgacgaggccgcggtcggcgccgtcctggacgcgttcgccacccgg
cgccgcgaccggggcgccaccgccctcaccgtgtcctggggcgcctggccgagacgacggatccgggcatgaccgggcacct
gcggctgagcggcctgcccgtcatggacaccgaaccggcgctcaccgcactcgcccacgccgtcaccggcgacgaaagcggcg
tcaccgtcgccgacgtgcgatgggagaccttcgcgcccgccaccaccgccacccgccgcaccgccctcttcgcggagctgccgg

aggcccgtacggccgtcgaagccctcgcacacgagagccggggcgggcagtccgccgcggacgcactgcgggagcggctgcg
caccctgtccgagtccgagcggtccgccgaactcctcgacctggtccgcgacaaggtcgccgtggtcctcgggcacgcgagcgcc
gacgccgtcgacgccgacctgcccttccgggacctcggcttcgactccctgaccgccgtcgacctgcgcggccagctcaccaccg
ccacaggcctgagcctgcccgcgaccctcgcgttcgaccaccccacccccgccgtgctggccgaccacctcaaggccgaactgct
cggcgagctgcccggggcccccgggaccacggccgacgccgtacccaccggccaggtgaccgacgaccccgtcgcgatcgtc
ggcatggcctgccgctacccgggcggggtgcgctcccccgaggacctgtggcggctcgtcatggccgagaccgacgcggtggg
cgaactgcccaccgaccgcggctgggacctggacggcctgctgtccggcggggcggacggccgcggccgcagcgccaccggc
cacggcgggttcctctacgacgccgccgagttcgaccccggcttcttcgggatctccccgcgcgaggccctggtcatggaccccca
gcagcgcatcgtgctggaggccgcctgggaggcgctggagcgggcggggatcgaccccgtcaccctgcgcggcaccgacgcc
ggcgtcttcgtcggcggcggcagcggcgactaccgcccgccggtggagttcggggagtggcagaccgcccagtcggcgagcct
cctctccggccggctggcctacagcttcgggctgaacgggcccaccgtctcggtggacaccgcctgctcctcgtcgctggtcgccct
gcacctggccgcccaggccctgcgcaacggcgagtgctccctcgcggtggccggcggtgtgaccgtcatggccaccccgaccgg
attcatcgagttcagcgcacagggcgcgctgtcggccgacggccgctgcaaggcgttctccgacgccgccgacggcaccggctg
gtccgagggcgtgggcatgctcgtcgtcgagcgcctctcggacgcgcggcgcaacggccaccgggtcctcgcggtcctgcgcgg
ctcggccatcaaccaggacggcgcctccaacggcctcaccgccccgagcggccccgcccagcagcgcgtcatccgccgtgccct
ggccgccgccggactgtccaccaccgacgtcgacgccgccgaggcccacggcaccggcaccaagctgggcgacccgatcgag
gcacaggccctgctggcgacctacggccaggaccgggaacggcccctgctgctcggctcgctgaagtccaacatcggccacacc
caggccgcggcgggcgtggcgggcgtcatcaagatgatcctcgcgatgcgccacgggacgctgccgaagaccctgcacgtcga
cacccccgaccaccaacgtggactggacggcgggcgcggtccggctgctcaccgagacgaccgactggcccgacgccgggcgtc
cgcgccgcgccgcggtctcggcgttcggcgccagcggcaccaacgcgcacgtgatcctggagcaggcacccgaggccgagcc
cgccgcggcgcccgccggacccgcccgggacgccggcaccctgccggtcctggtgtccggcaagacgcccggcgcgctgcgc
gcccaggcccgcaggctcctcgaccacgcggccgcgagccccgacgcgaccgtcggcgacctcgcgtactcgctggcgaccac
ccggtccgccttcgagcaccgggccgccgtcctggccacgggccgggacaccctcctcgacggcctcaccgcgctggccgccg
ggaacctcgcccccccacaccctggagggcgaggccggccgcaccggccggaccgcgttcctgttctccggccagggcagccag
cgcctcggcatgggccgcgagctgtacgagcggttccccgtcttcgccgacgccctcgacgaggtgctggcccacctcgacgtca
gcctggaccgcccgctgcgggaggtgatgtggggcgaggacgcgcaggccctcgacaacaccggctacgcccagcccgccctg
ttcgccgtcgaggtggccctgttccggctggtggagtcctggggcgtcacaccgcagttcatgggtggccactccatcggcgaggtc
accgcggcgcacgccgcgggcgtcctctcgctggcggacgcctgccggttggtgaccgcgcgggcgggcctgatgcgggcact
gcccaccggtggcgccatggtcgccctgcgtgccaccgaggacgaggtcatcccgctcctgaccgacgaggtcgcgatcgcggc
ggtcaacggcccgaacgccgtcgtcgtctccggtaccgaggaggcggtgctcctgatcgccggtcacttcggtgcgcaggggcgt
aagacgacgcggctgccggtcagccacgccttccactcgccgctgatggaccccatgctggccgacttccggcgggtggccgag
gagctgacgtaccacaagcccacggtgcccgtcgtgtcgaacctgaccggcgagacggccaccggcgaggacctgtgcacgccc
gagtactgggtccggcacgtccgcgaggccgtccgcttcgccgacggcgtccgcgccctcaccggccggggcgtgaccaccctg

ctggaactgggccccgacggggtgctgtccgccctggcccgggagtccgcaccggaggccacgctcgccgtaccggtcctgcgc
aagggccacggcgaggagaccaccctcgtcaccgcactggcacggctgcacctcggcggcgtcgcacccgactggtccgcgttc
ttcgcgggcaccggcgcccgccgggcggagctgccgacctacgccttcgaacacgcccgcttctggcccgacgaccggccggc
ggccccggtggcgtcccaagtagcagcagacgccaccgaccgcgagttctgggacgcggtcgagcgggaggacttcgcctccct
ggagtccgtcctcagcgtcgggggagacaccctctccaaggtgctcccggcgctgctcgactggcgccgggcccggcaggagca
gtccgtcgtggacggctggcggcaccggatcgtctggaagccgctgaccgaggtcccgggcggacggcccgccggtacctggct
cgccgtggtccccaccggatcgaaggacgacgcctggacgaccggggtgctcgccgcactcggcaccgacgtggtgcggctgg
aggcagccgacaccgccgaccgcgccgtcctggccgggcagttgcgtgagctcaccgcggacggcacgccgttcgccggggtc
gtctccctgctggccctgcgggagaccgcggacgggagcgcgcccaccgggctcgccctcaccaccgcactgctccaggccctg
ggcgacgccgcgatcggcgccccgctgtggtgcgtcacccgcggcgcggtgtccgtgggccgcgccgaggccctgcgcaggcc
cgtacaggccgccgtgtggggtctcggccgggtcgccgcgctggagcacccggagcgctggggcggactcgtcgacctccccg
aggtccttgaggagcgctcggccgccggcttcgccgcggtcctcgccggctcggacggtgaggaccaggtggccgtacggcccg
cggcggtcttcggccgacggctggtgcaggcacccggcaagcagtccggcccgcagtgggaccccagcggcacggtgctgatc
accggcggcacgggggccctcggcggccacgtcgcccgcaggctcgcccgcgacggcgtccggcacctggtgctcaccagcc
gccgcggcgaggacgcgcccggcgcgaccgcgctgcgggacgaactcctcgccctgggcgtccgcgtcaccgtcgccgcctgc
gacgtcgcggaccgcaccgcgctggaggccgtcctcgcggacatcccggccgaacacccgctgaccggtgtggtgcacgcggc
cggcgtcctcgacgacggcatcctcgaccgactgaccccggagcgcttcgaagcggtcttccgctccaaggtcacctccgcgttcct
gctggacgagctgacccgcgggctggacctgtcggtgttcgccctgttctcctccgcctcggcggccgtcggcaaccccggccag
gccaactacgcggcggcgaacgcggtgctcgacgcgctggccgaacaccgccgttcgcagggcttcccggcgacctcgatcgcc
tggggcgcctggggcggcggaggcatggccgacggcgccgccgccgacgcggccgcccagcgcgccggcatcggcgcgatg
gacccggagctggccctggaggccatgcgccgactggtcatggagaccgaaccgacggcggtcgtcgccgacgtcgcaccgga
gaggttcgcgctggccttcagcgccgcccgcccggcggcgctgctgcgggaactccccggctaccaggccgcgaccgcggcca
ccgcgccgcaggacgggagcgggtcggccgacggtggtctccgcgaccggctcgcgggcctgccgcacagcaggcgccacga
ggcggtgctcgacctggtccgcacccgggccgcccacgtcctcggccaccccgaccgggacgccgtcggcgccgaacggtcctt
ccgggacctcggggtcgactccctgggcgccgtggaactgcgcaaccagctcaacgccgccagcggcctgagcctgtccgcgac
cctcgtgttcgaccacccgacccccgccgccctcgccgagcacatgctgcggcagctcgtgcccgacgagaacggcaccaccga
caccaccgacgacgaggaagcggagatccggtccctgctgtcctcggtgtcactcgcccagctgcgcgacatcggcgtactggaa
cccctgctgcaactggccggacggggcacggcagacccggcaccggaggacgagcccggcgagtccatcgactccatggcctt
ggacgacctggtccgtgccgccctcgacaaccagtccgacgcgtcgcgcgattgacggagtagagacatgaacagcaccaacga
gaaggttgtcgaagcactccgtgccgcgatgaaggaagccgagcggctgcggcggcagaaccggcaactggtcaccgccgcca
ccgaaccgatcgccatcgtgagcatgagctgccggttccccggcgaggtgtactcgccggaggacctgtggcagctcgtcgcctcc
ggtacggacgccatctccggcttccccaccgaccgcggctgggacctggaggccctgcacaccagcgaggtcgacgaccgcgg
caccagcgtcagccagcgcggcggattcctcagctccgtcgccgacttcgaccccggcttcttcggcatctcgccccgcgaggcgg

tcaccatcgacccccagcagcgcctgctgctggagaccgggtgggaggccttcgaacgggccggcatcgacgcgaacgggctgc
gcggcagcaagaccggcgtcttcgtcggcaccaacggccaggactacgcctatctgaccgtcaggtccctggacgacgcgaccg
gcgacgtcggcaccggcatcgccgcgagcgccgcctccggccggctctcctacgcgctcggcctggaaggccccgcggtcacc
gtcgacacggcctgctcgtcgtcgctggtcgccctgcacctggccgtgcagtcgctgcgcaacgccgactgctcgctggcgctcgt
cggcggtgtcaacgtgatgtccacccccggctcgctggtcgagttcagccgtcagggcggcctcgcccgcgacggccgctgcaag
gcgttcgccgactcggccgacggcaccgggtggtccgagggcgtcggcgtgctgctcctggaacggctctccgacgcccagcgc
aacggccatcccgtactcgccgtggtgcgcggctccgccgtcaaccaggacggcgcctccaacggcttcaccgcgcccaacggc
cccgcccagcagcgggtcatccggcaggccctggagcgcgccgggctgtccccggcggacgtcgacgcggtcgaggcccacg
gcacgggcaccccgctcggcgaccccatcgaggcgcagagcctgctcgccacctacggccagggccgcgaacgcccccctgctg
ctggggacgatcaagtcgaacatcggccactcccaggccgccgccggtgtcgccggtgtcatcaagatggtcatggcgatgcggc
acggcgtcctgccgaagacgctgcacgccgacacgccctcgtcgcacgtggactggtcggcgggcgacgtctcgctgctgaccg
aggcccgtgactggcccgaggcggaccacccccggcgggccggtgtgtcgtccttcggcgtcagcggcaccaacgcgcacgtg
atcatcgagcaggcaccggcggacaccgcggcccccgaaacgcgggaggcgcccgcccggcagccgggcgtcctgccgtgg
gtgctgtccggccggaccgccgaggcgctgcgcgaccaggcggccgccctgctctcccgcctggagacggagccccgacccgc
cgcgccggacgtggcgctgaccctcgccacctccagggcgggcttcgagcaccggctggccgtcgtggccgacgacggcgacg
gcctgcgcgccgccctggccgcctgggccgcgaacggcaccgcccccggcgccgtggagggcctcgccgcgggccgggcga
agtccgcggtgctgttctcgggccagggctcgcagcggctcggcatgggccgggagctgtacggcgccttccccgccttcgccga
ggcgctggacgcggtgctcgccgagctggacccgctgctggaccgcccccctgcgggacgtgatgtggggcgacgacgccacgg
cgctgaacgagaccggccacacccagcccgcgctcttcgcgatcgaggtcgcgctgtaccgcctggtcgagtcgtggggcgtcac
ccccgacttcgtggccgggcactccatcggcgagatcgcggcggcccatgtggccggggtgttcccgctggcggacgcctgccgt
ctcgtggccgcccgcggccggctgatgcaggccctgcccgccggtggcgccatggtcgccgtccaggcgaccgaggacgaggt
ggtgccgctgctgaccgacacggtgtcgatcgccgcggtcaacggcccgtcgtcggtggtgatcgcgggggaggagaccgcggt
cctcgacatcgccggccgcctcgccgccgacggccgcaagacgtcacggctgtcggtctcgcacgccttccactcgccgctcatg
gagccgatgctggacgacttccgcacggtcgtcgccggcctgaccttcgccgagccggcgatccccgtcgtctcgaacctcaccgg
cgccctcgccgggaccggggagctgtgcgcccccgagtactgggtgcgccacgcccgggaggccgtgcgcttcgccgacgggg
cccgggccctcgccgccgaaggcgtcggcaccctcctggagttgggaccggacggcgtcctctccgccatggcccaggagtcgg
tccccgccggcaccctgaccgtgccggtcctgcgcaaggaccgccccgaggagaccgccgccgtcaccgccctggcccgcctcc
acacggccggcgtgcgcgtggactggcaggcattcttcgccggcaccgacgcccgccgggtggacctgcccacctacgccttcca
gcacgagcggttctggccgcaggtgtccatgagcgccggcgacgccgccggcctcggcctgacccccgccaaacacccccctgct
gggcgcgacgatggtgatggcgggctccgacgagctggtgctcaccggcaccctgtcgctgcgcacccacccctggctggccga
ccacgtcgtcagcggcatggtcttcttccccggcaccggcttcctggagctcgcgatccgggccgccgacgaggtcggctgcgcgt
ccgtcgaggacctcaccatcgtggcgccgctgatcctgcccgagcgcggcaccacccaggtgcagatccaggtgggcggcgcgg
acgaggacggccgccgtgagctgcgcttccactcgcgccccgccgacgcccccgaccaggactggtccctgcacgccaccggc

atgctcgccaaggacgaacggacgctgagcttcgacggcgcgcagtggccgccgcgcggcgccgactccgtcgacctggacgg
cctgtacgagcgctacgccgagacgggcctggactacggtcccgtcttccggggcctgcgcgcggtctggcgccgcggcgagga
actcttcgccgaggtctccctcgaccagcgggtcaaggacgccgacgccttcggcgtccacccggccctgctcgacgcggtgctgc
actcgaccgtcttcgccagcgccgacggcgaccagcggggcctgctgcccttctcctggagcggcgtctccctgcacgcctccggc
gcctccgtgctgcgcgtcaagatcaccaactgcggcccggacgccgtcgaactggtcgccgtcgacgtcgagggcgcccccgtca
tctcggtgacctcgctgaccctgcggtcggcctcgccccaggcggcggccgcgaacacccgccgcgacgagatgaactcgctgtt
ccacctggactgggtgccgcagaccgtgccagggatcaccgaccacaccggctgggcggtcctcggcgacgacaccctcgccct
ggccgacgccctgacatccgcgggcgtcaccccgctccacgccgccacgctcaccgagctggacacggcgacgggcaccatcg
ttgtccccgtcgtcggcgaccccacccgggttcccgagtcggcgcacgaagtgacccaccgcgtgctgcgcctcctccaggagtg
gcaggccgacgagcggttcgcccgctcccggctgctgatcgtgacccggggtgccgtcgcgaccggcgacgagagcgtcaccg
acgtcgccgcggcgcccgtatggggcctggtgcggtccgcgcaggcggagaaccccggccgcttcctcctcgtcgacctcgacg
actcggcggactccaccgccctgctccccaccctcccgggactgctcgaagccgacgagccgcaggccgtcgtccgcgccggca
ccgtacgggtcggccgcctcgcgcgcctcgcgtcggccggcgccctggtgcccccggcgggcaccccgtggcggctgggcagc
gccgccaagggcagcctcgacgggctggccctcatcccctgccccgaggtcctcgagccgctgaccggccgtcaggtccgcgtc
gacgtgcgggccgccggcgtcaacttccgcgacgtcctcaacgccctcggcatgtaccctggtgaggccgggctcttcggctccga
agccgcgggcgtcgtcaccgccaccggtccggacgtcaccggcctgcgccccggcgaccgcgtcatgggcatgctgttcggcgg
cttcggcccgctgggcgtcgtcgacgaacggctcctcacgcccgtccccgacgcctggtcgtgggagaccgccgcctcggtgccg
ctggtgttcctcaccgcctactacgccatggtcgacctcgccggactgcgccccggcgagaaggtcctcgtccacgcgggcgcgg
gcggcgtgggcatggccaccgtccaactggcccgccacctgggcgcggaggtcttcgccaccgccagcgagggcaagtgggac
gtcctgcggtcgctgggcgtcgccgacgaccacatcgcgtcgtcgcgcaccaccgacttcgagcaggccttcgccgccgtcaccg
acggtcgtggcgtcgacgtcgtcctcaacgccctgtccggcgaattcgtggacgcgtcgctgcgcctgctggcgcccggcggccg
cttcctggagatgggcaagaccgacatccgcgacgccgacaccctcccgggcgtcgactaccggtccttcgacctcggattcgccg
accccgaccggatccggcagatgctgaccgaactggtcgacctgttcgaccggggcgccctgcggccgctgcccatgaagacctg
ggacgtgcgtcgcgcgaaggacgccttccgctacatgagcctcgccaaacacgtcggcaagatcgtgctgaccaatcccgtcgcct
gggacccggacggcacggtcctgatcaccggtggcaccggcggtctcgccgccgaactggcccgccacctcgtcacggaacgct
cggtacgccacctgctgctggtcagccgccggggcccggacgcgccgggagcggcggagctccgcgaggagctgacggcgct
cggcgccgaggtggccgtcgtcgcgagcgacatcgccgatcgcgccgccgtggccggcctgctcgccgggatcccggcggagc
acccgctgacggccgtcgtccacaccgcgggcgtcctcgacgacggtgtcatcgcctcactcacccccgagcgcgtggccgccgt
gctccgcccgaaggtcgacgccgcctggcacctccacgagctgacccgcgacctggacctcgcggccttcgtcctctactcctcca
cggccggcgtcatgggcagccccggacagggcaactacgccgccggcaacaccttcctcgacgccctggcccaccaccggcac
agccagggcctgcccgcgacgtccctcgcgtggggcgcgtgggagcagggcgccggtatgacgagcaccctcgacgaccgcg
acgtacgccgcatcagcggcggcggcatgccgcccctgtcggtggagctgggcctcgcgctcttcgacgccgccaccacgaccg
acgaggcactggtcgtcccgctccccatcggctccggccccggcgcgctgccctcggcggacggcctgccggccctcctgcgcg

gcctcgtcgcgaccggccgccgctcggtggcgagcagcgccgcgcgcgtcccgcaaggacctggcccatcagctcaccggg
ctcggcgaggaggaacgcctacggttcctggtcgacctcgtccgcgccgaggccgccgtcgtcctcgcgcacccctcaccggagg
ccatcgccgccgaacgggagttccgccagctcggattcgactcgctgaccgcgatcgaactccgcaaccggctgtcgaccactacc
ggcatcgccctgtccgccaccctcgtcttcgactaccccacccccagaaggctcgcggagtacctgcgcgacgaactggtcggcgc
ggacaccgaagcgacgggcgggacccccgcgcccaaggccgcggcatccgacgaaccgatcgccatcgtcggcatgagctgc
cggttccccggcggcgtggactccccggaggccctgtgggacctcatcgcctccggcggcgacgcgatctcgggattcccggacg
accgcgactgggaccagcacgccatcggcggcggtgacctcaccggcgcgggcggcttcctgcgcggcgcggccgacttcgac
gccggattcttcggcatctcaccgcgcgaggccctggccatggacccgcagcagcgcctgctgctggaggtgtcgtgggaggccg
tcgagcgctccgggctcgacccggcggcgctgcgcggcagccggaccggcgtcttcgtcggcgtgagcgggcaggactactcca
cgctcatcatgcggtccaaggacgacgtggaaggccacgcgaccacgggcctcgcggtcagcgtcgcctccggccggctgtccta
caccctcggcctcgaaggcccggcggtctccgtcgacacggcctgttcctcgtcgctggtgtccctgcacctcgccgcgcaggcac
tgcgcagcggcgagtgctcactggccctggcgggcggtgtgacggtgatgtccaccgcgatgggcttcgccgggttctcccgcatg
ggcggactggcccacgacggccggtgccgggcgttctcggacgacgccaacggcaccggctggtccgagggtgtcggcatgct
ggtcgtcgagcgactgtcggacgcgcaagccaacgggcaccgcattctcgcggtggtgcgcggcagcgcggtcaaccaggacg
gtgcgtcgaacggtctgacggctccgaacggtccctcgcagcagcgggtcatccggcaggcgctggccagcggtggtctgtcccc
ggccgacgtcgacgccgtcgaggcccacggcaccggcaccccgctcggcgacccgatcgaggcgcaggcactgctggcgacg
tacgggcgtgaccgggacacggagcgtccgctgtggctcggttcggtgaagtcgaacctcggtcacacccaggcggcggccggt
gtggccggtgtcatcaagatggtgatggcgatgcggcagggcatcctgccgcgcacgctgcacgtgaacgagccgtcctcgcacg
tggagtggtcggcgggctcggtggagctgctgacggagcagacggcctggccggagggccgggtgcgccgggcgggtgtctcg
tcgttcggtatcagcgggacgaacgcccacgtcatcgtcgagcaggcgccgccggtcgcggcatcggcggagcgcccggacgtg
gtgccgggtgtggtgccgtgggtggtgtcggccaagtccgaggccgcgctggacgcgcagctggaccggatgcggtcgttcgcg
gccgacggcgcacaggacgccgcctcggtggacgtcggcttctcgctggcgacggcacgctcggcattcgaacaccgtgcggtgt
tgctgtcttcggccgagggtgcggtcgaggcggcgcgtggtgtggcggtcgatcgtccgttggcggtgttgttctcggggcagggtg
cgcagcggctggggatgggccgggagctgtacgagcgcttcccggtgttcgcggcggcgttcgacgaggttcttgccgagctgga
tccgttgctggaccggccgctgcgtgaggtgatgtggggtgaggacgccggtctgctgaacgagaccggttccacgcagcccgcg
ttgttcgcggtcgaggtggcgctgttcggtctggtgcggtcgtggggtgtgaagccggacttcctcgccggtcattcgatcggtgagg
tggcggccgcacatgtggcgggtgtgttctcgctggcggatgcctgccgactggtggcggcacgtgccgggttgatgcaggcgctg
ccgtcgggtggtgcgatggtggccgtccaggcgaccgaggccgaggtgctgccgctgctgaccgaggcggtgtcgatcgcggcg
gtcaacggtccgtcgtcggtggtcgtctcgggtgccgaggatgcggtgctggagattgcgggccgtctgtcggctgatgggcgtaa
gacgtcgcggttgtcggtgtcgcacgccttccactcgccgctgatggagccgatgctggatgacttccgtgcggtggtcggcggtct
gacgttccacgcaccgtcggtgccggtggtttccaacctgaccggtcgggtggctgctgcggaggagctgtgctcgccggagtact
gggtgcggcacgtccgtgaggccgtgcgtttcgccgacggggtacggaccctggtcggtgagggtgtgggcacgcttctggagtt
gggtccggacggtgtgttgtccgccatggctgcggagtcggtgtccgaagagaccgtgatggttccggttctgcggaaggaccggc

cggaggagaccgccgcggtcaccgcactggcgcggctgcacgtacagggcgtcgcggtcgactgggaggcgttcttcgccggc
accgccgcccgccgggtcgacctgcccacctatgccttccagcgtgagcggttctggccgcgactggccgacgccgccgccgtag
cgaccggtacggacgccgtggacgcggagttctgggccgcggtggagcgggaggacgtcgcgtccctcgcgtcgagcctggag
atcgacggcgattcgctcgaagccgtgctgcccgcgctgacatcgtggcgcactcgccgtcgcgtgcagtcggcggtggactcctg
gcgctaccacgaggtgtggcggccgctcacgggtgtgtcctcggccgcgccttccggggcgtggctggtgatcacctcggtcggc
atcaccgacgacgcgtgggtcgcttcggccgtcgcggcgctcggccccgacgcggtccgcctcgacgtcgacggcgaggaccg
cgcgcgactggccgagcggctcaccgaactggccgagaagggcccggagttcgcgggcgtcgtgtccctgctggcggtcgacg
agacgtgcggcgccacgggtgttcccgccggtctgaccggtaccaccgtcctgatccaggcgctgaacgacgcccggatcgcgg
ccccgctgtgggccgtgacgcggggcgcggtgtccgtgggccgctccgacgggctgcccggcctcgtgcagtccgcggtctggg
gactcggccgggtcgccgcactggagtacccgggacactggggcgggctggtcgacctgccggagacgctcgacgagcggtcg
gcacggcgcttcgccgccgtcctggcgggtgtggacggagaggaccaggtcgcggtccgggcgtcgggcgcgttcggccgccg
actggtgcccgcgccgagcggtgagcacgagaagcgctgggaaccctcgggcaccgtgctgatcaccggcggcaccggtgcgc
tgggcgggcacgtggcccgctggctggcccggcacggcgcgcggcatctgctgctgctcagccgcagtggtctggaggcgcccg
gcgcaccggagctgcgggacgaactgaccgcgctcggcgccgaggtcaccgtcaccgcctgcgacgcctccgaccgggccgg
cctcacggccgtcctcgcgtccgtaccggaggagtttccgctgaccgcggtggtgcacgcgtccggcacgctggacgacggtgtg
ctcgacgggctgaccgaggagcggttcgaggcggtgttccgctccaaggtcgccccggcgctcctgctggacgagctgacccgtg
agctggacctgtcggtgttcgccctgttctcctcgatcgcgggcgccgtcggcaaccccggccaggcgaactacgccgctgccaac
gtggtgctggacaccctcgccgaacggcggcgcgcggccggcctcccggcgacgtcgatcgcctgggggggcctgggcgggcg
acggtatggccgaccgggcggacgtgcgtacgggcggtgccgtcctggaccccgagtccgcgatcacggcactgtcccaggtgg
tggccgaaccggcggcgtcggtcgtgctcgccgacctgcgacagccacgcgtactggaggcgctcctgagcctccggccgagcg
cgctgctcgacgagctgcccgaagcgctccgggcggcggaggccgtgcgcgaggcccgccgggacagcgcgtcggcggtgtc
cggcctacgggaacagctgacgaacgggaccggcgccgagcgtctggccctggtgctttcgatggtgcgcacgcgggccgccgc
cgtcctcggacacaccggtgccgaggcgatcagcggcgacaaggccttccgggacttcggcttcgactcgttgacggccgtggaa
ctgcgcaaccagctcatgggcgcgaccggcctggccctgcccaccagcctcgtcttcgactaccccacccctcgtgccctcgccga
acacctcgtggccgagctggtgggcgagcggccggactcggacgctcccgtcgcgacccccgccgcgctcacggacgacccga
tcgtgatcgtcggcatggcgtgccgcttccccggcggggtgtactcgccgcaggaactgtgggagctgctggtcgacggccgcga
cggcatcgcgggattcccctcggaccgtggctgggacctcgacctcctcgccggcggcgggcagggcgccagcgcgaccggca
agggcggcttcctgtacgacgccgcggagttcgacgcggacttcttcgggatctcgccacgcgaggcggtggcgatggacccgca
gcagcgcctccttctcgaggtgtcctgggaggccttcgagcacgtcgggatcgacccggcgacgctgcgcggcagccagaccgg
cgtattcgtcggtaccaacgggcaggactacgccaatctcgtcctcaaggcgcgcgacgacgtggagggccacgcgggcaccgg
gctcgcggccagtgtcatctcgggcaggctgtcctacacgttcggtttcgagggtcccgcggtgaccgtggacaccgcgtgctcgtc
gtccctggtgtccttgcactgggccgcgcaggcactgcgcagtggcgagtgctcgctcgcactggccggtggcgtcaccctgatgg
cgacgtcgacgagcttcgccggcttcagccgccagggcggcctgtccccggacggccaggtcaaggcgttctccgacgacgcgg

acggcaccgcgtggtcggaaggcatcggcatgctcgtggtggagcggctctccgacgcccggcgcaacgggcacccggtgctcg
cggtggtccggggctccgcggtcaaccaggacggtgcgtcgaacggtctgacggcgccgaacggtccgtcgcagcagcgggtg
atccgccgggcgctggccggcggcggtctgtccccggccgatgtcgacgcggtggaggcgcacggcacgggtacgaagctggg
tgacccgatcgaggcgcaggcactgctggcgacgtacgggcgtgaccgtgacgcggagcgtccgctgtggctcggctcggtgaa
gtccaacctcggtcacacgcaggccgcggccggagtcgcgggtgtcatcaagatggtgatggcgatgcggcacggtgtgctgccg
cgcacgctgcacgtgagcgaaccctcgtcgcacgtggactggtcggcgggctcggtggagctgctgagggagaagacggcctgg
ccggagtcgggccgtgcgcgccgcgccggtgtctcgtcgttcggcatcagcggcacgaacgcgcacgtggtcctggagcaggcg
ccggccgccgaaccgccggtgacccagggaccggtggcggtcccgggtgtggtgccgtgggtggtgtcgggccggaccgagg
cggcgctggacgcccagctggagaggctgcggtcctacgcggccgacggtaccgcggcgtccccgctcgacgtcgggtactcgc
tggcggcgggccggtcggtgttcgagcaccgagcggtgctcctgtcctccgaggacggcgtcgtcgaggcggctcgcggtgccct
ggccgaccgccgaccggcggtgctgttctcgggacagggcgcgcagcggctgggcatgggccgggagctgtacgagcgtttccc
ggtgttcgccgaggccctggacgcggtgttcgcggagctggacctgtcgctggaccggccgttgcgcgaggtgatgtggggcgag
gacgccgctctgctgaacgggacgggttacacgcagcccgcgttgttcgcggtcgaggtggcgctgttccggctggcggagtcctg
ggggatcaccccggagtacgtggccggtcattcgatcggtgaggtggcggccgcgcatgtggcgggtgtgttcccgctgccggac
gcgtgccgcctggtggccgcccgttcccgtctgatgcaggcgctgcccgcgggtggcgcgatggtggccgtgcaggcgaccgag
gcggaggtgctgcctctgctgaccgacgaggtgtcgatcgcggcggtcaacggtccgtcgtcggtggtcgtctcgggtgtcgagga
cgcggtgctggagattgcgggcaagctgtcggccgagggccgtaagacgcatcgactgccggtgtcgcacgccttccactcgccg
ctgatggagccgatgctggaggacttccacgcggtggtcagcggtctgtcgttcaatgcaccgtcggtgccggtggtctccaacctc
accggcaggctcgccacgtccgaggaactgtgctcgcccgagtactgggtacgtcacgtccgtgagaccgtgcgcttcgccgacg
gagtacggaccctggtcggtgaaggcgtgaacaccctcctggagttgggcccggacggagtactctcggcgatggctgccgagag
cgtgcccgaggggacggtgacggttcccgtcctgcgcaaggaccggcccgaggagaccgcggccgtcaccgcactggcccggc
tgcacgtacagggcgtcacggtcgactggacggcgtacttcgccgggaccggtggtcgacgggtggacctgcccacctacgcctt
ccagcgcgagcgctattggccgcgcgcgtcggccgcggtgggcgacgtgtcgggcctggggctgacgtccgccgagcacccga
tgctgggtgccgcggtggtcatggccgggtcggacgaggtggtgctgaccggcaggctgtcgctcaccacgcacccgtggctggc
ggaccaccgggtcggcggtgcggtgtcgctgccgggtgccggcttcgcggaactcgccgtccgggcgggggaccaggtcggct
gcgaccgggtggacgacctcacgctccgggccccgctgacgctgcccgaacagggcgccgtgcagctccaggtgcggatcggt
acccccgacgacgagggcaggcgcccgctcggcgtgtactcctgccccgaaggcgcgacgggtgcgccgtggacgcggcacg
ccgaaggcacactcaccgacggtgccgaagacgccgccgccggattcgacgcgaccacctggccgcccaagggcgccgagca
ggtggacctggacggctactacgacgagtcggccgaggacggcgtcgcctacggcccggtgttccagggcctgagcgcggtatg
gcggcgcggcaccgaggtgttcgccgaggtcgcactgcccgagcaggtcacggacgcggcggcgttcgccgtgcatccggcgtt
gctcgaagccgtggcccaggccgccggtttcgcgatgccggaggacacggagcaccgcctggtggctgcctcgttcagcggcct
gtcggtgtacgccggagccgcgtcgagcgtccgggtgcgcctggccgccaccggcgcggacgcggtgtcgttcgccgcggtcga
catcggggggcgccccggtgctctccgcgaagtcgctggtcctgcgcccggccgaggtgggggagacggctgcccccggtgccg

ccgaggggggtggcgacgcttcgcgtcgagtggctcgacgcaccggacgcccacgccccggtggccgacggcggccggtgggc
cgtcgtcggtgccgacccgttcggcctcggcgacgccctggagtcggacggcggacggtccgtgcggcgggtggactcgctgac
gcagttgttcgacggcgaaggtgccgtcccgggcatggtcgtggtcccagtcgtccccgccggtgcgacggagccgctgcccgcg
tcggtgcgggccgtctcggcccgcgtgctggggctgctgcaggagtggtcggccgaccggaggctgtccgcctcgcggctggtgt
tcgtcacccgcggcggcgtcgccgtgggggacgaggacgtgcgcgacctggccgccgccgcggtgtggggcctggtgcggacc
gcccaggtggagagccccggcagcttcctgctggccgacctcgacgacacccttccgcagtccgcgatccccgctctcctggccg
ccgacgaggaccagttggcggtgcgggacgaggccgtccgggtcgcgcggctggtccgcgtgcccaaggaggaggtgccgga
cgcacggacctgggacgcggacggcacggtgctcatcaccggcggtacggggcgtctgggcggtgagctggcccgccacctgg
tcgccgagaagggtgtgcggcatctgctgctggccggccgccgggggccgaaggcgcccggggcggcggagctggtggcgga
cctgacggccgccggggccgaggtcaccgtcgccgcctgcgacatcgcggaccgcgccgacctggccgcgctgctcgcgtcgg
tccccgccggacacccgctgacggcggtcgtgcacacagccggtgtcagcgacgacggtgcgatcgcctcgctgacgcccgagc
gactgggcacggccttccggccgaaggtggacgcggcgtggaacctgcacgaggcgacgcgcgagctggacctgtcggcattc
gttctgcactcctccgtctccggtgtgctgggcagccccggccgggccggctactcggcgggcaacgcgttcctggacgcgctggc
ccaccaccgccgcggcctggggctcgcggcgatgtcggtggcctgggggccctggggccagggcagcggcacgaccggcgcc
ctgagcgccgaggacaaggagcgcatggcgcgttccgggatgtcgccgctgaccctcgaacagggcatgaggctcttcgacgag
gcgttcgacctgaatgaggctctggtggtctccctagggctgacggctgaacgctcctctcgatcggccggtgagataccgtcgattc
tccgagggctggtacggacccgtcgccgcactgcggcatccggagggcggtcggccggcctgctccagcgcttggaaggggtgc
gcgagaacgagcgggcgcagatgctggcggagttggtgcggaccgaggtcgctgccgtgctgggacaccccacaccggacacg
atcgtggccgaccaggagttccgtcggctgggcttcgactccttgaccgcggttgagctgcgcaaccggctgaattccgcgacggg
gttgaaactgcccaccaccttggtgttcgaccaccccactccgctggctgtgtccgagtacctcgcggcggccctgctgggcggctc
cacaccggacaccggactgtccccgctggccgaactcgaccggctcgaagcggcgctgtcggcgggcgaccccgacgaggtca
cgaagtccggagtcgtggtgcggcttcggcagttgctggcccggttgagtgaaaccggcacggagaccggaggactcgcggtga
cggaacgcatcgaggcggcgagtaccgacgaggtgttcgccttcattgacaacgagctcggccggatgaatgaccgctgagcccc
ggtcgatttttacaaggaaggttccctgaggatgccggacgacaagaaactcgtcgactacctgaagtgggtcaccgccgatctgca
cgagacccggcagcgcctgcgggaggtcgaatccggccggcacgagccggtggcgatcgtgggaatggcctgccggctgcctg
gcgaagtccgctccccggaggaactgtgggatctcgtcgcctccggcggtgacgcgatcaacagcttcccgaacgaccgtggctg
ggacctggatgtgctggccgccgacggacagggcggcagcgcgacgctcgagagcggattcctctacgacctcgcggacttcgat
ccggggttcttcgggatctcgccgcgtgaggcggtggcgatggacccccagcagcgcctgctgctggaggtgtcctgggaggcga
tggaacgcgcgggaatcgatccggtctcgctgcggggcaccaggaccggtgtcttcgtgggcaccagcgaccaggactacgtcca
cctggtgctggcctcgcaggacgacatggcggctacgccggtaccggtctggcggcgagcgtcctctcgggcaggctgtccttca
ccctcgggctcgaagggcccgcggtgacggtggacacggcctgctcctcgtcgctggtctcgctgcacctcgcggcgcaggcgct
gcgcaacggtgagtgctcgctggcactggccggtggcgcgacggtgatggcgacctccgcgaacttcgccggattcagccaacag
ggcggcctcgcacccgacgggcgctgcaagtccttctccgatgccgcgaacggcaccgcctggtcggagggcgccggcatgctg

ctggtggagcggctctccgacgcccggcgcaacggccacccggtcctcgcggtgctgcggggcagtgccgtgaaccaggacgg
cgcgtcgaacggtctgacggcgcccaacggcccctcgcagcagcgggtgatccgccaggccctggccagtggtggtctgtcacc
ggccgacgtggacgcggtcgaggcgcacggcacgggcaccacgctgggtgacccgatcgaggcacaggcgctcctgaccacct
acggccaggaccgggagcgcccgctgctgctgggctcgctgaagtcgaacatcggccacacgcaggccgcctccggcgtcgcc
ggtgtgatcaagacggtcatggcgatacagcacggagtgctgccccggagcctgcatctggacaccccgtcctcgcacgtggactg
ggacgccggtgcggtggagctgttgacggacagcagggcatggccggagacgggccgtgtgcgacgtgccggtgtgtcgtcgtt
cggcatcagcggcacgaacgcgcacgtgatcctcgagcaggccccgtcggacgaggacgccgcattcgacagcgttgtggagcc
cggtgtggtgccgtgggcgctgtcgggcaagtccgccgaggcgctgcgcgggcaggcggcacggttgctgtcgtggctccagga
gcaccccgaggcgcggccggcggacatcggattctcgctcgcgacgggccggacggccttcgagtaccgggccgtcgtaccgg
ccgccgacctggcggaagcggtggacgccctgacggcactggccgccgacgagccgacggccgcggtggtccacggcgtggt
ctcgggtggaaagctcgcgttcctgttctccgggcagggcagtcagcggatcggcatggccgcgagctgtacgagcgcttccccg
tcttcgccgaggcgctggacgccgtcctcgcccggctggacccggcgttggaccgcccgctgcgggacgtgatgtgggccgacg
acccggagccgctgaaccggacgggcttcatccagcccgcgctgttcgcggtggaggtggcgctgttccggctgctggaatcgtg
gggcgtcacccccgacttcgtggcgggccactccatcggcgagatcgcggccgcccatgtggcgggggtgttctcgctggaggac
gcctgccgactcgtgacggcccgcgcggggctgatgcaggcgctgccgacgggcggggcgatggtggcgctgcaggccaccg
aggaggaggtcctgccgctgctgtccgaccggctgtcgctcgccgcggtcaacggcccctcgtccgtggtgatctccggcgacga
ggacgcggcactggaggtcgcggcccggctgtcggcccagggccgcaagacgtccaggctgccggtcagccacgcggcccact
cgccgctgatggacgcggtgctggacgacttccgcgccgtcgtacagggcctgtcgccccggacgccggccatcccggtggtctc
ccacctcaccggcgacgtggcggtggccgaggaactgtgctcggtcgactactgggtgcggcacgtacgcgaggcggtgcgcttc
gccgacggcacgcggacgctcctcaagcagggcgtgaccaccttcgtcgaactgggcccggacgccgtgctgtcggctctggtcc
aggagacactcgcggacgagacggtggtcgtcgtacccgtcctgcgcaggaaccgtcccgaggaactcaccgccgtcaccgccc
tggccgagctgcacgccggcggcgtcacggtggactgggacgcgttcttccggggcacgggcacgcgtcgcgtcgacctgccga
cgtacgcgttccagcgtgagcggttctggccgacggtctcggcgcgcggcggtgacgccgcgggtctcgggctgatacccgccga
gcatccactgctgggcgcggcgatgtccgtggcgggctccgacgagctgctcctgaccggaacgctgtccgtggccacgcacccc
tggctggccgatcaccgggtcatgggccggatcaccctgcccggcacggcgttcgccgaactggccgtccgggccggggaccag
gcgggttgcgaccgggtggcggagctgaccctggacgccccgctggtcctcgctgcgcggggcgcggtctcggtccaggtgcgc
gtcggggaggcggacgagtccggccgacgcgcggtcaccgtccactcccggccaacggacgccgtggacggggactggacgt
cgcacgcccacggtgtgctcgtcgcctgtgagccgacgggcgggaccgagggggccgacgggctcgacgacggtgtgtggccc
cccaagggagcgcagccggtcgacctcgccgacctgtatccgcgactcgaccgggccggactggcctacgggccggtgttccag
gggctgcgggccgcgtggcgccgtgacggcgagtggtacgcggaggtgtcgctcacccaagaggcggcgtccaccgggacctt
cgcgatacacccggcgctgctggacggtgtcgtccgggccgccggtctgctggccgccggtgactttgacggtggtgtcgtgcccg
gttcgtggggcgggctcaccctgcacgcctccggtgcctcgacgctccgcgtcaggctggccgccacggacggggattccctgtc
gttcacggccgtcgacacggcgggcgccccggtggtctcggcggagtcgctcacgctgcgtccgctctccggcgaacagcgggt

ggcggtcgcggcagcaggcggccaggcatcgctgttcggtgtcgagtgggccaaggtcccgacgggttccgcgcccgtggcgg
gcacccgttgggccatcgtcggtgccgatgaactcgacctcgcctacgcgatgcaccgggccgacgagtcgatcaccggatacgg
ggacaccctggccggggcgatcggcgacagcggcgtcgcgccggacgtcttcctggtgcccgtcgtgggtgacgcggcggacg
acggacccgcgtcggtgcgcgcgttgacgacctgggtgctgggtctgctgcaggaatggctggcggacgagcggctggcgaact
cacggctcgtgttcgtgacccggggcgccatcgccctcgacggcgaggacgtccgggacctggccgcggcctcggtatggggcc
tggtgcgctccgcccagaccgagaaccccggcagcttcctgctggtggacctggacgacgcgttcgtctcggccgcggagctgcc
cggcctgctcaccctggacgagcagcaactggcggtgcgtgcgaacaccgtccgcgtggcccgtatggcacggctgcccgagcg
gagtccggcaggaccggcagtggacggtggcggcccgtgggccggccgcgggacggtgctgatcaccggtggtacgggtggc
ctcggcagcggcctggcgcgtcacctggcggccgaacacggcgtgcagcgactgctgttggtcagccgccgtggcgaatcggcg
ccgggggtcgcggaactgcgggcggagctgggtgcgctgggcgccgaggtctccgtcgccgcgtgcgacgtggccgaccggg
acgcggtggccgcgctggtggcgggcgtgccggccgagcatccgctcacggccgtggtgcacaccgccggtgtcctggacgac
gcgatgctgggttcgttgacgcctgagctcatggagcgggtgctgcggccgaaggtggacgcggcgtggcacctgcacgaggcg
acgcgcgggctggatctgtcggcgttcgtgctgtactcgtccgtctccggtgtgctgggtgccccgggacagggcaactacgccgc
ggccaacgcgttcttggacgcgctggtccggcaccgcaggggggctgggcctgccgggtacctcgctggcctggggcccgtgggc
cgagggcagcggcatgaccggctccctgaacaagtcggacgtggaacggatgtcccgatcgggtatgcccccactgtccatggaa
cagggcctggccctgttcgacgccgcgatcggccgggcggaaacgccggtcgtcccggcccgcgtcaacatcgcgggactgca
ggtgcagcaggcgctgccggccctgtggcgggacctggtcccggccgcgcgtcgcgcggccacggcgggggacaggtccgcc
gtcacgctgcgggagcggctgcgaggtctcgaaacggcggagcaggcaagcctcttcatggaggtggtggtggggtacgccgcc
ggtctgctcggccaccgggacgcaagcgcgatcaaccccgagcgtcgcttcctcgaactcggcttcgactcgctggcctccgtcgg
actccgcaaccagctcgccgacgtgctcggcatgcgactgccgtcctcagtggtgttcgacaacgagacgcccgtcaagctcgccg
ggtggctgcacaccgagttcgtgaaccaacacgatccgaaagccgcgcccacggcggccggtggcgcgcgaacggcccaccac
gcggacgagacggtggaagggctgttcttcaacgcggcgaaggccggcaagctggtcgagggcatgcgcctgctgagggcggt
cgcgaacacccggcccaccttcgagagcccggccgagctggaggagctgtccgctccggtcaccctggccgaaggcccgagca
ccccccgtctggtcttcgtcagcgcgccgggtgccaccgggggcgtccaccagtacgcccgtatcgcggcgcacttccggggcaa
gcggcatgtctccgcgctcccgctcatgggattcgcgccgggtgaaccgctgcccgccaccagcgaggccgcgggcagaatcgt
cgcggagagcgccctgcacgccagtgacggcgagccgttcgtcctgatcggccactcctccggcggctcgctcgcctatcaggcc
gcgggtgtgctggaggagacgtggggggtccgtcccgaggcggtcgtcctgctggacaccccgagcgttcgctacgagccgggt
gaaggaaacgacctcgaccagaccatgcgcttctacctggccgacgtcgactccccgtcggtcaacctcaacagtgcgcggatgtc
ggccatggcccactggttcatggccatgaccagcatcgaagcatcccccacgaccgctccgaagctcctggtcaagtgctcgctgc
cctgcaacggcctcatcttcgacacctcgtcggtgcccgtggacgaggtgcgcacgatcgaggccgaccacctgtcgctcgccaag
gagcactcggcactgacggcgcgggcgatcgaggagtgggtccgggccctgccggccgccggcgcgtgaccccacccggtca
ccgggacccgtatgtggcgtggccgccccggaaaccggggcggccacgccacgccccctcgtccaaccgccagaaggaggctc
catgaccacgtcctacgaactgcccgagatcaccgccgacccgcccgccgtactgaggctgagcccgctgctgcgcggcctgcag

gcccgaagcccggtctgccgggtcaagacccccgccggcgacgaggcatggctcgtgacccgtcacgccgagctcaagcaactg
ctgctcgacgagcgtctcggccgctcgcaccccgacccggcgaacgccccgcggtacgtcaggaacccgttcctggacatgctga
tcaccgacgccgaccccgagagcgcgcgcaagctgcacgccgagacccgcacactgctcacaccgaacttctccgccaagcgcg
tccgcgatctgaagccccgtgtggaggccatcgcggagggcgtgctggagggtttcgtcgcccaggggccgccggccgatctgca
cgggcagctgtccctgccgtactccctgatggtgctctgcgaactcatcggcgtgccgccgcaggaccgggcgcgcctggtggag
ctgctgtccggcatggccggggtggacgacccacagcgggtcgtggaggccagcaggccctcttcgcctacctggtggagctgg
cgggtcgtaaacaggccgaccccggcgacgacgtgatgtcccggctgtgcgagaccggggccccggccgagaaggtcggcccc
atggccgcggggctgctgttcgccgggctggacgccgtcgcgagccacgtcgacctcggcgtggtgctgctcgccaccaaccccg
acgcgcggcaggccgcgctgggggacccgcgcgtcatggccgccgccgtcgaggagatcctgcggacggccaaggccggtgg
ttcggtgctgccgcggtacgccagcgccgccgtcgagatcggcgaggtgaccatcgaggcgggcgacctcgtcctgctcgacttc
accctccccaacttcgacgagcaggtcttcgacgagccggagcgcttcgacatcacccgctcgcccaacccgcatctgacgttcgg
ccacggcatgtggcactgcgtcggcgccccgctggcccggctggagctgcggacgatgtacaccctgctcttcacccggctgccc
gagctgcggctcgacctgccggtggaggacctccaggtgctgggcggacgcctctcgggcggtctcaccgaactgcccatgcggt
ggtgacggccccgcccggaaggtgaaacgggccccgcccccaccatcaggtgggggcggggcccgtttcgtgccaccggtacg
tacggcggctcagtcctcgtgcacggtgatcgcctgggacgggcagatcctgcccgccagccgcacgtcctccgccaccggctcc
gccggctccccgtcgaggaccatgacgatcccgtcgtcgtcctggtcgaagacctccggcgccgacagcacgcactgccctgctc
cgatacaactgtcgatgtccacgctgatacgaaccatcattcctccatcggcgaatcgataccttctcgcgtgccccggtactcgcgtg
ccccggtacatgacgggtcggacgggggagtccgagtcccccgtccgacccgcgggcgagcgccgctaccagcgcacgggca
gcgcgtgcagcccgaacagcacgccgtcgtacttcagcggcagctcctcgaccggcaccgcgagttccagcgtcgggatccgctc
gaacagcgaccggtaggcgagctccatctcgacccggaccaggttctgccccaggcactggtgcaccccgtatccgaacgccacg
tggtggcgcgcggaccggctgggatcgaactggtgcgggcactcgaagacggtctcgtcgtggttggccgcggcgacgagcggg
acgatgccctcgcccgccttgatgagctgcccgccgatctccacgtcctcgacggccacccggaaggccaccaggtcggccacgg
agtagtagcgcagcagctcctccaccgcccggtcgtcgccgatccactgcgggttcatcagcagcgtcaccgcgccgagcccgat
gttgttcgccgtcgtctcgtggccggcgatgagcaacagcatccccacgcccgacagttcctgcggggtcagcttgccgtccgccag
cagccggctcatcaggtcgtcgcccggccgcttcgtcttgagcgcgatcagccggttgagatagcgcagcagcgccttggtggccg
cctcccgctgcccgtcggtcgaggtcagagtgaccagcacccgggtctgggcctcgaagaagtcgcagtcggccttcggtacgcc
cagcagcctggagatcaccagcgacggcaccggcagcgcgaagtccgagatgagatcggcggagttgcccccggcgagcatgg
cgtcgatgcgctcgtcgacgatcgcctggatcaccggccgcatctccctgatgcggcggacggtgaattccgggatcagcgtcttgc
ggaaccgggtgtgctccggcgggtccagggccacgaaccagccgggcacctggtcctgggccggcacgccaccggtcttcgcc
ggacgggggaagcccgggtgcgaggggttggagctgatccgggggtcggtgagcacggcacggacctcttcgtgccgggtcac
cagccagaccggggcaccggtgggcagcgtcgagcggacgagcccctcgcgcttgcgcaggccctcgtactccgcgggcggat
agggctggccgggccgccgcaacgggaagccgaccaggttcgaatcggtggtagtcatgtgtgcaattccctgagggtcggaacg
tcccggtcgttggcagttggtgccgagggggcggccgccggccgccccctcgggccggtcagcgcgcgccgtagaactcgc

ggatcttcccgacgatgtaggtctggtcctcttcggtcaggcccgtgtgcgtcggcaggtagaagccgtcctcggcgaacctggtcg
cgttcagcgtcggccagaccgggtcgaggtagccgggctgcatgctcatcggcttgaagaacacccgggtctcgatggaggcgtc
ggccaggaacgcgcgcagctcctcgcggcgctcggcgcgcaggtcgtacatccacagcacgtcccgcgggggcatcagggtga
tgccctcgatgtcccgcagcccctcgtcgtagcgcttctctatctcccggcgggtggccaggatctcgtccagctgctcgacctgggc
cagcgcgacggcggcctggaggttcgtcatacggaagttgtaagcgagcttcttgtgcaggaagttgtgttccttgttgaaggccatg
ccccgcagatgggccatctgctccgccagccgggggtcgttcgtgaggcagatgccgccctcgccagaggtgatgatcttgttggc
gaagagcgagaaacaggcgatgtcgccgaccggccgtacgccgtgcgcctccgccgtgtcctcgacgacccgcaggttgtagtc
gtgggccagctccatgatcgcgtccatgtcgcaccggcgcccgtagatgtgcaccggcatgatgaccttcgtgcgcggggtgatctt
ctcctcgatgcgggagacgtcgatattgagatcgtccccgcagtccacgaacaccggggtcgcgcccagatacgtcgccgcccac
gcggtcgcgatcatcgtgaattcgggaatcagcacctcgtcgcccgggccgacacccagcgcccgcagggcgagggtcagcgcc
gtggtcccggaggaacaggcgacgccgtgcgcgacgccgttgtaccgcgcgaattcgctctcgaattccttgaccttcggcccctgc
gacgagatccagccgtcgttgatcgcctcggtcacataggaaagctcccgccccttgagccagggctgcgaaaccggatacgtgaa
tgccatggaaacctcgctcagtcctttgccagtgcggggagtccaaggatcagatcggccgcggcgccccgcccgccggccgccc
gcagaacctgtccgaagtgctcggcgcgctcgcggaacgaacggtcgctgagcaccctggccagcttgtcgaccacgtcgtccgc
gtcgatggtctcggggcggtcgagcttcaggctcaccccgaagtcctggccgcgccgggcctggtcgtcgcagtccacccacaac
ggccgcacgaccaggggcttgccgaaatacaggccctcgtggaaaccgttgccgcccgcgtgggtgaagaagaccttcacgctcg
ggtgggccagcacatcgagctgggagggaacccagtccacgatccgcagattgtccggcagcagctccgccgagggcagcagct
tctgctgctcctggggcagcttccacaggaattggtgccggtcgctcatccgccgtgcgacctccaccagcgacgcgatgtgctccc
gcgtcatcctggtgatcgtgccgaagcccatgtagaccacggagtcgttgtgcgcgagccattcggagagaccgtcgtcctcgggc
gcctggggcagcggcggcaccatcgtgccgaccagcttcatcttggcgggaacggggaacgggtagtccagttcgggcaccgaa
tagcacaggaccatctcggacttgtcgatccggaccatcatcccgcgcgcctcgggggagatgcccagctccgcgcggacctgatt
gtcctcctccaccaccttgcgcatgtcggaggtcatgaacagccccagcgtgcgcattctgaacagctggttggccacccgctggac
gggggtcatgtccaacggcagaccggtgtgcggcacgggaaagtgaggcggcgtgtgcgacttggcgaagggcacgtgcgagg
tcagcacattgctgggcaggaacggcacacccagcacgaacgggatcttcttggtgaccgccagttcgtacgcgtactggcacatg
ctctcgatgaccatcagcgccggctcgacctcgtccaccacggcctcgagcgcccggtacttctccacccgcgagcgcggggcga
acgagtgcttgatgaccgcccggtgcgccttgaacctcgaccgctgcgtcacctcccggtacgtcttgtcgtcccaggtgacggccg
acatctcggacaccacctcgcccagggaagcgaactccaccggggtgtccaccttgagcgcctcgatctccgagcgccggttctcg
tccgtcgcgaaccacaggtcctcgactccccggcgggacagctcctcggccaggacgagcagcggattcaacagtccgctttcgg
gataactgacgaacagaatcggccgccgagtcgagcccatgacaataaccctccagaagtggcggcccggcagctcggcgccag
gtgtgcgcacggccggtcgccgagtggccgcgttcatcgatgcccgcgcccacagccggcgtcgtccgcccgggacgtcaaggg
acaagagtgcgggaagaagcgtgacagttacttggtctcacgccaggtcgaagagaatccctagtcttcgcgaccgacggatcgac
cggaccgccggaccgtggccgtccgtagcggatatgacgcgacgtcagacgagagatcacgttcttcttgtagggaaatctacagc
cttcccgcccatacgatccggctcgaagtgaagtatccgagccgacattcctcatgggtggtgcagcgtcatgacgagcggagcag

acagcgcgtcctgcggcgcgggcgatcagggggccggagcgcacgccgacgacccggtggtgatcgtcgggacggcgtgccg
gtaccccggcggcgtacggtcggccgcggacctgtgggacgtggtcgcctccggccaggacgtgatctccgcgttccccgccgac
cgcggctgggacctggaggccctcgccggtaccggccggggctccagcgccacccgtgagggcggattcctctacgacgcggc
cgacttcgacgccgggttcttcggcatctcccccgggaggcgctgggcatggacccgcagcagcgcctgacgctggaggtgtcc
tgggaggccgtggaacacgccgggatcgacccggtgagcctgcgcggcagccggaccggcgtcttcgtcggcacgaacggcca
ggactacgcgctcgtcacccagctgtcgacggaggacctggaagggcactccctgaccggactgtcccccgcggtggcctccggc
cgggtggcctacaccctcggactggagggccggcgatcaccgtggacacggccgcctcctcctccctggtcgccctgcactggg
cggcgcagtcgctgcgcgcgggggaatgctcgctcgccctggccggcggggtgacggtcatgtccaccccgacggccttcgtcg
ggcactcccggcagggcggcatggctcccgacgggcggtgcagggtgttctccgacgccgcggacggcacggcttgggccgag
ggcgtcggcatggtgctgctggaacggctgtcggacgcgcggcgcaacggtcatccggtgctggcggtgctgcggggcagtgcg
gtgaatcaggatggtgcgtcgaacggtctgacggcgcccaatggtccgtcgcagcagcgggtgatccgtcaggcgttggccaatgc
gggtctcactgcggctgatgtggatgcggtggaggcgcacggtacgggtaccaagctgggtgatccgatcgaggcgcaggcgctg
ctggccacgtacggccagggtcgtgaggtgccgttgcggctgggttcgttgaagtcgaacatcggtcatacgcaggccgctgccgg
tgtcgcgggtgtcatcaagatggtcatggcgatgcgccacggcgtcctccccaagaccctgcacagcgacaccgcctcctcccatgt
ggactggtcggcgggcgccgtggaactgctcaccgactcggtgccgtggccggagacggggcgcgcccggcgggccggggtg
tcgtcgttcggcatcagcggcaccaacgcgcacgcggtgctggagcaggccccgccggtcgacgaaccggtacgggagccggg
cacgggcacggcgcccgccgtggtgccgtgggtgatatccgcccgcaccgaggaagcgctcgacgcccaactggaccgcgtca
ggccgctcgcggccgccgacgccggactctcaccggtggacgtcggcttctcactggcggccggccggtcggtgttcgagcacc
gcgcggtgctgctcgccgcacggggcgagacggccctggcggcacgtggcacggcgggcggcgcgggagcactggcggcgc
tgttcccggggcaggggagccagcgcctcggcatggccgggagctgtacggccggttcccggtgttcgccgacgccctggaca
aggcgctgtccgtcctggaccgctacctgccgcgcccgctccgggacgtgatgtggggcgacgaccccgaggagctgaacctca
ccggctacacccagaccgccctgttcgcggtggaggtggcgctgttccgtctggtggagtccttcggcgtcacaccggacttcgtgg
ccggccactccatcggcgaggtggcggccgcccatgtggccggggtgttctcgttggaggacgcctgccggctggtggccgaac
gcggacggctgatgcaggaggtcgcggaccgcggcgcgatggtggccgtccaggcgaccgaggacgaggtgctgccgctgctg
accgagaaggtgtcgatcgccgcggtcaacggcccgtcgtcggtggtcgtctccggcaccgaggacgcggtcctggagatcgcg
ggacgactggcggccgacgggcgcaagacctcgcggctgcgggtgacgatggccgcccactcgccgctgatggaatcgatgctg
gacgcgttccgcgcggtgctcgaaggcctgacgttctccgcgccgaccctgcccgtggtctccaacctcaccggccgcatcgccga
gcccgaggagctgtgcgcgccggagtactgggtgcggcacgtacgccgggccgtccgcttcgccgacgggatcacgaccctgca
cacggccggcgtcggcgtcaccctcgaactgggccccggcggcatcctctccgccatggcccgggactgcctgggtgcggcgga
cctcacggtggtgcccgcgctgcgcgacgaccgcggcgaggaggccgccctggtgaccgcgctgagccgcctgcacgtcatcg
gcgcgccggtggactggccgcggatgttcgagggcacggccgcccaccgggtggacctgccgacgtacgccttccagcacgag
cgtttctggccgatcggcgcggacaccacgctgcggcacacggcggtcgacggtgaccatgccgaggcgcggcccgcgcccgc
accggccggcggggcgcggccgctcggcgagtggctggagtcgatgcccgccgccaagcgcgccgggtacctgctcgacctcg

tacgctccgaggccgccgccgtgctcgggcacggcggcgtggggcgcgtcggcgcccaccacatcttcaaggaactgggcttcg
actcgctgaccgccgtcgaactctgtgaccggctgagcgccgtcaccgggctacggctgcccagctcgctcgtcttcgactgcccg
acaccggcgtcggtcgccgaccacctgctggcgcacctcgtggacggctccccggagtcggctctccgcgaactcgacacgctgg
aagccgtggtgcgggccgcggcggtggacggcacggacgccgagcgcgcggcgatcgccgcacggctggaccaactcgtcct
ctccctaagggaattgacgcgaacggcggagctgcccgacgactccgaagaggacatcaagacggcgtcggtcgacagactgct
cgacatcatcgacgaagaattcgaaatcgcttaaagatcgacagctttcgttcataacgggatgaggacggaccggtgcaggaagac
cagcaggacaaagttgtcgactacttgaggcgagtcgccgcggacctccgacgtgcgcgccagcgcatcgacgaactggagtcca
agagccgcgagccgatcgcgatcgtgggcatgggctgccggttccccggcggggtcgcctcgccggaggggctgtgggacctg
gtcgcgtccgagggtgacgccatctccggcttcccggccgaccgcggctgggacctggccgcgctgaccggcgacggcgaggg
ccacagcgcgacccacgagggcggattcctctacgaggcgggcgacttcgacgccgggttcttcgggatctccccgcgtgaggcg
ctgggcatggacccgcagcagcggctgctgctggaggtctcctgggaggcgctggagcgggccggcatcgacccgctgtcgctg
cgcggcagccaggcgggcgtcttcgtcggctcgtaccactggaacccgggcacggcccgggcgacgggcgaactggacggcc
acgcgctgaccggcaccgcggccagcgtcctgtcgggacgcctcgcctacacactcggcctcgagggcccggcgctcaccgtcg
acaccgcctgctcgtcctcgctggtggccctgcacgtggcgatgcggtcgctgcgtgacgacgagtgctcgctcgccgtcgtcggc
ggcgtcaccgtcctgtccgaccccgccgtgttcgtcgagttcagcaagcagggcggactggcccccgacggccgctgcaaggcgt
tctccgacgccgcggacggcacgggctggtccgagggcgtcggcgcactcgtcatcgagcggctgtcggacgcgcggcgcaac
ggtcatccggtgctggcggtgctgcggggcagtgcggtgaatcaggatggtgcgtcgaacggtctgacggcgcccaatggtccgt
cgcagcagcgggtgatccgtcaggcgttggccaatgcgggtctcacggcggctgatgtggatgcggtggaggcgcacggtacgg
gtaccaagctgggtgatccgatcgaggcgcaggcgctgctggccacctatggccagggtcgtgaggtgccgttgcggctgggttcg
ttgaagtcgaacatcggtcatacgcaggccgctgccggtgtcgcgggcgtgatcaagatggtcatggcgatgcgccacggcgtcct
ccccaagaccctgcacagcgacaccgcctcctcccatgtggactggtcggcgggcgccgtggaactgctcacggagtcggtcgag
tggccggcgggggagcggccgcgccgctgcgcggtgtcgtccttcggcgtcagcgggacgaacgcgcacgcggtgctggagg
aagcaccgcaggaggagcccgccgtcgaggagccgccgaccacggacaacaccgccacggtggtcccctgggtgctctccggc
cgggccccggccgccgtgcgcgaccaggccgcgaaccttctctcccggctggacgccgaacccggcctccgcgcggtcgacgt
ggggtactccctcgcctcccggtcgctgttcgagcaccgcgccgtggtgctgggcgccggcatggacgacctgcgggagggactc
ggcaccctggcggccgaggagagcgccgcagccgtcgtccagggtgtcgcggacatcgagggcaagacggtgttcgtgttcccg
gggcagggttcgcagtgggtgggcatgggtgcccggctgctggaggagtcgcccgtcttcgccgagaggctggcggagtgcgcg
gaggcgctgggcaccttcgtggactggtcgttgacggacgtcctgcgccagatgccgggtgcgccgtccctggagcgggtcgacg
tcgtacagccggcgtcgttcgcggtgatggtgtcgttggcggcgttgtggcaggcgcatggtgtgcgtcctgatgctgtggtgggtca
ttctcagggtgagatcgccgcggctgtggtgtcgggtgcgttgtcgttggaggacggcgcgcgggtggtggcgttgcggagccag
gcgatcggccgtcggctggcggggcgtggcgggatgatgtcggtcgcgctgccggtcgcggaggtcgaggagcggctggacgg
tttcgcgggccgggtgtcggtcgcggcggtcaacgggccgcgttcggtggtggtttccggtgatccgcagggtctggacgacctgtt
cgcgcagctcacggacgagggtgttcgggtccggcggatcgcggtggactacgcctcgcactccgctcaggtcgaggaccttcac

gaggagctgctgtccgagctgggaccgatccgtccccgcgagtccgaggtgccgttcgtctcgacggtgtcgggcgagtggctgg
acaccagcgggatggacgccgagtactggtacggcaacctgcgcaacaccgtccggttcgaagcgggcatacggacgctgctcg
acgcggaccacggcgtgttcgtggaggtcagctcgcaccccgtgctggcgatggccgttcaggacgtcatcgaggacgccggcg
gtcacgccgtcgccgtggggaccctgcggcgctacgagggcgacctgtaccgcttcctgacctcgctggcggaggtgtacgtccg
gggcgtcccggtcgactggcggtccgtccacaccggcaccggtgcccgccgggtggacctgccgacctacgcgttccagcacga
acgcctctgggccatgccgcacgaggtcgagctcctcgcggcggcggaccccgccgacgccgagttctgggccgccgtggagc
gggaggacctgggctcgctcacctcccgtctgcacatcgacgaggactccctcgccgccgtgctgcccgccctgtcgtcgtggcgc
cgtgagcggcgtgagcagtccgcgctggacgcgtggcgctaccgcgtcgcctggtcgccgctcagcggtgtcccgcaggcttcgg
tgtcgggcacctggctggtggtcggtgccgaaggcatcgcggacgacgacgtggtggccgcgctgtccggccagggcgccgag
gtgcgccgactggtgctggacgagtcctgcgtcgaccgcacggtgctcgcggcccggctcgaaaacgcccacgacgtctcgggc
atcgtctcggtgctggcggcggcggaacagccgtgcgccgcgtaccccgcgctcgcctccggtctcgccctgacggtcgcgctcg
tccaggccctgggcgatgcgggcgtcgacgccccgctgtggtgcgtgacccgtggcgcggtctcgaccggccgttcggacctggt
gacgaacccggtgcaggcgcaggtgctgggcgtcggctggacggccgcgctggagcacccgcagcgctggggcggcatgatc
gacctgccccacacgctcgacgaacgcgccggccgacggctcgccgccgtcctcacgggtaccaccggcgaggaccagctcgc
catccggtcctccggtgtcttcacccgccgcgtggtccgggccgccgccggcagccagggcgcgggacgcgactggaagccgc
gcggcacgacgctgatcaccggcggctccggggcgctggccccggacctcgcgcgatggctcgcgggccagggcgccgagca
cctcgtgctggtcagccgccggggcccggacgcgcccggcgccgccgcgctggtggccgacctcgccgagctgggcacggag
gccgaagcggtcgcctgcgacatcagcgaccgcgacgccgtcgccggactgctgtcgaggctcaggaccgacgggcgcaccgt
gcggacgctcgtgcacgccgcggcggtcatcgagctggagtcgctggcggagaccaccatggcggagttcgccagggtggtgca
cgccaaggtcgccggtgcccggcacctggacgaactgctggacgacgaggagctcgacgcgttcgtcctcttctcctccatcaccg
gcatgtggggcagcggccggcacgccgcgtacggcgcgggcaacgcctatctgagcgcgctggccgaccaccgccgcgcccgt
ggcctgaccgccacgtccctgcactgggggaagtggcccgacgacgccgaggccgcgcagctcgacccgcaccagatccgccg
cagcggcctcgggttcatcgacgcgggcccggcgctggccggcatgcggcgggccctggacgacgacgagacggtgctcgcca
tcgccgacgtgaactgggacgtctaccacccgatcttcacctccggccggcccacgacgctgttcgacgaggtccccgaggtacgg
cggctgaccgagccggtggagcggaccgtgagcgcgggcgccgaaggggagttcgcggcccggctgcgtgacctgcccgccg
ccgagcgggaccgcctgctgctggacgtggtccgcggccaggccgcgaccgtcctcgggcacgcctccggtgaggcgttccccg
aacggcgtgccttccgtgacgtcggcttcgactccgtcacctcggtggacctgcgcaaccggctggtgaccgccaccgggctgcgg
ctgccgagcaccctggtcttcgactacccgacccccctcgccctggcggagttcctccgcgacgagatcgcgggcaccgccacca
ccgctcccgtggtgtccgccgccgtgaccgacgagccgatcgcgatcgtcggaatgagctgccgctaccccggcggcgcgcaca
cgcccgaggacctgtggcggctgctcgtcgacggtgcggacgtgatctccgagttccccgcggaccgcggctgggacgccgacg
ccctgtacgacccggaccccgaccggcacggcaggacgtactccgtccagggcgggttcctgcacgacgccgccgacttcgacg
ccgggttcttcggcatctcgccccgtgaggccgtggcgatggacccccagcagcggatgctgctggagaccgcgcacgaggcgct
cgaacgcgccgggatcgaccccgactcgctgcggggcagctcgaccggcaccttcatcggcgcgacctaccaggactacacctc

cggggtcccgaacgcggcagaatccgagacgtacatggccaccggcaccgcgtccagcgtgctgtccggccgggtctcgtacctc
ttcggcctggagggcccggcggtcacggtcgacacggcctgctcctcctcgctggtcgccctgcacctcgcctgccagtcgctgcg
caacggcgagagcacgctcgcgctcgcgggtggtgtcgccgtcatgtcgaccccgagccccttcgtcggcttcagccggcagcgg
gccctggccgtcgacggccgctgcaagccgttctccgacgacgcggacggcatgaccctcgccgagggcgtcggcctcgtcctg
ctggagcggctctcggacgcccgccgcaacggacaccaggtcctcgcggtcgtccgcggcagcgccatcaaccaggacggcgc
ctccaacggcctgaccgcccccaacggcccctcccagcagcgcgtcatccgccaggccctcgccaacgcgggtgtctcgcccgc
cgacgtcgacgtggtagaggcacacggcaccggcaccaagctgggtgacccgatcgaggcccaggccctgctggccacctacg
gtcaggaccgggagacaccgctgcggctgggctccctcaaggccaacatcggtcacacccaggcggccgcgggtgtcgcgggc
gtcatcaagatggtcatggccctgcgcgaaggagtcctgcccaagacggtcaacgtgagtgagccctcgtcgcgggtggactggtc
cgccggagcggtggagctgctgaccgaggccgagaagtggcagccggagggacgcacccgccgggcgggtgtgtcgtcgttcg
gcatcagcggcaccaacgcccacctgatcctcgaacaggcaccggacgccgaggccgacggaccgaacccgccgccgacca
cgcgggcgtcgtgccctggctcgtctccgcgaagtccgacgccgcgctgcgcgaccaggccgcccggctgctcgcccgcgtggg
cgacagcccggaactgccgcccgtacgggtcggctggtcgctggccaccacccggccccgcctggagcaccgcgcggtgctgct
cggtgagacgcacgaggacttcacccgctgcctggaggcgctcgcgacggccggcaccacgccgggagtcgtccgcggcgtcg
ccgcgggcggcgacgccgggcccgtgttcgtcttccccggccagggctcgcagtggtggggcatgggccgtgaactgctggaga
cctccgaggagttccggtccgccgtcgacgcgtgcgccgaggccctggccccgcacatcgactggtcgctgcgggacgtcctggc
gggcgccggtgaccccgcgatgctggagcgcgtggacgtggtccagcccgcgctgttctcgatgatggtggcgctgtccgcgctct
ggcgctcccacggtgtcgaacccgccgccgtgatggggcactcgcagggcgagatcgccgcggcgtgcgtcgcgggcgccctg
tccctgccggacgccgcggccgtcgtggcactgcggagcaaggccctgctggccctgtccggaaagggcggcatgatgtccgtg
gccgcctccgccgagcaggtgaccgccctcctggagccctggggcgaccggctctcgctggccgccgtgaacggcccgtcctcc
atcgtcgtctccggtgaccccgaagccctcgccgagctcaaggagcagtgcgcgaccggcaacatccgcgcccgcatggtcgcg
gtcgactacgcctcgcacggctcgcaagtggagctcatccaaggcgagttggcgcagctgctggcctcggtcacgcccctggtgcc
gaaggtgccgttctactcgaccgtgaccggtgagcggctcgacacgcccacgatggacggcgcctactggtacaccaacctgcgg
cagaccgtacagctggagcgggcgacgcgagcactgctcgccgacgggcaccggctcttcgtcgaggtgagcccgcaccccgtc
ctgaccggggccgtccaggagaccgcggaggccgcggacaccgaatccgcggtggcactcggatcgctgcgccgcgacgagg
gcgacaccaagcgcttcctgacctccctcgccgaggcgcacgtccacggcgcgtccgtcgactggcgcgcggcgttccccgcctc
ctcctcccaggcccccacccccgtcgaactgcccacctacgccttccagcgccgccggtactggccggaggcggagctgcccgcc
ccgggcggcaccggcgccggcacggaagcggacgccgccttctgggggctcgtcgagaacggcgacctggacaccctcgccg
gcgaactcggcgtcgtcgagaacgccggactctcctccctgggcgcgctgctgccggccctctcggcgtggcggaccaagtcccg
gcaggcctccgcggccaacaccctgcggtacaaggtggactggacccggctccgcgagcccggcggcacgaccccggccggc
acctggctggtggtcgcaccggcacggagcggcgacgacccgtgggtcgcggccgccgtcgacgccctgggccccgacgccgt
ccgcgtcgacgtcgaggacaccgaccgggacgggctgaccgcactcctggccgccaccgccgagagcaaccccggcctcacg
ggcgtggtgtcgctgctgggcctcgtcgaggagcggcaccccggcttcacctcggtgcccgacgggctggccatgacgctggccc

tggtgcaggcactgggcgacgcgggaatccaggcaccgctgtggtgcctcacccgggacgccgtcgcggtcggccgcgacgac
aaggtcacccaccccgtccagggcgcgctgtggggcctcggcctcgtcaccgccctggagcagcccggccgctggggcggtctg
atcgacctccccgacaccatcgacccgcgaaccgccgcccgcctgcgcgccgtcctggccgcaccggacggcgaggaccaggt
ggccgtccgcgcctcgggcaccttcggccgcaggcttctccacgccgcccccggcggcaaccgccccgagcggcgctggtggg
agcggggaaccgtgctcatcaccggcggcacgggcggcctcggcggccgcgtcgcccggtgggtcatcgagcgcggcgcctg
gcacgtcgtcctcaccagccgtcgcggaccggacgcccccggcgccgccgagctccgcgccgaactcgagggcctgggcgccc
gggtgaccatcgcggcctgcgacgccgcggaccgcgacgcgctgcacggactgctggcgggcctgcccgccgaactccccctg
acggccgtggtccacgccgccggtgtcgccgacggcgacgccgacgcacagtccctcaccctggaacagctcgacgcgctcctg
cgctcgaagctgaccgccgccctgcacctgcacgaagccacccaggacctcgacctcgacgcgttcgtcctgttctcctcgggcgc
gggcatctggggcagcggcggacaggcgggctacgccgccgcgaacgccttcctcgacaccctcgccgagcaccgccgggcc
cggggcctgaccgccacggccgtctcctggggagcgtgggccgaggccggcatggccacggtcgccgcggtgcacgaccggct
gagccggcgcggcgtcctgtcgatggagccgtccctggccatcgccgccctccagcagagcctcaccgacgacgacacccaggt
cacggtgacggacatggactgggagctgttcaccccgagcttcaccgccacccgccccagcccccctgctgtccggcatccccgag
gtccgcgcggtgctgtccgcccaggaagccccggccgacgacgacggcgacgagtcggcgctgcgccgacggctcgaagcgct
gtccgacgccgaacgcgggcgcgccctgctcgacgtcgtccgcgtcgaggcgtccgccaccctcggccacgaagcggccgacg
ccatccccgccgaccgggccttccgcgacgtcggattcgactccgtgaccgccatcgagctgcgcaaccggctccgggtggcgac
cgggctggccctgcccgccgccctggtgttcgactacccgacgccgaccgcgctcgcccagtgcctgcgctcgtacctcttcccgg
acaccgccccggcggagaaggcggacgacccggaagcgcacatccgcgaggccctcaccaccgtccccatcagccggctccg
caaggcgggcctgctggacatggtgttgcagctcgccaacgaagaagccgagaccgccaccgccgcgcccgacgccgcgtcgt
cgatcgactcgatcgacgacatggacgccgagagcctgctgcgactggcgaccgagagcacgacgaactgaaagcgacctgga
gaagaacgatgaacacctccaccaaccaatacgtcgaagcgctgcggtcgtccctgaaggaaatcgagcggctgcggcaacaga
accaacagctggtcgcggcggcggtggaaccgatcgcggtcgtgggcatcggctgccgattcccgggaggtgtggcctcgccgg
aggacctgtggcgactcgtcgccgagggccgcgacgcgatcgcggacttccccaccgaccggggctgggacctgggcgcccctc
gcgggtgacggccccggcggcagcctcgcccagcagggcgggttcgtctacgacgcggccgagttcgaccccggcttcttcggc
atctccccccgcgaggcggtcgcgatggacccccagcagcgcatgctgctggaggtgtcctgggaggcgctggagcgggccgg
catcgacccgtcgtccctgcgggagagcccgacgggtgtgttcgtcggcaccaccggccaggactacagcaccgtcatcaccggc
tccgacgaggacgccgaggtctactccaccaccgggcacgcggcgagcgtcatctccgggcggctgtcctacacgctcggctgcg
aaggccccgccgtcaccgtcgacacgggctgctcctcgtcgctggtcgccctgcacctggccgcgcaggcgctgcgcggcggcg
agtgctccctcgccctggccggcggtgtgacggtgatgtcgacgcccggcgcgttcgtctccttcacctcccagggcggcctggcc
gccgacggccggtgcaagtccttctccgacgacgccgacggcacgggctgggccgagggctccggcgtcctgatcctggagcgc
ctctccgacgcccagcgcaacggccacccggtgctcgcggtgctgcgcggcagcgccgtcaaccaggacggtgcctccaacggc
ctgaccgcccccaacggcccctcccagcagcgcgtgatccgcgccgccctggagaacgcgcacctcgcgcccgccgacgtgga
cgcggtggaggcccacggcaccggtacgaccctcggtgacccgatcgaggcacaggccctgctggccacctacggccaggacc

gggaacgcccgctgctgctcggctcgctgaagtcgaacatcggtcacacccaggccgcttcgggcgtcgccggtgtgatcaagatg
gtcatggcgatacgcaacggcgtcctgccccggaccctgcacgtcacggagcggtcgtcgcacgtggagtggtcggcgggctcg
gtggacctgctgacggaatccgtacagtggcccgaggcgggccgggtgcgccgggcgggcatctcgtccttcggcatcagcggc
acgaacgcgcacgtcatcgtcgagcaggcgccggccgtcgagtcaccggccgccgaggagccgatggtatcgcccggcgtcgt
gccgtgggtggtgtccggcaggtccccggaggcgctggacgcgcaactggaccgcatcacgacgttcgcgtcggaccaccccga
cgccacaccgctcgacgtgggcctctcgctcgccgccggccggtcggtgttcgagcacagcgcggtgctgctggcctccggtgaa
ggcgtcctcgaggccgcccgcggggtggccgcggcggacgagagcctgctcgcggtgctgttctccggccagggctcgcagcg
gctcggcatgggccgcgaactgtacggccgcttccccgtgttcgccgaggccctcgacgcggttctggcggagctggacccgcta
ctggaccgcccgctgcgcgaggtgatgtggggcgacgacgccgaggcgttgaacgagacgggccacacccagcccgcgctgtt
cgccgtcgaggtcgcgctgttccggctggtggagtccctcgggatcaccccggacttcgtggccggtcactccatcggcgagatca
cggccgcgcacgtcgcgggggtgttcccgctggcggacgcctgccggctcgtcgcggcgcgaggccgtctgatgcaggccctgc
cgtccggtggtgcgatggtcgccgtccaggcgacggaggccgaggtgctgccgctgctgaccggcaccgtgtcgatcgcggcgg
tcaacggccccacgtccgtggtgatcgcgggggaggaggccgcggttctcgacctcgcgggccgtctcgccgcacagggccgca
agacctcgcgcctgtcggtctcgcacgccttccactcgccgctcatggagccgatgctggacgacttccgtcaggtcgtcgccggcc
tgacgttcgaggcgccgtcgataccggtcgtgtccaacctgacgggccgggtggccacgtcggaggagatccgcaccccggagta
ctgggtgcggcacgtccgggaggccgtgcgcttcgccgacggtgtccgggccctggtggccgagggcgtgggcaccctgctgga
gctgggcccggacggtgtctgctcggcgatggccgccgagtcggtacccgagggcaccgtggtggtcccggtcctgcgcaagga
ccggcccgaggagaccgcggccgtgacgacgctggcccggctgcacgtgggcggcgtccgcgtggactggtcggcgttcttcgc
cggcaccggcgcccgccgggtggacctgcccacctacccgttccagaacgagcgatactggcccgcgggggtcgcgaacaaca
ccggtgacatgcgtgccgccggactggactcggccgggcacccccctgctcggcgccgcggtggaactggccaacggcgaaggc
gcgctcttcaccagccgcctgtcggtccagtcgcacccgtggctggccgaccacgtcgtcggcggccgcgtgctgctgcccggca
cggccttcgtggagctggcgatccgcgccggtgacgaggtcggctgcgaccgggtcgaggagctggcgctggccgctccgctgg
tgctgcccgaacagggcgcgatccagttccaggtgcgggtcggcgcggccgacgacgccgggcgccggtcggtgggcatgtact
cgcgggccgagggcgccgtcgacgccccgtggtcggggcacgcgaccggtgtgctcgcccccggcacgggcacggccgggg
ccggcttcgacgccacggtgtggccgccggccggcgccgaaccggtggacctggagggctgctacgaacgcctctccgagatcg
gcttcgactacggaccgctgttccggggcctgcgggccgcatggcggcgcggcgacgaggtgttcgccgaagtgagcctgcccg
agggcgtggaggccgacgcggtcgcgttcggactgcaccccgcgctgctcgacgccgcccagcacgcggccgccttcggcgac
ctcggagcgatcagccacggcggactcccgttctcctgggagggcgtgtcgctgcacgcctccggcgcctccgccgtacgggcca
ggctgtcccgtaccggcgaggacatggtgtcgatcgccgtcgccgacaccaccggcggcctcgtcgcctccgtcgattcactggtct
cccgccgcgtccccaccgagcagctgaccgaggccgactccgtgcgcccgggactcgctgttccgcgtggaatggtccgccgtccg
ggccgagcccgtggcggccgccggaccggtggcnnnnnnnnnnnnnnnnnnnnccgccctggccgaggcgctgcgcgcc
accggcacccccgccgacgggtacgcggacctggacgccctgttcgccgcgggcgccgtgcccgatgcggtgctcgtccccgtg
accggcggcgagccgggcgcggagcaggccgcggcgacgcacgccgtgaccggctccgtcctggccctggcgcagcgctgg

ctgaccgacgagcggtccgccgacgcccgcctggtcttcgtgacgcgaggcgcggtggccgtcgccggagacgcccccactgac
ctcgccgcggccgccgcgtggggcctggtgcgctccgcccagaccgagaatccgggccggttcggcctgctggacctcgacacc
gccgccgcctcgctcgcgtccgtaccggccgcgctggctgtcgacgagccgcagctggcggtccgcgacggacagccgctggc
cgcccgactggcacgcctcgcggtgccggacaccgcccccgaggccgtctgggacgacaacgggaccgtcatggtcaccggcg
gcaccggcggcctgggcgccgtgatggcccgtcacctggtcgccgaccgcggtgtacgacggctgctgctcgtcagccgccggg
gcctcgccgccgccggcgccgaggacctggtcgccgagctcacggcacaggacgccgacgtgcgcgtcgccgcctgcgacgtc
tccgaccgcaccgcgctgaccgggctgctggagtcgatcgacgacgcccacccgctgcgggccgtcgtgcacaccgcgggtgtc
gtcgacgacggcgtcatcggctcgctgacccccgagcgcctgagcgccgtactgcgccccaaggtcgacgcggtgtggaacctg
cacgagctgacccgcgacctggacctgaccgcgttcgccgtgttctcgtcggtgtccgccaccctgggcagcgcgggacaggcca
actacgcggcgggcaacgccttcctggacgccctcgccgcccaccgccgcgccgcgggcctgccgggcctgtccctggcctggg
gcccctggacccagtcggtgggcatgacgggctcgctgaccgacgtggacgtggagcgcatcgcccgactgggcatgccgccgc
tctccgtcgaacagggcgtgaccctgttcgacgcggccctggccggtacggaaccggtggccctgccggtacggctcgacctgcc
ggcgctgcgcgccaagggcgagatcccccgctgctgcgcggactcatccgggtccccacccgccgcgccgccgcacagtcctc
gaacgccgtcgactccctcgtgcagcgactcgtcccccctcaccgacgaggagcgccgcgaggtcctgctggacctggtgcggagc
caggtggccctggtgctcgggcacgccggcgcgggctccgtcgacgtggcccgccagttccaggacctgggcttcgactccctga
ccgcggtcgaactgcgcaaccgcctcaacaccgcgtccggcctgcggctgcccgcgacgctcgtcttcgactaccccacgccggg
tgtcctggccgactacctgctggacgagctgttcggcgccgaggcggagacggccgcccccgtgcccgtacgggccacggcgcc
ggtcgccgacgaccccatcgtgatcgtcggcatggcgtgccgctacccgggcgacgtgggctcgccggaagacctgtggcggat
ggtcaccgagggcgccgacgcggtctccggcttcccgaccgaccgcggctgggacatcgacgggctgtacgaccccgacccgg
accacccgggcaggacccacgtccggcacggcggcttcctgcacgacgcgggcgagttcgacgccgagttcttcgggatgagcc
cgcgcgaggcactcgccaccgactcccagcagcgcctgctgctggagacgtcgtgggaggccatcgagcggtccggcatcgacc
cggtgagcctgcgcggcagccagaccggtgtcttcgccggcgtgatgtacaacgactacggcaacgccctgacgggcgaggagtt
cgagggcttccgcggcaacggcagcgcaccgagtgtggcctccggccgggtgtcctacaccttcgggctggagggcccggcggt
cacggtcgacacggcgtgctcgtcgtcgctggtcgcgatgcactgggcggcccaggccctgcgcgcgggcgaatgctcgctggc
gctggccggtggtgtgacggtcatgtccacccccaacaccttcgtggagttctcgcggcagcgcggcctggccgccgacggccgg
tgcaaggcgttctccgactcggccgacggtgtcggctggtccgagggtgtcggcatggtggtgctggagcgtctgtcggacgcccg
gcgcaacggccaccaggtcctcgcggtcgtacgcggcagcgcggtcaaccaggacggcgcttccaatggtctgacggctccgaa
cggcccgtcccagcagcgggtcatccgccaggccctggcgagcggtcacctgacgtccaccgacgtggacgtcgtcgaggcgca
cggcacgggtacgacgctgggcgacccgatcgaggcgcaggcactcctggcgacgtacggtcgtgaccgggacgcggagcgtc
cgctgctcctcggctcggtgaagtccaacatcggtcacacacaggccgctgccggtgtcgccggtgtcatcaagatggtcatggcga
tgcggcagggcatcctgccgcgcacgctgcacgtgacggaaccctcgacgcacgtggagtgggaggcgggctcggtcgagctg
ctgaccgagcagacggcctggcccgagggccgggtgcgccgggcgggtgtctcgtcgttcggtatcagcgggacgaacgccca
cgtcatcgtcgagcaggcaccgttcaccgactcgccggtcccggagccgacggtgtcgccggacgtggtgccgtgggtggtgtcg

ggccggaccggcgcggcgctggacgcccagctggaccgggtcaagacgttcgcggccgagcgcgccgacatcgcacccgtgg
acctgggcctttccctggcgacgtcacggtcgctgttcgggcaccgtgcggtgctgttgtcttcggccgagggtgtggtcgaggcgg
cgcgtggtgtggcggtcgatcgtccgttggcggtgttgttctcggggcagggtgcgcagcggctggggatgggccgggagctgta
cgagcgcttcccggtgttcgcggcggcgttcgacgaggttcttgccgagctggatccgttgctggaccggccgctgcgtgaggtgat
gtggggtgaggacgccggtctgctgaacgagaccggttccacgcagcccgcgttgttcgcggtcgaggtggcgctgttccgtttggt
gcggtcgtggggtgtgaagccggacttcctggcgggtcactccatcggtgaggtggcggctgcccatgtggcgggtgtgctgtctct
gggcgatgcggcgaagctggtggcggcgcggggtcgtttgatgcaggcgctgccgtcgggtggtgcgatggtggccgtccaggc
gaccgaggccgaggtgctgccgttcctgaccgaggtggtgtcgatcggggcggtcaacggtccgtcgtcggtggtcgtctcgggtg
ccgaggatgcggtgctggagattgcgggccgtctgtcggctgacgggcgtaagacgtcgcggttgtcggtgtcgcatgcgttccact
cgccgctgatggagccgatgctggatgacttccgtgcggtggtcggcggcttgacgttccacgcaccgtcggtgccggtggtttcca
acctgaccggtcgggtggctgctgcggaggagttgtgctcgccggagtactgggtgcggcacgtccgtgaagcggtccgtttcgcc
gacggggtacggaccctggcggccgagggcgttggcacgctgttggagctggggccggacggtgtgctgtccgccatggctgcg
gagtcggtgtccgaagagaccgtgatggttccggttctgcggaaggaccggccggaggagaccgccgcggtcagtgcactggcg
cggctgcacgtacagggcgtcccggtcgactgggaggcgttcttcgccgggaccgccgcccgccgggtcgacctccctacctatg
ccttccagcacgagcggttctggcctgccgggatgctgagcaaccccggcgacatgcgggccgtgggacaggcgtctcccggac
acccgctgctcggtgccgcggtggaactggccgacggcgaaggcgcgttgttcacgaaccgcctgtcggtgcggtcgcacccgtg
gctggccgaccacgccgtgatgggacgggtgctcctcccgggcacggccttcgtggagctggcgatccgcgccggtgacgaggt
cggctgcgagcggatcgaggacctggcgctcgccgcgccgctcgtcctgcccgagcggggcgcggtccagctccaggtgcggg
tcggcgcggccgacgacgccgggcgccggtccgtgggcgtgtactcgcggatcgagggcgcggtggacgtgccgtggacccag
cacgcgatgggtgtgctggttccggggtccggtgccgccggtgccgcgttcgacgcgacggtgtggccgccgaccggtgccgaa
ccggtggacgtgaccggcttctacgaggcccgcgccgaggacgggttcgtgtacggacccgagttccggggactgcgggcggcg
tggctgcgcgacggtgaggtgttcgccgaggtgagtctgcccgacggggcacaggccgccgcggacgcgttcggtctgcacccg
gcgctgctcgacgcggtgctgcacgcgggcggactgatcggctcggagcggcaggcggtgcccttctcgtgggagggcgtgtca
ctgcacgcctccggcgcctccgccgtgcgcgtcaggctgtcgcgcgcctcggacggtgctctggcgatcgacatcgccgacacct
cgggcgctccggtcgccacggtggactcgctgctggcccgggagatctcggccgaccagctcaccgaagccgacacacgggac
cgggactccctgttccgcctggagtggtccgcggcggtaacggccgaccacgcggtcgtgggaccggtggccgtcgccggagcc
gatgtcctcggcctggccgaggccctgcgtgccgcggacgtcgacgtcgcggtgcacacggacctgggggcgctcgccacctcg
gacggcccggtgccgggcgtcgtcctggtgcccgtggccggcgatgccgacgccacggtgatcgacgccacgcacggggcaac
gacccttgccttgggacgactgcagcagtggttggccgacgagcggttcggcgggtcgcgtctggtgttcgtgacgcggggtgccg
tggccgtggacgccggtggcgtcgccgacctggccgccgcgtccgtgtggggcatggtgcgctcggcgcagacggagaacccc
ggctgcttcggtctggtcgacctcgacgcgtccgcgtcggccgaactgccgcgcgccctcggtacggacgagccgcaggtggttgt
ccgcgacggcgaggtcctggcgggccggctggcccgtaccgcgacgagcggcggcctggtcccgccgatggacggccgtgg
cgcctgggcagccgggcgaagggcagcctggacgccctcgagctggccccgtgcccggaggtgctggagccgctgaccggcc

gcgacgtgcggatccgggtccgggcggccgggctgaacttccgtgacgtactgaacgcgctggacatgtaccccggcgacgccg
gcgtcttcggctccgagtcctccggcgtcgtcctcgaggtcggacccgaggtgacggggctgcgccccggcgaccgcgtcacgg
gcgtggtggtcggcggcttcggctcgctggtggtggcggacgaacggcgcctggcgcgcatgcccgacgcctggtcgtgggaga
cggccgcgtcggtgccgctcgtgttcctcaccgcgtactacgggctggtcgagctggccggcctgcggcggggcgagaaggtcct
gatccacgccggcgcgggaggcgcgggcatggcggcgatccagatcgcccgccacctgggtgccgaggtgttcgccaccgcga
gcgagggcaagtgggacgtcctgcgttcgctgggcgtcgccgacgaccacatcgcgtcgtcgcgcaccaccgacttcgaggcgg
cgttcgccgccgtcgcgggcgacggtgggatcgacgtcgtactgaactccctggcgggggagttcgtggacgcgtcactgcggct
gctgacctccggcggccgtttcatcgagatgggcaagaccgacatccgggacgcggcaggcctgcagggcatccgctacgtgtcg
ttcgacctgggatgggtggaccctgcccacatccagccgatgtgggacgtcctcatggggctgttcggcgaaggcgtgctgcggcc
cctgccggtcaggacgtgggacatccggcgggcacgcgacgcgttccgctacatgagcctcgccaagcacgtcggcaagatcgt
gctgaccctgccccgcacctgggcgccggacggcacggtcctgatcaccggtggcaccggcggtctcgccgccgaactggcccg
ccacctggtcacggaacgttccgtgcgccacctgctgctggtcagccgccggggcccggacgcgccgggagcggcggagctcc
gcgaggagctgacggccctcggggccgaggtgaccgtcgtcgcgagcgacatagccgatcgcgccgccgtggccggcctgctc
gccgggatcccggtggagcacccgctgacggccgtcgtccacacggcgggcgtcctcgacgacggtgtcatcgcctcactcaccc
ccgagcgcgtggccgccgtgctgcgccccaaggccgacgcggtgtggaacctgcacgagctgacccgtgacctggacctggcc
gccttcgtggtgttctcgtcggtgtccggcacgttcggcggcgcgggacagggcaactacgcggcgggcaacgccttcctggacg
cgctcgcccaccaccgtgccgcccagggcctgcccacggtctccctggcctggggtgcctgggcccagggcgcgggcatgacca
gcggtctcgacgaccgcgacatccgccgtgccgccgaagcgggcatgccgctgctgtcggtggaagaggggctggcactcttcg
acgccgccctggccaccggcgaacccgccgtgctcccggtacgactggacctggcggtgctgcgcacgcggggcgaggccccg
gcgctgctgcgcggcctgatccgtgaccgctcccggcgcaccgcggtggccgggccggccgccacggccgacctgccgcagcg
gctgagcaggctggagcgggccgaacggctcgaagccctgctggatctcgtgcgtgcccaggcggccctggtgctcgggcacgc
gagcggtgacgcagtcgacccggagcgggcgttccgcgacctcggcttcgactccctcacggccgtcgagctgcgcaacaagct
gaacaccgcgaccgggctgcgactgcccgccacgatggtcttcgactaccccaccgtcggggtgctggcggaccacctgctggag
gagctgacgggcccggacaccgagccggccgtcgccgaaccgacgcgaacggccactcaggccgacgacccggtcgtggtcg
tcggaatgagctgccgctaccccggaggggtgagctcccccgaagacctgtggcggctcgtcaccgagggcggccacgcgatgt
cgggcttcccgaccaaccgcggctgggacctcgactccctctacgaccccgacccggagcggacggggaccgtgtacacccggt
ccggcgggttcctgcacgacgccggcgagttcgaccccggcttcttcgggatgagcccgcgcgaggcgatggcgacggacatcc
agcagcggctgctgctggaactctcctgggaggccgtcgagcgggccggcatcgacccggcgacgctgcgcggcagccagacc
ggtgtcttcgccggtgccatgcacaacggctacggcgcgacgctggcgggcgaggagttcgagggcttccgcggcaacggcagc
gcactgagcgtggcctccggccgcatctcgtacaccttcggtttcgagggcccggcggtcacggtggacacggcgtgctcgtcgtc
gctggtcgccatgcacctggcggcgcaggcgctgcgcgccggcgagtgctcgctcgcggtggccggtggtgtgacggtgatgtc
gactcccgagacgttcgtggagttcgcccgccagcgcggcctcgcacccgacggcctgtccaaggcgttctcggactcggccgac
ggtgtcggctggtccgagggcgcgggcatggtcgtgctggagcggctgtccgacgcccggcgcaacggccaccccgtgctcgcc

gtgctccgtggctcggccgtcaaccaggacggcgcgtccaacggtctgaccgccccgaacggcccctcgcagcagcgggtcatc
cgccgggccctggccagcggtggtctgtccccggccgacgtcgacgccgtcgaggcccacggcaccggcaccaagctgggcga
cccgatcgaggcccaggcactccttgcgacctatggtcgtgaccgggacgcggagcgcccgctgtggctgggttcggtgaagtcg
aacctcggccactcccaggcggcggccggtgtggccggtgtcatcaagatggtgatggcgatgcggcacggtgtgctgccgcgca
cgctgcacgtgaacgagccctcgacgcacgtcgactggtcggcgggcgccgtggaactgctcaccgagccggtcgagtggcccg
actcgggccgtgcgcgacgcgcgggcgtgtcgtcgttcggcatcagcggcaccaacgcgcacctgatcctcgagcagcccccga
ccgtcgacgaagccgtcgccgacggcgccgttcccgactccgccgcgacggagtccggcctcgtgccgtgggtgctgtccggca
agtcgccggaggccctgcgcggccaggcggcgcgcctgctgtcgcacgtccaggcgctctccgcgccgcgcccggccgatgtg
gccttctccctcgtcaccgagcgcacagccttcgaacaccgggccgtcgtcctggccggtgacctgacggacgcggtgcggggac
tggcggcgctggccgtcggcgagcccggcgccgcgacgatcagcggctcggtctcgggtggcaaactcgccttcctgttctccgg
gcagggcagccagcggatcggcatgggccgggagctctacgagcgcttccccgtcttcgccgaggccctggacgcggtgctggc
cgagctggagccgctgctggacggttcgctgcgcgaggtgatgtggggtgacgacaccggtccgctgaacgagaccggtcacac
ccagcccgccctgttcgcggtcgaggtggcgctgttccggctggtgagctcgtgggggtgcggccggacttcctggccgggcac
tccatcggcgagctggccgcggcccacgcggcgggcgtgctgtccctgccggacgcgtgccgtctggtcgccgcgcgaggccg
gctgatgcaggcgctgcccacgggcggtgcgatggtggccgtcaaggccaccgaggccgaggtgctgccgctgctgaccgacac
ggtgtcgctcgcggcggtcaacggcccctcgtccgtggtgatcgcgggtgacgagaccgaggtgctggacatcgcgggccgactc
gcggcggaaggccgcaagacgtcgcgcctgtcggtgtcgcacgccttccactcgccgctcatggagccgatgctggacgacttcc
gggcagtggtcggcgacctgacgttcgccgaaccctccgtcccggtcgtctccaacctcacgggccggcaggccacttcgcagga
gctgcgctccccggagtactgggtgcggcacgtccgcgaaacggtccgcttcgccgacggtgtgcggaccctcgccgaccacgg
cgtgaccaccttcctcgagctgggccccgacggcgtgctctccgccatggcacaggagtccctgtccgacgggaccgcgaccgtg
gtgcccgtactgcgcgggaaccggcccgaggaaacggccgcgctcaccgcactggcccacgcgtacaccgccggtgtcccggt
ggactgggccgggttcttcaccggcacgggtgcccgccgggtggagctgccgacgtacgcgttccagcgccagtggttctggccg
accggctcggccggtgcggcgaacgcggccgccgtcggcctcacctcggtcggccacccgctgctcaacggcgcggtggagct
ggcggacggcgaaggcgtgctgttcaccggccgcctgtcgctccagtcccacccgtggctggccgatcacgcggtcctgggccg
ggtcctgctgccgggaacggccctgctggagctggcgatccgcgccggtgacgaggtggactgcgaccgggtggaggaactgac
cctggccgcaccgctcgtgctgccggagcacggcgccgtacaggtacagctgcgcgtcggcgtcgccgacgacaccggccgcc
gtaccgtcacggtccactcccggcccgagggcgccgacgagcagtcctggtcgcagcacgcctcgggtgtgctcgccggtggtgc
gcagccggccggcacggacttcgacgccgtggtgtggccgccgacgggagccgagccgctcgacgtgaccggctgctacgagc
ggttcgccgaggtcggcttcgcgtacggcccggtgttccggggcctgcgggccgcatggcggcgcggcgacgacgtgttcgccg
aggtggcactgtccgacgacgtccaggcggacgccgcggcgtacggtctgcaccccgctctgctggacgcggccctgcacgcct
ccctgctcgccggcgacggcggcgagggcggactgccgttctcgtgggaaggcgtgacgctgcacgccaccggtgcgtcggcg
ctgcgggtacggctctcccggtccggcgcggacgcggtgtcgatcgcggtggccgacacctccggagagccggtcgcctcggtg
gactcgctgctcgtacgggccgtttcgggtgagcaactgagcgatgccgcggcgctgagccgcaacgcgctgttccggccggact

ggactcccgtgcgggccgaggcggtggccgaccggccgatggccgtggtcggaccggacgctctcggcctggccgagggactg
acggcgaccggggcgagcgtcgcgacgcacgtcgatctggcgtcgctttcggcatccgacgccccggtacccggtgtggtgctcg
tgggcgtcgccgggcgcgcggacaccggtccggtggccgccgcgcacgagatgaccgcggaggtactgggccggctgcagga
gtggctggccgaggagaggttcgtcggctcgcgcctggtgttcgtgacccgcggcgcggtgggcggcacggacccggccgccg
cggcggtgtggggcctggtgcgttccgcgcagacggagaaccccggctgcttcggcctggtggacctcgacgacgcgtcgtccgt
ggagttcctgcccacggcgctcggcgcggacgagccgcaggtggccgtccgcgacggtgaggtcatggcggcccgactggcgc
gcacgaccgcgggccggccggacgcgggccccgtgtgggagcccgacggcacggtactgatcaccggtggcacgggcggcct
gggcgccgtcctcgcccgccacctggtcgccgagcgcggcgtgcggcgactgctgctcgccagccggcgcggcggcgagagc
gccggcacggtcgaactggtcgccgaactggcggcccacggcgccgacgcccaggtggtggcgtgcgacgtcgccgaccgcg
acgcgctggccgcgctcctcgccgcacacccggtgtcggcggtcgtgcacaccgcgggcgtcctggacgacggtgtcatcggctc
gctgacaccggaacgcatggcggcggtgctgcgcccgaaggtcgacgcggcgtggaacctgcacgagctgacccgtggcctgg
acctgtcggcgttcgtggtgttctcctccctgtccggcctcctcggtggcgccggccagggcaactacgcggccggaaacgccttcc
tggacgcactgatggaacaccggcgcgccgaggggctgcccggggtgtcgctggcatggggtccctgggaccggacgggcggc
atgaccggcaccctggccgacgcggacgcggagcgcctcgcgcgctccggcgtgccgccgctgtcggcggagcagggcgtga
cgctgttcgacgcggccctggccaccgacgggcccgtggtggttccggtacggctggacctgcccgcgctgcgggcccagggcg
aggtgcccgcgctgctgcgcggcctggtccgcacccgttcccgccgggccgtcgtggccgggtcgggtacggccgcaggcctcg
tacagcggctgggccgtctcgacggcaccgagcggcacgaggtgctgctcgacctggtacgcggccaggtcgccctggtgctcg
gccacgcgaagggcgacgaggtcgaccccgggcgggcgttccgcgacctcggcttcgactcgctgacggcggtcgagctgcgc
aaccggctgaacaccgcgaccgggctgcgactgcccgccacgctggtcttcgactacccgtccgtacggagcctggcttcgtacat
cctcgacgagctgatgggcggaccggacgccgagccggtcgtgccgcagctctcgcggaccgcggtggccgacgacccgatcg
tcgtggtcgggatggcctgccggtacccgggcggggtgagctcgcccgaggacctctggcgactcgtcaccgagggcaccgacg
cgatctccgacttcccgaccaaccgtggctgggacgtcgacggcctctacgacgcggacccggaacacgccggtacctcgtacac
ccgctccggtggtttcctgcacgacgcgggcgagttcgaccccgagttcttcgggatgagcccgcgtgaggcgcttgccaccgact
ctcagcagcgcctgctgttggaggcgtcgtgggaggcgttcgagcgggccggtatcgatccggcgtcgctgcgcggcagtcagac
cggtgtcttcgccggtgtgatgtacaacgactacggaaaggccctggcgggggaggagttcgagggcttccgcggtaacggcagc
gccgcgagcgtcgcctcgggccgggtgtcctacaccctgggtctggagggcccggcggtcacggtcgacacggcgtgctcgtcgt
cgctggtcgcgatgcactgggccgctcaggcgctgcgcggcggcgagtgctcgctggcgctggccggtggtgtgacggtcatgtc
gacgcccaccacgttcgtggagttctcgcggcagcgcggcctggccgccgacggtcggtgcaaggcgttctcggactcggccgac
ggtgtcggctggtccgaaggtgtcggcatgctggtgctggagcgtctctccgacgcccggcgcaacggccaccagatcctcgccgt
gctgcgcggtagcgcggtcaaccaggacggtgcgtcgaacggtctgaccgctccgaacggtccctcgcagcagcgggtgatccgt
caggcgctcgccagcggtggactctcgcccgccgatgtggacgcggtggaggcgcacggtacgggaacgacgctgggtgaccc
gatcgaggcgcaggcgttgctggcgacctatggtcgggaccgggatgaggagcgtccgctgctgctcggttcggtgaagtccaac
atcggtcacacgcaggccgcggccggcgtggccggtgtcatcaagatggtcatggcgatgcggcagggtgttctgccgcagaccc

tgtacgccgacaccccgtcctcgcacgtggactgggaagcgggcgcggtcgagctgctgacagagaacaccgcctggcccgagg
cgaatcgagtgcgccgggcgggtgtgtcgtcgttcggtatcagtggcaccaacgcgcacgtgatcgtcgagcaggctccctcggtg
aaggagccgagccgggagccggtggtctcgccgggtgtggtgccgtgggtggtgtccgcaaggaccgaggcggcgctcgacgc
ccaggtggaccggctgcggtccttcgtggcggacgacacggcagcgacctcggtcgacgtcgggctgtcgctggccacgggccg
gtcgctgttcgaccaccgagcggtgctgctggcctcggacgagggcggcctggccgaagcggcgcgcggcacggccaccggcg
aggggccgctcgcggtgttgttctcgggccagggtgcgcagcggctgggcatgggccgggagctgtacggccgtttccccgtgttc
gccgaggccctggacgcggtgctggcggaactggagccgttggtcgagggctcgctgcgtgaggtgatgtggggtgaggacgcc
ggtctgctgaacgagaccggttacacgcagcccgcgttgttcgcggtcgaggtggcgctgttccggctggtggagtctctcgggatc
accccggagtacgtggccggtcattcgatcggtgaggtggcggccgcgcatgtggcgggtgtgttctcactgccggacgcctgccg
cctggtggcggcgcggggtcgtttgatgcaggcgctgccgtcgggtggtgcgatggtggccgtccaggcgaccgaggaagaggt
gttgccgttcctgaccgaggcggtgtcgatcgcggcggtcaacggtccgtcgtcggtggtcgtttccggtgccgaggatgcggtgct
ggagatcgcgggccgtctgtcggctgatgggcgtaagacgtcgcggctgtcggtgtcgcacgcgttccactcgccgctgatggagc
cgatgctggatgacttccgtgcggtggtcggcggtctgacgttccacgcaccgtcggtgccggtggtttccaacctgaccggtcggg
tggctgctgcggaggagctgtgctcgccggagtactgggtgcggcacgtccgtgaggccgtgcgtttcgccgacggggtacggac
cctggtccaggaaggcgtgaacacactcctggagttgggtccggacggtgtgctgtccgccatggccgcggaatcggtgtccgaag
agaccgtgacggttccggttctgcgcaaggaccggccggaggagaccgccgcggtcaccgcactggcaaggctgcatgtgcggg
gcgtcgcggtcgactggtcggcgttcttcgccgggaccggtgcccgccgcgtcgacctgcccacgtacgcgttccagcgtcagtgg
ttctggccggccggtacggcgatgtcggtcgtggacgcgaccgcggtgggactcacgcccgccgggcacccgctgctcaacggc
tcggtggaactggcggtcggcgaaggggtgctgttcaccagccggctgtcggtgcggtcgcacccgtggctggccgaccacgcc
gtgatgggccgcgtgctgctgcccggcacggccttcgtggagctggcgatccgcgccggtgacgaggtcggctgcgaccggatc
gaggagctgacgctggccgcgccgctggtcctgcccgagcagggtgcggtgcaggtgcaggtctcggtgggcgcacccgacga
gtcgggcaggcgcaccgtggaggtccactcgcgtcccgagggcgcggtggacgtgccctgggcacagcacgccacaggtgtgc
tgacggaggggacggccgtcaccgacgccggtttcgacgccgaggtgtggccgccgaccggtgccgagccggtggaactggcg
ggctgctacgagcagttcgccgaggagggcttccagtacgggccggccttccagggcctggaagcggcatggcggcgggcga
ggacatcttcgccgaggtgcggctgcccgagagcgtcgagggcgacgccgacgggttcggcctgcaccccgccctgctcgacgc
ggccctgcacgccgcgctgccggccgcggccggcacggaggacggcggactgccgttctcgtgggagggcgtctcgctgcacg
cctccggtgcctcggcgctgcgggtccggctgtcccccgccggtgcgaacgctctcgcgatcagcgttgccgacgcttcgggtgcg
cccgtcgcgtcggtggagtccctgctggtccgcgcggtgtcgggcgagcagctgagtggcccggaggcaccggtccgggactcg
atgttccgggtggactggtcgccggtgcgggtggccgggtcggccggtgccgagtcggtggcgctggtgggtccggacgtgttcg
ggctggcccgggttctgccggacgcgcttcaccaccaggagctggccgccttggtggcttcggacgcggcggtcccggacgcggt
gctgattcccgtacccggggagccggacggtgagccggtcgagtccgtgcacacctcggtgtcctgggccctgggccagttgcag
gcgtggctggaggaggagcggttcgccgcatcgcgtctggtgttcgtgacgcgtggcgcggtggacggtgttgatctggccgctgc
ctcggtgtggggtctggtgcgttcggcgcagacggagaccccgggccggttcggcctggtggacctggacgcggccgacacgtc

cttgaccgcactgccgcaagccctgggagtggatgagccgcagctggccatccgcgagggcgagatcacggccgcgcgtctggc
ccgggtgccgggcacaggttccgaagcggcggcctgggacgccgagggccgggtgctcgtcaccggtggcaccggtggcctgg
gcgcgctggtggcgcggcacctggtggccgaacatggcgtgcggcagttgctgctcgtcagccgccggggccttggcgtcgacg
gtgtggacgacctggtcgcggagctggcggagctgggcgctgaggccgtggtggccgcgtgtgacatcgccgaccgtgacggtc
tcgccgagctgctcgctcagcacccggtcagtgccgtggtgcacgcggcgggtgtcctggacgacggtgtgatcggctcgctcact
cccgagcgcatgtccgcggtgctgcgtccgaaggtcgacgcggcgtggaacctgcacgagctgacccgcgacctggacctgacc
gccttcgtggtcttctcgtccgtcgcaggtaccttcggtggagcgggtcagggcaactacgcggctggtaacgccttcctcgacgcg
ctcgtacagcggcgccgtcaggacggtctgccgggcgtctcgttggtgtggggcccgtgggaccagtcgggcggtatgaccggca
cgctcaccgaggccgatctccagcgcatggcccgtgcgggcatgcccgcgctgtcggccgagcagggtgtcgccctgttcgacac
cgctctggcggccgacgagcccgtcgtgctgccggtgcggctcgacctgccggtactgcgcgccctgggcgaggtgccgtccctg
ctgcgcggtctggtgcgggtgcgttcccgtcgggccgcggtggccggttcgagcgccgccgccggcctggtgcagcggctgagt
cggctggaggaagccgaccggcgtgaggtgctgctggacctcgtccggagccaggtggccctggtgctcgggcacgcaggcgg
tgacgagatcgatccggcacgggcgttccgcgctctcggcttcgactcgctcacggcggtggaactgcggaaccgcttgagcacg
gtctcgggcctgcgactgccggcgacgctggtgttcgactacccgacgccggatgtgctggcggactacctactggacgagctgttc
ggagccgaggccgaggcatcggtgccggtgcgggcgctgccgcccgtggccgacgacccggtggtcatcgtggggatggcgtg
ccggtatccgggtggggtgagctcgccggaggacctgtggcggctcgtctccgaaggtgccgacgcggtgtccggtttcccgacc
aaccgcagttgggacgtcgacggcctgtacaacccggaccccgaccaccaggggaccgcctacacgcgctccggtggtttcctgc
acgacgcgggtgggttcgacccggagttcttcgggatgagcccgcgtgaggcgctggcgaccgactctcagcagcgcctgctgtt
ggaggcgtcgtgggaggcgttcgagcgggccggtatcgatccggcgtcgctgcgcggcagccagaccggtgtcttcgccggcct
catgtacaacgactacaacgtcgtcctcggcggagacgagttcgagggtttcaagggcagcggcacctcgccgagcgtggcctcg
ggccgtgtctcgtacacgctgggccttgaaggtccggcggtgaccgtggacacggcgtgctcgtcgtcgctggtcgcgatgcactg
ggcgatgcaggccctgcgcagtggtgagtgctcgctggcgctggcgggcggtgtgacggtgatgtcgacgccgtcggtgtttgtcg
agttctcgcggcagcgtggtctgtcggcggacggtcggtgcaaggcgttctcggactcggccgacggtgtcggctggtcggagggt
gtcggcatgctggtgctggagcgtctctccgacgcccggcgcaacggccaccagatcctcgcggtcgtacgcggtagcgcggtca
accaggacggtgcgtcgaacggtctgacggcgccgaacggtccctcgcagcagcgggtgatccgtcaggcgctcgccagcggtg
gactctcgcccgccgatgtggacgcggtggaggcgcacggtacgggtacgacgctgggtgacccgatcgaggcgcaggcactcc
tggcgacctatggtcgtgaccgtgacgaggagcgtccgctgctgctcggttcggtgaagtccaacatcggtcacacgcaggccgcg
gccggtgtcgccggtgtcatcaagatggtcatggcgatgcgtgagggtgtcctcccgcagaccctgcacgtcgacactccgtcctcg
cacgtggagtgggaggcgggcgcggtcgagctgctgacggagaacacggcctggccggagtccggtcgtgttcggcgggccgg
cgtttcgtcgttcggcatcagcggcaccaacgcgcacgtcgtcctcgagcaggctccctcggctggcgccgctcaggccgggccg
ggtgagtcgccgacggtggagtccggcgtggtgccgtgggtcctgtcgggacggacggccgaggcgctgcgggcacaggccga
tcgcttgctgtcggctgtgcgggacggcgggacccggtccgtggacgtcgggttctcgctggccacgagccggtcggtgttcgacc
accgggctgtcgtgctcgccgacgaccacgagcacgcggtacgtgccctggcggccctgtcggccggcgcaccggaccccgcg

gtgctggaaggttcgcccaccggcgggaagacggcactgctgttctccggccagggctcgcagcggctggggatggccggga
gctgtacgagcgcttcccggtgttcgcggcggcgctggacgcggttctcgccgagctggagccgcagctggacggttcgctacgg
gacgtgatgtggggtgagcgcccggagctgctggagcagaccgggtacgcgcagcccgcgctgttcgcggtcgaggtggcgctg
ttcggtctggtgcggtcgtggggtgtgaagccggacttcctggcgggtcactccatcggtgaggtggccgccgcgcacgtggcggg
tgtgctgtcgctgccggacgcgtgccgactggtcgccgcgcgaggccgtttgatgcaggcactgcctgccggcggtgcgatggtg
gccgtccaggcatccgaggcggaggtgctgccgctgctgaccgacgaagtgtcgatcgcggcggtcaacggtccgaacgcgctg
gtcatcgccggtggcgaggatgcggtgctggagatcgcgggacagttggcggctgacgggcgtaagacgtcgcgtctgtcggtgt
cgcacgcgttccactcgccgctcatggagccgatgctggacgacttccgcgcggtggtcgagggtctgcggttcggggcccccga
ggtgccggtggtgtcgaacctgaccggacgggtggccacttcggaggagctgtgctcgccggagtactgggtgcggcacgtccgt
gaggccgtgcgtttcgccgacggggtgcagaccctggtcggtgagggtgtgggcacgcttctggagctgggtcccgacggtgtgct
gtccgccatggcccaaagctcggtgccggacggcaccgtgacggttcccgtcctgcggaagggggcggcccgaagagctcgcgg
ccatgacggcactcgcacacgtccacgtgaccggcaccccggtgagctgggaggacgtgttcgagggccgcggcgcccggcgg
gtggaccttccgacgtacgcgttccagcgtcagtggttctggcctgcgggtgcgttgagtgccccgggtgacatgcgtgcggccggt
ctggggtctgccgggcatccgttgttgggtgcggcggtggagctggcgggtggtgagggtgtcctgttcaccggtcgtctgtcggtg
cagtcgcatccgtggctggcggatcacgtagtgatgggncgggttctgctgcccggtaccgcgctggtggagctggcgatccgtgc
cggtgacgaggtcggctgcgaccggatcgacgagctgacgctcgccgcaccgctggtcctgcccgagcggggcgccgtacaggt
gcaggtccgcgtgggcgtcgccgacgaagccggtcgacgtacggtcacggtccattcccgcaccgacggtggtgaggaactgcc
gtggtcgcagcacgcgatgggtgtgctggttcctggagcggatacggccgacaccggtttcgacgccactgtgtggcccccggcc
ggggccgagccggtggacctggccggcttctacgaggcacgcgccgaggacggcttcgcctacgggccggagttccaggggct
gcggacggcgtggcggcgtgacggcgaggtgttcgccgaggtggcgttgccggaagccgagggccgggaggccgacgggttc
ggcatcaaccccgccctgctcgatgccgcgctgcacgcgtcggtgttcgccggtctcgattccgacgaccgtgggggcctgccgtt
ctcgtgggaaggcgtctcgctccacgcgtccggcgcctctgccgtacgggtgcggttgtcccgggccggtgcgagcgcggtgtcg
atcgcgctcgccgacgctgcgggtactcctgtcgcgtcggtggattcgctgttggtccgtgccgtgtcgcccgagcagttgggcggc
ccggaggtgacggtcggggactcgatgttccgggtggactgggcaccggtgcgggggccgcttcaccggccgtcgaatcggtg
gcgctggtgggcccggacgtgttcggactggctgaagccctgcccgacacggtccttcacccggacctggcgtcgttggcggcgg
gggagggaacggtgccggatgcggtgctgatccctgtggccggtgtgtccggcgacgacccggtcgcttctgtccacgacctggtg
gcctgggtgttgggtcagttgcaggtgtggttggaggaggagcggttcgcgggttcgcgtctggtgttcgtgacgcggggtgcggtg
ggcggcgccgatctggctgctgcttcggtgtggggtctggtgcgttcggcgcagtcggagaaccccggttgcttcgggctgctgga
cctcggcacggaagacgtgacgtccctgccgttggcggaggcgttggcttccgacgagccgcagctcgccgttcgtgaaggcgag
ctgcgggcagcccgactggcccgcgtgtcggacgccgtgtccgacacggtcgtctgggacgccgagggccgggtgcttatcactg
gtggcaccggtggtctgggcgcgctggtggcgcggcacctggtggccgaacacggtgtgcggcggctgctgttggtcagccgtcg
gggtctcgccgcccatggtgtggacgacctggtcgcggagctggcggagctgggcgccgaggccgtggtggccgcgtgcgacat
cgccgaccgtgacggtctcgccgagctgttggctcagcacccggtcagtgccgtggtgcatgcggcgggtgtcctggacgacggt

gtgatcggttcgctgactcccgagcgggtgtccgcggtgctgcgtccgaaggtcgacgcggcgtggaacctgcacgagctgaccc
gcgacctggacctgaccgccttcgtggtgttctcgtccgtcgcaggtaccttcggtggggccggccagggtaactacgcggctggta
acgccttcctggatgcgctcgtacagcggcgtcacgaagagggcctggccggtgtgtcgctcgtgtggggtccctgggaccagtcg
ggtggtatgaccggcacgctcaccgacgccgatctccagcgactggcccgctccggcatgccggcgttgtcggtcgagcagggcg
tggcgctgttcgacgcggccctggccggagtggaacccgtggtggtgccggtgcggctcgaccttccggcgctgcgtgcctcggg
cgaggcgccgtccctgctgcgcggtctggtgcgggtgcgttcccgtcgggccgcggtgacgggttcgagcgccgccgccggcct
ggtgcgccgactgtccgggctgaacggtgaggagcgccgtgaggcactcctggacctggtccggagccaggtggctctcgtgctg
gggcatgtcagtggagacgagatcgacccgtcgcgggcgttccgcgccctcggcttcgactcgctcacggccgtggagctgcgga
accggttgaacaccgtgaccgggctccgactgcccgccacgctcgtcttcgactacccgagcgtccaggccctggcgtcgttcgtc
ggggaggaactgctgggctcggacgcggcagtcgccgcgccggtcccgtcgcgcacgatggtgagcgacgaccccgtggtgat
cgtggggatggcgtgccggtatccgggtggggtgagctcgccggaggacctgtggcggctcgtcaccgatggcaccgacgcggt
gtccggtttcccgaccaaccgtggctgggacgtcgagggtctctacgacgcggatccggggcacgcgggtacgtcgtacacgcgc
tccggtggtttcctgcacgacgcgggcgagttcgacccggagttcttcgggatgagcccgcgtgaggcgctggcgaccgactcgca
gcagcggttgctgttggaggcgtcgtgggaggcgttcgagcgggccggtatcgatccggcgtcgctgcgcggcagtcagaccggt
gtcttcgccggtgtgatgtacagcgactacggcagcacgttgaacggcaaggagttcgagggtcatcagggccagggcagcgcgg
gcagtgtggcctcgggccgtgtctcgtacacgttcgggctggagggcccggcagtgacggtcgacaccgcgtgctcgtcgtcgctg
gtgtcgctccacctcgccgcgcaggccctgcgcagcggtgagtgctcgctggcgctggccggtggtgtgacggtcatgtcgacgc
cgtcggtgttcgtggagttctcgcggcagcgtggtctgtcggcggacggtcggtgcaaggcgttctcggactcggccgacggtgtc
ggctggtcggagggtgtcggcatgctggtgctggagcgcctctccgacgcgcagcgcaacggccaccggattctggcgctcgtac
gcggtagcgccatcaaccaggacggtgcttccaacggtctgacggctccgaacggtccgtcccagcagcgggtgatccgtcaggc
gctcgccagcggtggactctcgcccgccgatgtggacgcggtggaggcgcacggtacgggtacgacgctgggtgacccgatcga
ggcgcaggcgttgctggcgacctatggtcgggaccgtgacgaggagcgtccgctgctgctcggttcggtgaagtccaacatcggtc
acacgcaggccgcggccggcgtggccggtgtcatcaagatggtcatggcgatgcgtgagggtgtcctcccgcagaccctccatgt
gacggcgccttcgtcgcacgtggactgggaagccggtgcggtggagctgctccgggagaacacggcctggccgcagtccggtca
cgcacgccgcgcgggcatctcgtccttcggtatcagtgggaccaacgcgcacgtgatcgtcgagcaggctccctcggtgaaggag
ccgagccgggagccggtggtttcgccgggtgtggtgccgtgggtggtgtccgcaaggaccggggcggcgctcgacgcccaggt
ggaccggctgcggtccttcgtggcggacgacacggcagcgacctcggtcgacgtcggcttctcgctggtgaccggccgttcggtgt
tcgagcaccgtgcggtgctgctgtccaccgacgaccgtgtgatcgaggcggcccggggcacggcggccggtgagggttcgctgg
cggtgctgttctccgggcagggcagccagcggatcggtatggggcgtgagctgtacgcccggttcccggtcttcgcggacgccctg
gacaaggccctgtcggtgctcgacgagcacctggaccggccgctgcgggacgtgatgtggggcgatgacccggacgagctgaac
cggaccggctgcacgcagcccgcgttgttcgcggtcgaggtggcgctgttccggctggtggagtctctcgggatcaccccggactt
cgtggccggtcactccatcggtgagatcgctgcggcacacgtcgcgggggtgttctcgctggaggacgccggccgactcgtcgcg
gcacgcgccgggttgatgcaggcgctgcctgcgggtggcgcgatggtggccgtgcaggcgaccgaggcggaggtgctgcctctg

ctgaccgacgaggtgtcgatcgcggcggtcaacggtccgtcggtggtcgtctcgggtgtcgaggacgcggtgctggagattgc
gggcaagctgtcggccgagggccgtaagacgcatcgactgccggtgtcgcacgccttccactcgccgctgatggagccgatgctg
gaggacttccacgcggtggtcagcggtctgtcgttcaatgcaccgtcggtgccggtggtctccaacctcaccggcaggctcgccac
gtccgaggaactgtgctcgccccagtactgggtacgtcacgtttgtgaggcggtccgcttcgccgacggagtacggaccctggctga
cgagggtgtggatacgttcctcgagctgggtccggacggtgtgctgtccgccatggctgccgagagcgtgccggacggcactgcc
gtcgtgccggtcctgcgcaaggaccggcccgaggagaccgcggcggtcaccgccctggcccggctgcacgtacagggcgtcac
ggtcgactggacggcgttcttcgccgggaccggcgcccgtcgggtggacctgccgacgtacgcgttccagcgtcagtggttctggc
cggtgggtgcgttgagtgcgccgggtgacatgcgtgcggctggtctgggctctgcggggcatccgttgttgggtgcggcggtggag
ctggcgggtggtgagggtgtcctgttcaccggtcgtctgtcggtgcagtcgcatccgtggctggccgatcacgtggtcatgggtcgg
gttctgctgcccggtaccgcgctggtggagctggcgatccgtgccggtgacgaggtcggctgcgaccggatcgacgagctgacgc
tcgccgcaccgctggtgctgcccgagcagggtggcgtacagctgcagctgtgggtcggtggccccgacgagacgggcagtcgca
cgctcggcattcactcccgccccgacagcggcgacgagctgccgtggacgcagcacgcgacgggtgtgctgaccgccggtacgg
acgccaccgaaggtggcttcgacgcgacggaatggcccccggccggcgccgagcccgtggaactcacgggctgctacgaccgg
ttcgccgacatcggcttcgcgtacggccccggattccaggggctgcgggcggcatggcggcgtgacggtgacgtcttcgccgagg
tgagcctccccgagggactggccgccgacgcgggagtgttcggcctgcaccccgcgctgctcgacgccgcgatgcacgcctccct
gctcgccgacggcgatacacaaggcggcggcgggctgccgttctcgtgggaaggcgtgtcactgcacgccaccggcgcctccga
cgtccgggtgcggttgaccccgaccggcgacaacgcgatatccatcgcgctcgccgacacctcgggcgccccggtcgcgtcggt
ggattcgctgctggtccgtaccgtgacgcccgagcagctgaacggccccgaaaagccggtcggcgaatcgatgttccgggtggac
tgggcaccggtacgggcagccgccccggcgggcacgcacacggtggcgctggtgggcccggacacgttcgggctggcagaga
ccctgccgggcatggtcctgcacccggatctggagtccttgacggcctccggactggccctgccggacaccgtcctgatccccgtg
gccggtgggcccgacgacgacacggtcgactccgtccacgatctggtgtcccgggtgttgggtcagttgcaggtgtggttggagga
ggagcggttcgtcggttcgcgtctggtgttcgtgacgcggggtgcggtgggcggcgccgatctggctgctgcttcggtgtggggtct
ggtgcgttcggcgcagtcggagaaccccggttgcttcgggctgctggacctcggcacggaagacgtgacgtccctgccgttggcg
gaggcgttggcttccgacgagccgcagctcgccgttcgtgaaggcgagctgcgggcagcccgactggcccgcgtgcccgcgccg
gacacggcccccgaggccgtgtggggtgcggacggcaccgtggtgatcaccggtggtaccggcggtctcggcgcggtgacggc
ccggcacctggtggccgaacacggcgtccggcggctcctgctggtcagccgtcggggccttggggccgaaggcgccacggacct
ggtcacggagctggagcgactgggcgcgagcgtctcggtcgccgcgtgcgacgtggcggaccgggacgcgctggccgaggcc
ctggcctcggtcgacggcgaccacccgctgagcgccgtggtccacaccgccggtgtcctggacgacggtgtgatcggttcgctga
ctcccgagcgggtgtccgcggtgctgcgtccgaaggtcgacgcggcatggaacctgcacgagctgacccgcgatgcggacctgg
ccgccttcgtggtgttctcgtccgtcgccggtaccttcggtggagcgggtcagggcaactacgcggccggcaacgccttcctcgacg
cgctcgtacagcggcgtcacgaagagggcctggccggtgtgtcgctcgtgtggggcccgtgggaccagtcgggcggtatgaccg
gcacgctcaccgacgccgatctccagcgcatggcccgctccggcatgccggcgttgtcggtcgagcagggcgtggcgctgttcga
cgcggccctggccggagggaacccgtggtggtaccggtgcggctcgaccttccggcgctgcgcaccctgggggagacaccgg

cgctgctgcgcgggctgatccacacccccgcgcgcaggaccgtgacggccgtgcccgacgccgccgaggggctcacgcagcg
gctgggcggactgagcgacgacgaacgccgtgacgtcctcctggaactggtccgcgaccaggccgcggcggtcctcggccacg
cggacgggcaggcgatcgcctccacccgccaattccaggacctgggattcgactccctcacgtccgtggagttccggaaccgcatg
aacagggccacggcgcttcggcttccggccacgctcctgttcgactacccgaccccggccgaactcgtgggctacctccacaccca
gctcgcggtggaaggggacgccggacccgggtcgatcctcggcgcgctcgacacgctggagaaagcgatcgcggagctgaacg
tcgacgcgcaggtccataagcaggtgtccggacgccttgaggtgctccgaaccaagtgggaggccctgcgcggcgaagcacgca
ccgaagaagaggagttcgacatcgactcggcctcggacgacgaggtgttcgcgctcctcgacgacgagctcggcctgtcctgaagt
ccgccgccggccaccgcgaggcggtggccggccgcccggccgggggtgtccccggccgggcggtcacaggcggatccgtga
ccgcccggccgcggaaaactagcaattttccgacttcccatcctttgaaatggggtggctgtcgcgccgatgtgacccggaggcaag
agccgtcgaaggaagggacgacacgatgacgatcaccaccgggaaatcggacgcggtgctgccccacggcgccgcacggtga
gcggccgcgaggtgtccgaagcaaccatccggctcgcgccggcggccgagctgttcggtgcggtcgaaacgcggtcaaggcctg
aagcccttcagcgtcgcgaggaaaggatcttccatgaaggctctggtgttgtcgggcggggcgggcacccgtctgcgaccgttcag
ctactccatgccgaaacagctcatgccgatcgccaacaagcccgttctggaacatgtgttggagaccatccgtgagctgggcgtcac
cgaggtgggagtgatcgtcggtgaccgggggccgcacatagcggccgtcctcggcgacggttcccgcttcggcgtgcggctgac
cttcatcgaacagaaggagcccctcggcctcgcccacaccgtctcggtggcccggcccttcctcggcgacgacgacttcgtgatgta
tctcggggacaacatgcttcccgacggcatcaccgaactcgccgaggagttcgccgagcaccgccccgccgcccaggtcgtcgtc
cgcaaggtcgcggacccgcgtgccttcggggtggccgaactcgggccggacggcgaggtcgtccgcctcgtggagaagcccgc
ggaaccgcgcagcgacctcgcgctgatcggcgtgtacttcttcacctccgccatccacgaggccgtggacgccatcgaacccagcc
accgggggcgagctggagatcaccgacgccgtccagtggctcgtgaccgccggcgcggacgtacgcgcccgtgagtaccacggg
tactggaaggacaccggtcgcgtcgaggacgtactggagtgcaaccggaaggtgctcgagggcatcgagcactcggtcgcgggg
gaggtcgacgacgccagtgaactgaccggccccgtgatcgtcgaggccggtgcccgcgtcgtccgctcccggatcgagggcccg
gcgatcatcggcgcgggcaccctcgtggaggacagccgcgtcggcccgtacacggccctcggccgtgactgcgtgctgcgccac
acccgaatcgaccactccatcaccttggaaggtgccatggtgacccacgttcgcggtctgcacggctcgctgatcggccgctcggcc
accgtcgacgccaccgaggtgggcgcgggccgccaccggatcgtcgtcggctgccacgcccacgtcgaggtgacggcatgagg
ctcctggtcaccggcgcggccggcttcatcggctcgacgtacgtccgtacgctcctggacggcggctatcccggctacgaggacgc
ccacgtcaccgtcctggacaagctgacctacgcgggcaaccgcgacaacctgcccggcacgcacccccgcatgacgttcgtccgc
ggcgacatcaacgacctgccgctcctgctcgatctgctgcccgggcacgacgccgtggtgcacttcgccgccgagtcgcacgtcga
ccgctccctgacggccgcggcggacttcatccggacgaacgtctgcggcacccagaacctcctcgaagcgtgcctgcgcagcggt
gtccagcgcgtcgtgcacgtctccaccgacgaggtgtacggctccatctccgagggctcctggaccgaggagtggccactggagc
ccaacaccccgtacgcggcctccaaagcggcctccgacctggtggcgcgctcgtactggaagacgcacggcctggacctgtcgat
cacccgctgctccaacaactacgggccgtaccagcaccccgagaagctcatcccgctgttcgtcaccaatctgctcgagggccgga
ccgtcccccctctacggggacggccgcaacatccgcgagtggctgcacgtcgacgaccactgccggggcatccagctcgtcctgac
cagcgggcggtccggtgagatctacaacatcggcggcggcgacgcatacaccaacgtcgagatcacccggcggctgctggagct

gtgcggcgccgacgagtccatggtccgccacgtcgccgaccgcaagggacacgacctgcgctactccatcgcggaggacaagat
ccgggacgagctgggatacgtcccgctgacctccttcgaacgcggcctctccgacacggtcggctggtaccgggacaaccccggc
tggtggaaggcgtccaagccgcaggaggaagcgaagtgaccgcagtggcccaaggaacccccatgcggatcctgatgatcccga
gccccgtcgtcacccacctcatgcccttggtcccccctggcatgggcactgcgctccgcgggccacgagctcctcgtcgtcggacag
ccggacctgatgggcgtggcccggcaggccgggctgaacgccgtgagcatcggcgaccggttcggcatggaggaggtcttccac
ggaatgctggaaccgggcaagcgccccatcgagctgtggggccggctccaccccgatcacctgaagcacttcccccggtctgga
aggaccacggcgagcgcgtactgcccgcctacctggagctcgcccgcgcgtaccgccccgacctgatcgtggccgatccgatgg
agttcaacaccctcgtggtgggcgggctgctgggcgtcccggtcctgcaccaccggttcggtgtcgacgcggtgtccgagccggtg
cgcgcggccgcgcggggcgcgatgcgggattcctgctgggccctgggcctcgacgagctgcccgatcccgacattcagctcgac
ccctgcccccccgagcctgcaactgcccagcctcgatgaggcccttcccatccgctacgtgcccttcaacggcagtggcgaggtgcc
cgcctggctccgcgaggagcgaccgtcggccacggggaagcggcgcgtcgtggtctcgctggggacccgtacgctcgcgctcaa
cggagtgcccttcgtgcgcggcctgttgcgggccttcgacggtctgcgggacgtcgaggccgtcgccaccgtcccggaggcgttc
cggggcgagatcggagccgtgccgggcaacgtgcgcatgaccgacccggtgccgctccacctgctcgtggagacctgcgacgc
ggtcgtccaccacggagggtcgggcacggtgctgaccgccgtgtccgccgggctcccgcacctggtactgccgcagatggccga
ccagttcgggcacgccgaccagctggtcgcggcgggggcgggcctcgcgatcgacgacgccgcggggcaggacgacacggtg
cgactgcggtgcgcgctggaggaactgctgtcggagcccggctacgccaaggcggcgtgggaactgcgcgaagccatgcggga
gatgcccaccccggccgaggtcgtggccggactcgggcgactcctctgaccccgtccgccctcgaccggtgccgacccaccccc
gtcacccggtgtgcggccgaacggccgcacacccaccctcacaaggagcgtcgatgtccgaccagcccggccagtccgcccacc
ccgccgtcgagtgcgacctccccgacggccggtccgtcatgggcatccatcccctggagacggtcacgctctgggggggagatcgc
cggggattcccccctacgcgcgcgcctccgccgggctgggccccgatgacgtgatcctggacatcggcgcgcacatcggactggc
ctcgatgcggttcgccgaacaggcccccggcgcccggatcctgtccttcgagcccgccccggtgacctacgcctgcctggcggac
aacgtcacccggcacgtcccgacgggcaccgcgttcccccaggcggtgggcggcgaggcgggcaccgcggagctcaccttcca
ccccttcaactcctcgacgtcgaccctgcacgccgacccggaggacgacctgcgcaacttcaaggcgttcgtcgacaacaccgag
gtgccgggggacgcccgcgagctgatgctcagcgccttcggcgagaagcagacggtgaccgtcgacgtgaccacggtgaccgc
cgtggtgaaggagcacggcatcgagcggatcggtctgctgaagatcgacgtcgagcgcggtgaactcgaggtgttgcgcgggatc
gacaccggactctggccgcggatccagcgcgtgctcctcgaagtgcacgacatcgacggccggctcggcaggatcgtcgccgag
ctggccggcctcggcttcgaggtcacggtgtcgcaggcgccggtcttcgtcggcggcagcgtgttcagcctgctcgcgacacggcc
ctgacctcagagccgtgtcgtgaccgaatccccccctggacccccagctgaacacgtccaggacgcgccggtagtagtccgggccg
aagccgagccggtccacctcggggacgagggcccgcagcttgtcgatggagatggtgtgatgactgcccgtctcctggtactcgcg
gcgggccgtcagaccgagaccgcgctccagatggtcgacgatcgtgtcgaccgggaccgcggcgccggaggcgacgttgaccg
tctcggccgttaggtccagcgccagcagacggtcgatcacggtcaccgtgtcggtcacatcgatcagatcacgggtggcggcacgg
tggatgcggaccacaccgccccgcaactgccggaccagggtgggcagtagctggtgcggtggctgcccgggaccgaccagatg
gctcaggcgcaggatcagatggtcggcacccgtggcgcgcagctgctcctcgagggcgagtttgtgcgcgccgtaaggggagca

gggcaccacgggccggtcctcccgcccgggcccgtccgccgccccgtacatcccggtggcggacgtcgagaagaacaacagcc
gccgcccgtcggccttgcagcgcagcgcggtctcccgcagcagcgccgcctcacgggcgaagtcggcgtcggaggtgccggac
gcccaggagactccggcagcgagcgccacggtccccgggtgccggtcggccatcggtcgcagactcccggcgaggaatccgtt
gcccacaatgtccatgcgtcgagtcctgatcttcgagatgagggggggcggccgtgccgggcgtagaagaccagcgatggcccc
gggaggtcaaccatgtgggcggatgtccaaggatgtccttagtgtttcgccggaccgggcggcccgtggaacccgcgctcgcgtc
gggcgccgaccagcggcgccacccgcccggcccccgcgagccgcgggaggaccgtaccgaaccaccgaaacccagaccttg
gaccgaaccaaccgaaccctttcggaagtacaaaccgcaggcttagtcgtgtgggcctggatgacgttgacgcgcctcctcggaact
atctaatgtgcggttccggagccgggcagtccagggcattccgaacgaggcccggtcatcggttttctgacgcatttctagcgcgtgg
ttctgaacgctctgcggaacctgaaagattggcaggctcgtggctaattccttacttgcagacttggtaccgggcgttactcgcggata
cacggacgaccaaggggtcaaccagccgcagttcgaccagccgggcctgtgtatggcctccctcgatccgaccctgacgatccga
caggcgaatcaggagttcttccgccagttcggcgcctcctcggcggacgtgtgcgggcgcagcttccaggagctggtgcacccga
gcgtgcagcagccgctgatgcgccagttctcacggctggtcgagggcaagcggcagcgcttcgccacccatgtggtcggggtccg
gcaggaggattccgccttcgccagcccccctgacggccatcgcggtccgcggcggtacgcccgaggacgcgtcgatcctggtgctg
atgccgcagacccaggggtccgacgacgccggagtggtcaccaaccggaagaagatcctcaccgagatagacgcacgcatcctc
gagggcatagccgccgggatctccacggtcccgctcgcctcacggctctacctcagccggcagggtgtcgagtaccacgtgaccg
gattgctccggaagctcaaggtgcccaaccgggccgccctggtctcgcgggcctactccatgggggtgctcaatgtgggcacctgg
ccgccgaaggtcgtcgaggaattcatcaagtgacacccataaaggtttgacgtcgaccgaccggccaaccggccgaaacagctcg
gccgcggcggaactttcgggccaggaaagcccgacggttttccgccgtggccgagctgtcgtccgtgggtaaacccgcggtaagt
gcgggagggcaagggaaacgcccagagattccgtgcccggggcggcgcggtcttgtgcccgctcggccgtcgcggggatagtg
gtatcgcgccgaaagtgaacgagacgaaggacgagcatggctgaaatacccgtggacgacacgctgtggtgccggaacttccgtc
ccacccccgacgcacccgtgcgcctggtctgcttcccgcacgcgggcggttccgcgagcttctacctgcccctcgcctcggcgctg
acgccggacgccgacgtcctggcgatccagtaccccgggcgccaggaccgccgccgcgaaccctgcgtggacgacatcgcgac
actcgccgaacaggtccacacggcgctcgccggctggctggaccggccggtggtgttcttcggtcacagcatgggcgccctggtg
gcgttcgaggtggcccgccgcatggaacgcgaaggccagggccccgcacggctgttcgcctcggggcgccgcgcgccctcccg
ctaccgcgacgagaacgtccaccagcgcgacgacgacggcatcgtcgccgaactgcgagacatgagcggtacggacacccggg
tgctcggcgacgaggagatgctgcggatggtcctgcccgccatccgtgccgactaccgcgcggtggagacctaccgcagcgagc
cgggcgcgtcggtgcgctgccccgtcacggtcatggtgggcgacgacgaccccaagacctccctcgacgaggccgaggactgg
aaggggcacaccaccggcgggttcgacatgcaggtctactccggtgggcacttctacctcagcagtcaggcgcccgccgtcatcac
ggccctgcgcggtcacctggccgccgtgccggcctctccccagggctgaggccgggcggcagccggtcgggacacagcgggc
cggggcctcgcggtacgcggacacacgccgtccgtcggcagtacgggtgataccccctgccggcggcgcgcgtgtcggtggaccc
gcggcggcccggcgggtgacgctgcgtcggacaaatagccggaatatagcgctatggcatcagatagctgatggctgatacgtca
aacgcgcgacggtccggtgtccggctccgatcgaggagagcgatcg

A nucleotide sequence of a key gene *MandQ* (SEQ ID NO. 2) is shown below (1072bp):

gtcacccacctcatgcccttggtccccctggcatgggcactgcgctccgcgggccacgagctcctcgtcgtcggacagccggacct
gatgggcgtggcccggcaggccgggctgaacgccgtgagcatcggcgaccggttcggcatggaggaggtcttccacggaatgct
ggaaccgggcaagcgccccatcgagctgtggggccggctccaccccgatcacctgaagcacttcccccggtctggaaggacca
cggcgagcgcgtactgcccgcctacctggagctcgcccgcgcgtaccgccccgacctgatcgtggccgatccgatggagttcaac
accctcgtggtgggcgggctgctgggcgtcccggtcctgcaccaccggttcggtgtcgacgcggtgtccgagccggtgcgcgcgg
ccgcgcggggcgcgatgcgggattcctgctgggccctgggcctcgacgagctgcccgatcccgacattcagctcgacccctgccc
cccgagcctgcaactgcccagcctcgatgaggcccttcccatccgctacgtgcccttcaacggcagtggcgaggtgcccgcctggc
tccgcgaggagcgaccgtcggccacggggaagcggcgcgtcgtggtctcgctggggacccgtacgctcgcgctcaacggagtg
cccttcgtgcgcggcctgttgcgggccttcgacggtctgcgggacgtcgaggccgtcgccaccgtcccggaggcgttccggggcg
agatcggagccgtgccgggcaacgtgcgcatgaccgacccggtgccgctccacctgctcgtggagacctgcgacgcggtcgtcc
accacggagggtcgggcacggtgctgaccgccgtgtccgccgggctcccgcacctggtactgccgcagatggccgaccagttcg
ggcacgccgaccagctggtcgcggcgggggcgggcctcgcgatcgacgacgccgcggggcaggacgacacggtgcgactgc
ggtgcgcgctggaggaactgctgtcggagcccggctacgccaaggcggcgtgggaactgc

## Claims

1. A biosynthetic gene cluster for a polyene macrolide natural product mandimycin, **characterized in that** a nucleotide sequence of the mandimycin biosynthetic gene cluster is shown in SEQ ID NO. 1.

2. A preparation method for the biosynthetic gene cluster for a polyene macrolide natural product mandimycin of claim 1, **characterized by** comprising the following steps:
preparing a strain containing the mandimycin biosynthetic gene cluster into a seed culture, and performing cultivation, fermentation, extraction, isolation, and purification on the seed culture to obtain the natural product mandimycin.

3. The preparation method for the biosynthetic gene cluster for a polyene macrolide natural product mandimycin according to claim 2, **characterized in that** the strain containing the mandimycin biosynthetic gene cluster is *Streptomyces netropsis* DSM 40259.

4. A preparation method for an antifungal polyene macrolide natural product mandimycin B, **characterized by** comprising the following steps:
knocking out a *MandQ* gene in the strain containing the mandimycin biosynthetic gene cluster of claim 1 to obtain an engineered strain CPU002; preparing a seed culture, followed by cultivation, fermentation, extraction, isolation, and purification to obtain the natural product mandimycin B; **characterized in that** a nucleotide sequence of the *MandQ* gene is shown in SEQ ID NO. 2.

5. The preparation method according to claim 4, **characterized in that** the engineered strain CPU002 is obtained by knocking out the *MandQ* gene in the mandimycin biosynthetic gene cluster of *Streptomyces netropsis* DSM 40259 using intergeneric conjugation between *Streptomyces* and *Escherichia coli*, and the resulting engineered strain CPU002 with a glycosyltransferase gene deleted is subjected to fermentation, extraction, and purification to obtain mandimycin B.

6. A polyene macrolide natural product mandimycin or mandimycin B and a pharmaceutically acceptable salt thereof, **characterized in that** a chemical structure of the natural product mandimycin is shown in formula I, and a chemical structure of the natural product mandimycin B is shown in formula II:

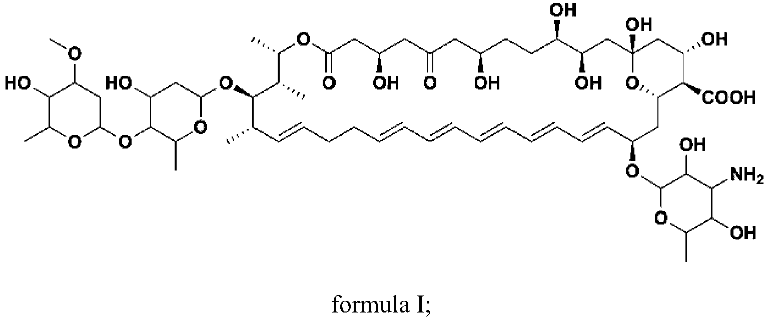

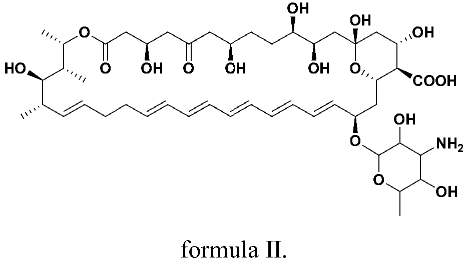

7. Use of the polyene macrolide natural product mandimycin or mandimycin B and a pharmaceutically acceptable salt thereof of claim 6 in the preparation of an antifungal drug.

8. The use according to claim 7, **characterized in that** the fungus is any one of various key fungal pathogens, such as *Candida, Aspergillus, Cryptococcus, Mucor*, and *Fusarium* species.

9. A pharmaceutical composition containing an antifungal drug, **characterized by** comprising the polyene macrolide natural product mandimycin or mandimycin B and the pharmaceutically acceptable salt thereof of claim 6.

10. The pharmaceutical composition according to claim 9, **characterized in that** the pharmaceutical composition is in the form of a capsule, a powder, a tablet, a granule, a pill, an injection, a syrup, an oral solution, an inhalant, an ointment, a suppository, or a patch.

11. Use of the pharmaceutical composition containing an antifungal drug of claim 9 in the preparation of an antifungal drug.

12. The use according to claim 11, **characterized in that** the fungus is any one of various key fungal pathogens, such as *Candida, Aspergillus, Cryptococcus, Mucor,* and *Fusarium* species.
